# EUROPEAN PATENT SPECIFICATION

(11) **EP 2 451 959 B1**
(45) Date of publication and mention of the grant of the patent: **11.01.2017**
(21) Application number: 10737815.0
(22) Date of filing: 09.07.2010
(51) Int. Cl.: C12N 15/82

(54) **DISRUPTION OF CKX3 AND AT LEAST ONE OTHER CKX GENE IN A PLANT OR PLANT CELL LEADS TO IMPROVED TRAITS**
SPALTUNG VON CKX3 UND MINDESTENS EINEM ANDEREN CKX-GEN IN EINER PFLANZE ODER PFLANZENZELLE FÜHRT ZU VERBESSERTEN MERKMALEN
DISSOCIATION DE CKX3 ET D'AU MOINS UN AUTRE GÈNE CKX DANS UNE PLANTE OU CELLULES VÉGÉTALES POUR TRAITS AMÉLIORÉS

(30) Priority: 10.07.2009 EP 09165164
(43) Date of publication of application: 16.05.2012
(73) Proprietor: Schmülling, Thomas, 14052 Berlin (DE); Bartrina Y Manns, Isabel, 10829 Berlin (DE); Werner, Tomas, 10829 Berlin (DE)
(72) Inventor: Schmülling, Thomas, 14052 Berlin (DE); Bartrina Y Manns, Isabel, 10829 Berlin (DE); Werner, Tomas, 10829 Berlin (DE)
(74) Representative: Gulde & Partner
(86) International application number: PCT/EP2010/059880
(87) International publication number: WO 2011/004003

(56) References cited:
- EP-A- 1 580 270
- US-A1- 2004 031 073
- SCHMUELLING T ET AL: "CYTOKININ AS A REGULATORY FACTOR FOR YIELD AND BIOMASS DISTRIBUTION IN CROP PLANTS" PHYTOHORMONES IN PLANT BIOTECHNOLOGY AND AGRICULTURE, KLUWER ACADEMIC PUBLISHERS, AMSTERDAM, NL, 1 January 2003 (2003-01-01) , pages 97-108, XP009053589
- WERNER T ET AL: "Regulation of plant growth by cytokinin" PROCEEDINGS OF THE NATIONAL ACADEMY OF SCIENCES OF USA, NATIONAL ACADEMY OF SCIENCE, WASHINGTON, DC, US, vol. 98, no. 18, 28 August 2001 (2001-08-28), pages 10487-10492, XP002240696 ISSN: 0027-8424

### SEQUENCE LISTING

<110> Schmülling, Thomas
<120> Disruption of CKX3 and at least one other CKX gene in a plant or plant cell leads to improved traits
<130> P663409EP
<160> 61
<170> PatentIn version 3.3
<210> 1
   <211> 523
   <212> PRT
   <213> Arabidopsis thaliana
<400> 1
   Met Ala Ser Tyr Asn Leu Arg Ser Gln Val Arg Leu Ile Ala Ile Thr
   1               5                   10                  15
   Ile Val Ile Ile Ile Thr Leu Ser Thr Pro Ile Thr Thr Asn Thr Ser
               20                  25                  30
   Pro Gln Pro Trp Asn Ile Leu Ser His Asn Glu Phe Ala Gly Lys Leu
           35                  40                  45
   Thr Ser Ser Ser Ser Ser Val Glu Ser Ala Ala Thr Asp Phe Gly His
       50                  55                  60
   Val Thr Lys Ile Phe Pro Ser Ala Val Leu Ile Pro Ser Ser Val Glu
   65                  70                  75                  80
   Asp Ile Thr Asp Leu Ile Lys Leu Ser Phe Asp Ser Gln Leu Ser Phe
                   85                  90                  95
   Pro Leu Ala Ala Arg Gly His Gly His Ser His Arg Gly Gln Ala Ser
               100                 105                 110
   Ala Lys Asp Gly Val Val Val Asn Met Arg Ser Met Val Asn Arg Asp
           115                 120                 125
   Arg Gly Ile Lys Val Ser Arg Thr Cys Leu Tyr Val Asp Val Asp Ala
       130                 135                 140
   Ala Trp Leu Trp Ile Glu Val Leu Asn Lys Thr Leu Glu Leu Gly Leu
   145                 150                 155                 160
   Thr Pro Val Ser Trp Thr Asp Tyr Leu Tyr Leu Thr Val Gly Gly Thr
                   165                 170                 175
   Leu Ser Asn Gly Gly Ile Ser Gly Gln Thr Phe Arg Tyr Gly Pro Gln
               180                 185                 190
   Ile Thr Asn Val Leu Glu Met Asp Val Ile Thr Gly Lys Gly Glu Ile
           195                 200                 205
   Ala Thr Cys Ser Lys Asp Met Asn Ser Asp Leu Phe Phe Ala Val Leu
       210                 215                 220
   Gly Gly Leu Gly Gln Phe Gly Ile Ile Thr Arg Ala Arg Ile Lys Leu
   225                 230                 235                 240
   Glu Val Ala Pro Lys Arg Ala Lys Trp Leu Arg Phe Leu Tyr Ile Asp
                   245                 250                 255
   Phe Ser Glu Phe Thr Arg Asp Gln Glu Arg Val Ile Ser Lys Thr Asp
               260                 265                 270
   Gly Val Asp Phe Leu Glu Gly Ser Ile Met Val Asp His Gly Pro Pro
           275                 280                 285
   Asp Asn Trp Arg Ser Thr Tyr Tyr Pro Pro Ser Asp His Leu Arg Ile
       290                 295                 300
   Ala Ser Met Val Lys Arg His Arg Val Ile Tyr Cys Leu Glu Val Val
   305                 310                 315                 320
   Lys Tyr Tyr Asp Glu Thr Ser Gln Tyr Thr Val Asn Glu Glu Met Glu
                   325                 330                 335
   Glu Leu Ser Asp Ser Leu Asn His Val Arg Gly Phe Met Tyr Glu Lys
               340                 345                 350
   Asp Val Thr Tyr Met Asp Phe Leu Asn Arg Val Arg Thr Gly Glu Leu
           355                 360                 365
   Asn Leu Lys Ser Lys Gly Gln Trp Asp Val Pro His Pro Trp Leu Asn
       370                 375                 380
   Leu Phe Val Pro Lys Thr Gln Ile Ser Lys Phe Asp Asp Gly Val Phe
   385                 390                 395                 400
   Lys Gly Ile Ile Leu Arg Asn Asn Ile Thr Ser Gly Pro Val Leu Val
                   405                 410                 415
   Tyr Pro Met Asn Arg Asn Lys Trp Asn Asp Arg Met Ser Ala Ala Ile
               420                 425                 430
   Pro Glu Glu Asp Val Phe Tyr Ala Val Gly Phe Leu Arg Ser Ala Gly
           435                 440                 445
   Phe Asp Asn Trp Glu Ala Phe Asp Gln Glu Asn Met Glu Ile Leu Lys
   450 455 460
   Phe Cys Glu Asp Ala Asn Met Gly Val Ile Gln Tyr Leu Pro Tyr His
   465 470 475 480
   Ser Ser Gln Glu Gly Trp Val Arg His Phe Gly Pro Arg Trp Asn Ile
   485 490 495
   Phe Val Glu Arg Lys Tyr Lys Tyr Asp Pro Lys Met Ile Leu Ser Pro
   500 505 510
   Gly Gln Asn Ile Phe Gln Lys Ile Asn Ser Ser
   515 520
<210> 2
   <211> 501
   <212> PRT
   <213> Arabidopsis thaliana
<400> 2
   Met Ala Asn Leu Arg Leu Met Ile Thr Leu Ile Thr Val Leu Met Ile
   1 5 10 15
   Thr Lys Ser Ser Asn Gly Ile Lys Ile Asp Leu Pro Lys Ser Leu Asn
   20 25 30
   Leu Thr Leu Ser Thr Asp Pro Ser Ile Ile Ser Ala Ala Ser His Asp
   35 40 45
   Phe Gly Asn Ile Thr Thr Val Thr Pro Gly Gly Val Ile Cys Pro Ser
   50 55 60
   Ser Thr Ala Asp Ile Ser Arg Leu Leu Gln Tyr Ala Ala Asn Gly Lys
   65 70 75 80
   Ser Thr Phe Gln Val Ala Ala Arg Gly Gln Gly His Ser Leu Asn Gly
   85 90 95
   Gln Ala Ser Val Ser Gly Gly Val Ile Val Asn Met Thr Cys Ile Thr
   100 105 110
   Asp Val Val Val Ser Lys Asp Lys Lys Tyr Ala Asp Val Ala Ala Gly
   115 120 125
   Thr Leu Trp Val Asp Val Leu Lys Lys Thr Ala Glu Lys Gly Val Ser
   130 135 140
   Pro Val Ser Trp Thr Asp Tyr Leu His Ile Thr Val Gly Gly Thr Leu
   145                 150                 155                 160
   Ser Asn Gly Gly Ile Gly Gly Gln Val Phe Arg Asn Gly Pro Leu Val
                   165                 170                 175
   Ser Asn Val Leu Glu Leu Asp Val Ile Thr Gly Lys Gly Glu Met Leu
               180                 185                 190
   Thr Cys Ser Arg Gln Leu Asn Pro Glu Leu Phe Tyr Gly Val Leu Gly
           195                 200                 205
   Gly Leu Gly Gln Phe Gly Ile Ile Thr Arg Ala Arg Ile Val Leu Asp
       210                 215                 220
   His Ala Pro Lys Arg Ala Lys Trp Phe Arg Met Leu Tyr Ser Asp Phe
   225                 230                 235                 240
   Thr Thr Phe Thr Lys Asp Gln Glu Arg Leu Ile Ser Met Ala Asn Asp
                   245                 250                 255
   Ile Gly Val Asp Tyr Leu Glu Gly Gln Ile Phe Leu Ser Asn Gly Val
               260                 265                 270
   Val Asp Thr Ser Phe Phe Pro Pro Ser Asp Gln Ser Lys Val Ala Asp
           275                 280                 285
   Leu Val Lys Gln His Gly Ile Ile Tyr Val Leu Glu Val Ala Lys Tyr
       290                 295                 300
   Tyr Asp Asp Pro Asn Leu Pro Ile Ile Ser Lys Val Ile Asp Thr Leu
   305                 310                 315                 320
   Thr Lys Thr Leu Ser Tyr Leu Pro Gly Phe Ile Ser Met His Asp Val
                   325                 330                 335
   Ala Tyr Phe Asp Phe Leu Asn Arg Val His Val Glu Glu Asn Lys Leu
               340                 345                 350
   Arg Ser Leu Gly Leu Trp Glu Leu Pro His Pro Trp Leu Asn Leu Tyr
           355                 360                 365
   Val Pro Lys Ser Arg Ile Leu Asp Phe His Asn Gly Val Val Lys Asp
       370                 375                 380
   Ile Leu Leu Lys Gln Lys Ser Ala Ser Gly Leu Ala Leu Leu Tyr Pro
   385                 390                 395                 400
   Thr Asn Arg Asn Lys Trp Asp Asn Arg Met Ser Ala Met Ile Pro Glu
   405 410 415
   Ile Asp Glu Asp Val Ile Tyr Ile Ile Gly Leu Leu Gln Ser Ala Thr
   420 425 430
   Pro Lys Asp Leu Pro Glu Val Glu Ser Val Asn Glu Lys Ile Ile Arg
   435 440 445
   Phe Cys Lys Asp Ser Gly Ile Lys Ile Lys Gln Tyr Leu Met His Tyr
   450 455 460
   Thr Ser Lys Glu Asp Trp Ile Glu His Phe Gly Ser Lys Trp Asp Asp
   465 470 475 480
   Phe Ser Lys Arg Lys Asp Leu Phe Asp Pro Lys Lys Leu Leu Ser Pro
   485 490 495
   Gly Gln Asp Ile Phe
   500
<210> 3
   <211> 524
   <212> PRT
   <213> Arabidopsis thaliana
<400> 3
   Met Thr Asn Thr Leu Cys Leu Ser Leu Ile Thr Leu Ile Thr Leu Phe
   1               5                   10                  15
   Ile Ser Leu Thr Pro Thr Leu Ile Lys Ser Asp Glu Gly Ile Asp Val
               20                  25                  30
   Phe Leu Pro Ile Ser Leu Asn Leu Thr Val Leu Thr Asp Pro Phe Ser
           35                  40                  45
   Ile Ser Ala Ala Ser His Asp Phe Gly Asn Ile Thr Asp Glu Asn Pro
       50                  55                  60
   Gly Ala Val Leu Cys Pro Ser Ser Thr Thr Glu Val Ala Arg Leu Leu
   65                  70                  75                  80
   Arg Phe Ala Asn Gly Gly Phe Ser Tyr Asn Lys Gly Ser Thr Ser Pro
                   85                  90                  95
   Ala Ser Thr Phe Lys Val Ala Ala Arg Gly Gln Gly His Ser Leu Arg
               100                 105                 110
   Gly Gln Ala Ser Ala Pro Gly Gly Val Val Val Asn Met Thr Cys Leu
           115                 120                 125
   Ala Met Ala Ala Lys Pro Ala Ala Val Val Ile Ser Ala Asp Gly Thr
       130                 135                 140
   Tyr Ala Asp Val Ala Ala Gly Thr Met Trp Val Asp Val Leu Lys Ala
   145                 150                 155                 160
   Ala Val Asp Arg Gly Val Ser Pro Val Thr Trp Thr Asp Tyr Leu Tyr
                   165                 170                 175
   Leu Ser Val Gly Gly Thr Leu Ser Asn Ala Gly Ile Gly Gly Gln Thr
               180                 185                 190
   Phe Arg His Gly Pro Gln Ile Ser Asn Val His Glu Leu Asp Val Ile
           195                 200                 205
   Thr Gly Lys Gly Glu Met Met Thr Cys Ser Pro Lys Leu Asn Pro Glu
       210                 215                 220
   Leu Phe Tyr Gly Val Leu Gly Gly Leu Gly Gln Phe Gly Ile Ile Thr
   225                 230                 235                 240
   Arg Ala Arg Ile Ala Leu Asp His Ala Pro Thr Arg Val Lys Trp Ser
                   245                 250                 255
   Arg Ile Leu Tyr Ser Asp Phe Ser Ala Phe Lys Arg Asp Gln Glu Arg
               260                 265                 270
   Leu Ile Ser Met Thr Asn Asp Leu Gly Val Asp Phe Leu Glu Gly Gln
           275                 280                 285
   Leu Met Met Ser Asn Gly Phe Val Asp Thr Ser Phe Phe Pro Leu Ser
       290                 295                 300
   Asp Gln Thr Arg Val Ala Ser Leu Val Asn Asp His Arg Ile Ile Tyr
   305                 310                 315                 320
   Val Leu Glu Val Ala Lys Tyr Tyr Asp Arg Thr Thr Leu Pro Ile Ile
                   325                 330                 335
   Asp Gln Val Ile Asp Thr Leu Ser Arg Thr Leu Gly Phe Ala Pro Gly
               340                 345                 350
   Phe Met Phe Val Gln Asp Val Pro Tyr Phe Asp Phe Leu Asn Arg Val
           355                 360                 365
   Arg Asn Glu Glu Asp Lys Leu Arg Ser Leu Gly Leu Trp Glu Val Pro
       370                 375                 380
   His Pro Trp Leu Asn Ile Phe Val Pro Gly Ser Arg Ile Gln Asp Phe
   385                 390                 395                 400
   His Asp Gly Val Ile Asn Gly Leu Leu Leu Asn Gln Thr Ser Thr Ser
                   405                 410                 415
   Gly Val Thr Leu Phe Tyr Pro Thr Asn Arg Asn Lys Trp Asn Asn Arg
               420                 425                 430
   Met Ser Thr Met Thr Pro Asp Glu Asp Val Phe Tyr Val Ile Gly Leu
           435                 440                 445
   Leu Gln Ser Ala Gly Gly Ser Gln Asn Trp Gln Glu Leu Glu Asn Leu
       450                 455                 460
   Asn Asp Lys Val Ile Gln Phe Cys Glu Asn Ser Gly Ile Lys Ile Lys
   465                 470                 475                 480
   Glu Tyr Leu Met His Tyr Thr Arg Lys Glu Asp Trp Val Lys His Phe
                   485                 490                 495
   Gly Pro Lys Trp Asp Asp Phe Leu Arg Lys Lys Ile Met Phe Asp Pro
               500                 505                 510
   Lys Arg Leu Leu Ser Pro Gly Gln Asp Ile Phe Asn
           515                 520
<210> 4
   <211> 540
   <212> PRT
   <213> Arabidopsis thaliana
<400> 4
   Met Asn Arg Glu Met Thr Ser Ser Phe Leu Leu Leu Thr Phe Ala Ile
   1               5                   10                  15
   Cys Lys Leu Ile Ile Ala Val Gly Leu Asn Val Gly Pro Ser Glu Leu
               20                  25                  30
   Leu Arg Ile Gly Ala Ile Asp Val Asp Gly His Phe Thr Val His Pro
           35                  40                  45
   Ser Asp Leu Ala Ser Val Ser Ser Asp Phe Gly Met Leu Lys Ser Pro
       50                  55                  60
   Glu Glu Pro Leu Ala Val Leu His Pro Ser Ser Ala Glu Asp Val Ala
   65                  70                  75                  80
   Arg Leu Val Arg Thr Ala Tyr Gly Ser Ala Thr Ala Phe Pro Val Ser
                   85                  90                  95
   Ala Arg Gly His Gly His Ser Ile Asn Gly Gln Ala Ala Ala Gly Arg
               100                 105                 110
   Asn Gly Val Val Val Glu Met Asn His Gly Val Thr Gly Thr Pro Lys
           115                 120                 125
   Pro Leu Val Arg Pro Asp Glu Met Tyr Val Asp Val Trp Gly Gly Glu
       130                 135                 140
   Leu Trp Val Asp Val Leu Lys Lys Thr Leu Glu His Gly Leu Ala Pro
   145                 150                 155                 160
   Lys Ser Trp Thr Asp Tyr Leu Tyr Leu Thr Val Gly Gly Thr Leu Ser
                   165                 170                 175
   Asn Ala Gly Ile Ser Gly Gln Ala Phe His His Gly Pro Gln Ile Ser
               180                 185                 190
   Asn Val Leu Glu Leu Asp Val Val Thr Gly Lys Gly Glu Val Met Arg
           195                 200                 205
   Cys Ser Glu Glu Glu Asn Thr Arg Leu Phe His Gly Val Leu Gly Gly
       210                 215                 220
   Leu Gly Gln Phe Gly Ile Ile Thr Arg Ala Arg Ile Ser Leu Glu Pro
   225                 230                 235                 240
   Ala Pro Gln Arg Val Arg Trp Ile Arg Val Leu Tyr Ser Ser Phe Lys
                   245                 250                 255
   Val Phe Thr Glu Asp Gln Glu Tyr Leu Ile Ser Met His Gly Gln Leu
               260                 265                 270
   Lys Phe Asp Tyr Val Glu Gly Phe Val Ile Val Asp Glu Gly Leu Val
           275                 280                 285
   Asn Asn Trp Arg Ser Ser Phe Phe Ser Pro Arg Asn Pro Val Lys Ile
       290                 295                 300
   Ser Ser Val Ser Ser Asn Gly Ser Val Leu Tyr Cys Leu Glu Ile Thr
   305                 310                 315                 320
   Lys Asn Tyr His Asp Ser Asp Ser Glu Ile Val Asp Gln Glu Val Glu
                   325                 330                 335
   Ile Leu Met Lys Lys Leu Asn Phe Ile Pro Thr Ser Val Phe Thr Thr
               340                 345                 350
   Asp Leu Gln Tyr Val Asp Phe Leu Asp Arg Val His Lys Ala Glu Leu
           355                 360                 365
   Lys Leu Arg Ser Lys Asn Leu Trp Glu Val Pro His Pro Trp Leu Asn
       370                 375                 380
   Leu Phe Val Pro Lys Ser Arg Ile Ser Asp Phe Asp Lys Gly Val Phe
   385                 390                 395                 400
   Lys Gly Ile Leu Gly Asn Lys Thr Ser Gly Pro Ile Leu Ile Tyr Pro
                   405                 410                 415
   Met Asn Lys Asp Lys Trp Asp Glu Arg Ser Ser Ala Val Thr Pro Asp
               420                 425                 430
   Glu Glu Val Phe Tyr Leu Val Ala Leu Leu Arg Ser Ala Leu Thr Asp
           435                 440                 445
   Gly Glu Glu Thr Gln Lys Leu Glu Tyr Leu Lys Asp Gln Asn Arg Arg
       450                 455                 460
   Ile Leu Glu Phe Cys Glu Gln Ala Lys Ile Asn Val Lys Gln Tyr Leu
   465                 470                 475                 480
   Pro His His Ala Thr Gln Glu Glu Trp Val Ala His Phe Gly Asp Lys
                   485                 490                 495
   Trp Asp Arg Phe Arg Ser Leu Lys Ala Glu Phe Asp Pro Arg His Ile
               500                 505                 510
   Leu Ala Thr Gly Gln Arg Ile Phe Gln Asn Pro Ser Leu Ser Leu Phe
           515                 520                 525
   Pro Pro Ser Ser Ser Ser Ser Ser Ala Ala Ser Trp
       530                 535                 540
<210> 5
   <211> 533
   <212> PRT
   <213> Arabidopsis thaliana
<400> 5
   Met Ser Tyr Leu His Ala Ser Leu Leu Arg Lys Arg Thr Met Leu Ile
   1               5                   10                  15
   Val Arg Ser Phe Thr Ile Leu Leu Leu Ser Cys Ile Ala Phe Lys Leu
               20                  25                  30
   Ala Cys Cys Phe Ser Ser Ser Ile Ser Ser Leu Lys Ala Leu Pro Leu
               35                  40                  45
   Val Gly His Leu Glu Phe Glu His Val His His Ala Ser Lys Asp Phe
           50                  55                  60
   Gly Asn Arg Tyr Gln Leu Ile Pro Leu Ala Val Leu His Pro Lys Ser
   65                  70                  75                  80
   Val Ser Asp Ile Ala Ser Thr Ile Arg His Ile Trp Met Met Gly Thr
                   85                  90                  95
   His Ser Gln Leu Thr Val Ala Ala Arg Gly Arg Gly His Ser Leu Gln
               100                 105                 110
   Gly Gln Ala Gln Thr Arg His Gly Ile Val Ile His Met Glu Ser Leu
           115                 120                 125
   His Pro Gln Lys Leu Gln Val Tyr Ser Val Asp Ser Pro Ala Pro Tyr
       130                 135                 140
   Val Asp Val Ser Gly Gly Glu Leu Trp Ile Asn Ile Leu His Glu Thr
   145                 150                 155                 160
   Leu Lys Tyr Gly Leu Ala Pro Lys Ser Trp Thr Asp Tyr Leu His Leu
                   165                 170                 175
   Thr Val Gly Gly Thr Leu Ser Asn Ala Gly Ile Ser Gly Gln Ala Phe
               180                 185                 190
   Arg His Gly Pro Gln Ile Ser Asn Val His Gln Leu Glu Ile Val Thr
           195                 200                 205
   Gly Lys Gly Glu Ile Leu Asn Cys Thr Lys Arg Gln Asn Ser Asp Leu
       210                 215                 220
   Phe Asn Gly Val Leu Gly Gly Leu Gly Gln Phe Gly Ile Ile Thr Arg
   225                 230                 235                 240
   Ala Arg Ile Ala Leu Glu Pro Ala Pro Thr Met Val Lys Trp Ile Arg
                   245                 250                 255
   Val Leu Tyr Leu Asp Phe Ala Ala Phe Ala Lys Asp Gln Glu Gln Leu
               260                 265                 270
   Ile Ser Ala Gln Gly His Lys Phe Asp Tyr Ile Glu Gly Phe Val Ile
           275                 280                 285
   Ile Asn Arg Thr Gly Leu Leu Asn Ser Trp Arg Leu Ser Phe Thr Ala
       290                 295                 300
   Glu Glu Pro Leu Glu Ala Ser Gln Phe Lys Phe Asp Gly Arg Thr Leu
   305 310 315 320
   Tyr Cys Leu Glu Leu Ala Lys Tyr Leu Lys Gln Asp Asn Lys Asp Val
   325 330 335
   Ile Asn Gln Glu Val Lys Glu Thr Leu Ser Glu Leu Ser Tyr Val Thr
   340 345 350
   Ser Thr Leu Phe Thr Thr Glu Val Ala Tyr Glu Ala Phe Leu Asp Arg
   355 360 365
   Val His Val Ser Glu Val Lys Leu Arg Ser Lys Gly Gln Trp Glu Val
   370 375 380
   Pro His Pro Trp Leu Asn Leu Leu Val Pro Arg Ser Lys Ile Asn Glu
   385 390 395 400
   Phe Ala Arg Gly Val Phe Gly Asn Ile Leu Thr Asp Thr Ser Asn Gly
   405 410 415
   Pro Val Ile Val Tyr Pro Val Asn Lys Ser Lys Trp Asp Asn Gln Thr
   420 425 430
   Ser Ala Val Thr Pro Glu Glu Glu Val Phe Tyr Leu Val Ala Ile Leu
   435 440 445
   Thr Ser Ala Ser Pro Gly Ser Ala Gly Lys Asp Gly Val Glu Glu Ile
   450 455 460
   Leu Arg Arg Asn Arg Arg Ile Leu Glu Phe Ser Glu Glu Ala Gly Ile
   465 470 475 480
   Gly Leu Lys Gln Tyr Leu Pro His Tyr Thr Thr Arg Glu Glu Trp Arg
   485 490 495
   Ser His Phe Gly Asp Lys Trp Gly Glu Phe Val Arg Arg Lys Ser Arg
   500 505 510
   Tyr Asp Pro Leu Ala Ile Leu Ala Pro Gly His Arg Ile Phe Gln Lys
   515 520 525
   Ala Val Ser Tyr Ser
   530
<210> 6
   <211> 524
   <212> PRT
   <213> Arabidopsis thaliana
<400> 6
   Met Ile Ala Tyr Ile Glu Pro Tyr Phe Leu Glu Asn Asp Ala Glu Ala
   1               5                   10                  15
   Ala Ser Ala Ala Thr Ala Ala Gly Lys Ser Thr Asp Gly Val Ser Glu
               20                  25                  30
   Ser Leu Asn Ile Gln Gly Glu Ile Leu Cys Gly Gly Ala Ala Ala Asp
           35                  40                  45
   Ile Ala Gly Arg Asp Phe Gly Gly Met Asn Cys Val Lys Pro Leu Ala
       50                  55                  60
   Val Val Arg Pro Val Gly Pro Glu Asp Ile Ala Gly Ala Val Lys Ala
   65                  70                  75                  80
   Ala Leu Arg Ser Asp Lys Leu Thr Val Ala Ala Arg Gly Asn Gly His
                   85                  90                  95
   Ser Ile Asn Gly Gln Ala Met Ala Glu Gly Gly Leu Val Val Asp Met
               100                 105                 110
   Ser Thr Thr Ala Glu Asn His Phe Glu Val Gly Tyr Leu Ser Gly Gly
           115                 120                 125
   Asp Ala Thr Ala Phe Val Asp Val Ser Gly Gly Ala Leu Trp Glu Asp
       130                 135                 140
   Val Leu Lys Arg Cys Val Ser Glu Tyr Gly Leu Ala Pro Arg Ser Trp
   145                 150                 155                 160
   Thr Asp Tyr Leu Gly Leu Thr Val Gly Gly Thr Leu Ser Asn Ala Gly
                   165                 170                 175
   Val Ser Gly Gln Ala Phe Arg Tyr Gly Pro Gln Thr Ser Asn Val Thr
               180                 185                 190
   Glu Leu Asp Val Val Thr Gly Asn Gly Asp Val Val Thr Cys Ser Glu
           195                 200                 205
   Ile Glu Asn Ser Glu Leu Phe Phe Ser Val Leu Gly Gly Leu Gly Gln
       210                 215                 220
   Phe Gly Ile Ile Thr Arg Ala Arg Val Leu Leu Gln Pro Ala Pro Asp
   225                 230                 235                 240
   Met Val Arg Trp Ile Arg Val Val Tyr Thr Glu Phe Asp Glu Phe Thr
   245 250 255
   Gln Asp Ala Glu Trp Leu Val Ser Gln Lys Asn Glu Ser Ser Phe Asp
   260 265 270
   Tyr Val Glu Gly Phe Val Phe Val Asn Gly Ala Asp Pro Val Asn Gly
   275 280 285
   Trp Pro Thr Val Pro Leu His Pro Asp His Glu Phe Asp Pro Thr Arg
   290 295 300
   Leu Pro Gln Ser Cys Gly Ser Val Leu Tyr Cys Leu Glu Leu Gly Leu
   305 310 315 320
   His Tyr Arg Asp Ser Asp Ser Asn Ser Thr Ile Asp Lys Arg Val Glu
   325 330 335
   Arg Leu Ile Gly Arg Leu Arg Phe Asn Glu Gly Leu Arg Phe Glu Val
   340 345 350
   Asp Leu Pro Tyr Val Asp Phe Leu Leu Arg Val Lys Arg Ser Glu Glu
   355 360 365
   Ile Ala Lys Glu Asn Gly Thr Trp Glu Thr Pro His Pro Trp Leu Asn
   370 375 380
   Leu Phe Val Ser Lys Arg Asp Ile Gly Asp Phe Asn Arg Thr Val Phe
   385 390 395 400
   Lys Glu Leu Val Lys Asn Gly Val Asn Gly Pro Met Leu Val Tyr Pro
   405 410 415
   Leu Leu Arg Ser Arg Trp Asp Asp Arg Thr Ser Val Val Ile Pro Glu
   420 425 430
   Glu Gly Glu Ile Phe Tyr Ile Val Ala Leu Leu Arg Phe Val Pro Pro
   435 440 445
   Cys Ala Lys Val Ser Ser Val Glu Lys Met Val Ala Gln Asn Gln Glu
   450 455 460
   Ile Val His Trp Cys Val Lys Asn Gly Ile Asp Tyr Lys Leu Tyr Leu
   465 470 475 480
   Pro His Tyr Lys Ser Gln Glu Glu Trp Ile Arg His Phe Gly Asn Arg
   485 490 495
   Trp Ser Arg Phe Val Asp Arg Lys Ala Met Phe Asp Pro Met Ala Ile
   500 505 510
   Leu Ser Pro Gly Gln Lys Ile Phe Asn Arg Ser Leu
   515 520
<210> 7
   <211> 3342
   <212> DNA
   <213> Arabidopsis thaliana
<400> 7
   atggcgagtt ataatcttcg ttcacaagtt cgtcttatag caataacaat agtaatcatc     60
   attactctct caactccgat cacaaccaac acatcaccac aaccatggaa tatcctttca    120
   cacaacgaat tcgccggaaa actcacctcc tcctcctcct ccgtcgaatc agccgccaca    180
   gatttcggcc acgtcaccaa aatcttccct tccgccgtct taatcccttc ctccgttgaa    240
   gacatcacag atctcataaa actctctttt gactctcaac tgtcttttcc tttagccgct    300
   cgtggtcacg gacacagcca ccgtggccaa gcctcggcta aagacggagt tgtggtcaac    360
   atgcggtcca tggtaaaccg ggatcgaggt atcaaggtgt ctaggacctg tttatatgtt    420
   gacgtggacg ctgcgtggct atggattgag gtgttgaata aaactttgga gttagggtta    480
   acgccggttt cttggacgga ttatttgtat ttaacagtcg gtgggacgtt atcaaacggc    540
   ggaattagtg gacaaacgtt tcggtacggt ccacagatca ctaatgttct agagatggat    600
   gttattactg gtacgtacca cgatcttttt cacacagaga ttaaaaaaaa cagtaatagt    660
   gattttaact tcgtacgttt ctgatagaca acaaagaact tcgtacgttt ttcgaagttt    720
   tttcgtcttt ttcattttag atctgcgcgg ccatttttgg ttatgctatt gtttgtttgt    780
   attgtttgtc tctgtttatt tatttctcga acttgttgat agcttttctt cttttcacac    840
   atcaatctaa tcaccttttt tggtcttaag attagaaaga agatacggac taggtaaaaa    900
   taggtggttg taaacgtaga cgcattaaaa aaatattggt ttttttattt tttgataagc    960
   aaaattggtg gttggtctaa gattataaac ttgatattaa tgcaaaggtc gatctagcaa   1020
   tagaagatta atcaatattc ttggtgtttt aacaacagat tatttcatca ttaaaatcgt   1080
   gaaacaaaga aattttggta gtatacatta cgtgtagttt tgttagttta ttaaaaaaaa   1140
   tagtatatag ttttgttaaa acgcgattta tttagtaaca cattagtata ttacacgttt   1200
   aaccaactaa actttttttt ttgaataatt atgttctata tttcttactc aaattatgca   1260
   aatttcgtgg attcgaagtc aaatttctgc gaaatttaca tggtcatata ttataaaact   1320
   gttcatataa cccggtgaac aaacagacaa ttaagggttt gaatggttac ggcggttggg   1380
   gcggacacaa ccgtcaatag atcagaccgt tttttattta ccattcatca attatattcc   1440
   gcagtggttt ggggtaaaaa aaatagaaga aaaccgcagc ggaccaattc cataccgttt   1500
   ttacatacaa ataaacatgg tgcgcaacgg tttattgtcc gcctcaaaaa tgaaatggac   1560
   taaaccgcag ataaattaga ccgctttgtc cgctgcctcc attcatagac taaaaaaaaa   1620
   caaccaaaaa aaaaatggtc ccacgcccat gattttacac gaggtttctt gtggcgtaag 1680
   gacaaaactc aaaagttcat aacgtttggt cctaaccagg tgtaatggat taagtaacag 1740
   tcaattttct tattatagct gtatccatta tgtccacata tgcatccata tacattacac 1800
   tgttggtctc aagtgtagtt agattacgaa gactttcaag ttccattttt tggttaggag 1860
   ataaacataa tttaatgata ccgactttag cactctaggc tcaaaacaag tacagaagag 1920
   aatagtttta tttcaaactc gttgcattgt tgtatcaatt aattgtgtta gtctttgtat 1980
   attcttacat aacggtccaa gtttgttgaa atagtttact tactaaactt ttcctaatgg 2040
   ggtcaaattt tattttatag gaaaaggaga gattgcaact tgttccaagg acatgaactc 2100
   ggatcttttc ttcgcggtgt taggaggttt gggtcaattc ggcattataa caagagccag 2160
   aattaaactt gaagtagctc cgaaaagggt atgttaaatt tgtaaattat gcaactacag 2220
   aaaattctat gaaatttatg aatgaacata tatgcatttt tggatttttg taggccaagt 2280
   ggttaaggtt tctatacata gatttctccg aattcacaag agatcaagaa cgagtgatat 2340
   cgaaaacgga cggtgtagat ttcttagaag gttccattat ggtggaccat ggcccaccgg 2400
   ataactggag atccacgtat tatccaccgt ccgatcactt gaggatcgcc tcaatggtca 2460
   aacgacatcg tgtcatctac tgccttgaag tcgtcaagta ttacgacgaa acttctcaat 2520
   acacagtcaa cgaggtccgt acatacatac aatcataaat catacatgta taattgggag 2580
   atctttatgc attattcaat tatattaatt tactttagtt atttaactta tgcaggaaat 2640
   ggaggagtta agcgatagtt taaaccatgt aagagggttt atgtacgaga aagatgtgac 2700
   gtatatggat ttcctaaacc gagttcgaac cggagagcta aacctgaaat ccaaaggcca 2760
   atgggatgtt ccacatccat ggcttaatct cttcgtacca aaaactcaaa tctccaaatt 2820
   tgatgatggt gtttttaagg gtattatcct aagaaataac atcactagcg gtcctgttct 2880
   tgtttatcct atgaatcgca acaagtaagt ttaactcgat attgcaaaat ttactatcta 2940
   cattttcgtt ttggaatccg aaatattctt acaagctaat tttatgcggc gtttttaggt 3000
   ggaatgatcg gatgtctgcc gctatacccg aggaagatgt attttatgcg gtagggtttt 3060
   taagatccgc gggttttgac aattgggagg cttttgatca agaaaacatg gaaatactga 3120
   agttttgtga ggatgctaat atgggggtta tacaatatct tccttatcat tcatcacaag 3180
   aaggatgggt tagacatttt ggtccgaggt ggaatatttt cgtagagaga aaatataaat 3240
   atgatcccaa aatgatatta tcaccgggac aaaatatatt tcaaaaaata aactcgagtt 3300
   agacgataat taatcctatt gttagtcgtt ctcaccactt tg 3342
<210> 8
   <211> 2991
   <212> DNA
   <213> Arabidopsis thaliana
<400> 8
   atggctaatc ttcgtttaat gatcacttta atcacggttt taatgatcac caaatcatca     60
   aacggtatta aaattgattt acctaaatcc cttaacctca ccctctctac cgatccttcc    120
   atcatctccg cagcctctca tgacttcgga aacataacca ccgtgacccc cggcggcgta    180
   atctgcccct cctccaccgc tgatatctct cgtctcctcc aatacgccgc aaacggaaaa    240
   agtacattcc aagtagcggc tcgtggccaa ggccactcct taaacggcca agcctcggtc    300
   tccggcggag taatcgtcaa catgacgtgt atcactgacg tggtggtttc aaaagacaag    360
   aagtacgctg acgtggcggc cgggacgtta tgggtggatg tgcttaagaa gacggcggag    420
   aaaggggtgt cgccggtttc ttggacggat tatttgcata taaccgtcgg aggaacgttg    480
   tcgaatggtg gaattggtgg tcaagtgttt cgaaacggtc ctcttgttag taacgtcctt    540
   gaattggacg ttattactgg tacgcatctt ctaaactttg atgtacatac aacaacaaaa    600
   actgtttttg ttttatagta tttttcattt tttgtaccat aggttttatg ttttatagtt    660
   gtgctaaact tcttgcacca cacgtaagtc ttcgaaacac aaaatgcgta acgcatctat    720
   atgttttttg tacatattga atgttgttca tgagaaataa agtaattaca tatacacaca     780
   tttattgtcg tacatatata aataattaaa gacaaatttt cacaattggt agcgtgttaa    840
   tttgggattt ttgtaatgta catgcatgac gcatgcatat ggagcttttc ggttttctta    900
   gatttgtgta gtatttcaaa tatatcattt attttctttc gaataaagag gtggtatatt    960
   tttaaaatag caacatttca gaatttttct ttgaatttac actttttaaa ttgttattgt   1020
   taatatggat tttgaataaa taatttcagg gaaaggtgaa atgttgacat gctcgcgaca   1080
   gctaaaccca gaattgttct atggagtgtt aggaggtttg ggtcaatttg gaattataac   1140
   gagagccaga attgttttgg accatgcacc taaacgggta cgtatcatca tattttacca   1200
   tttgttttag tcagcattca tttttcatta gtaattccgt ttcaatttct aaattttttt   1260
   agtcaataga aaatgattct tatgtcagag cttgattatt tagtgatttt tattgagata   1320
   aaataaaata taacctaacg gaaataatta ttttactaat cggataatgt ctgattaaaa   1380
   cattttatga tattacacta agagagttag agacgtatgg atcacaaaac atgaagcttt   1440
   cttagatggt atcctaaaac taaagttagg tacaagtttg gaatttaggt caaatgctta   1500
   agttgcatta atttgaacaa aatctatgca ttgaataaaa aaaagatatg gattatttta   1560
   taaagtatag tccttgtaat cctaggactt gttgtctaat cttgtcttat gcgtgcaaat   1620
   ctttttgatg tcaatatata atccttgttt attagagtca agctctttca ttagtcaact   1680
   actcaaatat actccaaagt ttagaatata gtcttctgac taattagaat cttacaaccg   1740
   ataaacgtta caatttggtt atcattttaa aaaacagatt tggtcataat atacgatgac   1800
   gttctgtttt agtttcatct attcacaaat tttatataat tattttcaag aaaatattga   1860
   aatactatac tgtaatatgg tttctttata tatgtgtgta taaattaaat gggattgttt   1920
   tctctaaatg aaattgtgta ggccaaatgg tttcggatgc tctacagtga tttcacaact 1980
   tttacaaagg accaagaacg tttgatatca atggcaaacg atattggagt cgactattta 2040
   gaaggtcaaa tatttctatc aaacggtgtc gttgacacct cttttttccc accttcagat 2100
   caatctaaag tcgctgatct agtcaagcaa cacggtatca tctatgttct tgaagtagcc 2160
   aagtattatg atgatcccaa tctccccatc atcagcaagg tactacacat ttacattttc 2220
   atcatcgttt ttatcatacc ataagatatt taaatgattc atcattgcac cacattaaga 2280
   tattcatcat catcatcgtt acattttttt ttgcatctta tgcttctcat aatctactat 2340
   tgtgtaggtt attgacacat taacgaaaac attaagttac ttgcccgggt tcatatcaat 2400
   gcacgacgtg gcctacttcg atttcttgaa ccgtgtacat gtcgaagaaa ataaactcag 2460
   atctttggga ttatgggaac ttcctcatcc ttggcttaac ctctacgttc ctaaatctcg 2520
   gattctcgat tttcataacg gtgttgtcaa agacattctt cttaagcaaa aatcagcttc 2580
   gggactcgct cttctctatc caacaaaccg gaataagtac atacttctct tcattcatat 2640
   ttatcttcaa gaaccaaagt aaataaattt ctatgaactg attatgctgt tattgttaga 2700
   tgggacaatc gtatgtcggc gatgatacca gagatcgatg aagatgttat atatattatc 2760
   ggactactac aatccgctac cccaaaggat cttccagaag tggagagcgt taacgagaag 2820
   ataattaggt tttgcaagga ttcaggtatt aagattaagc aatatctaat gcattatact 2880
   agtaaagaag attggattga gcattttgga tcaaaatggg atgatttttc gaagaggaaa 2940
   gatctatttg atcccaagaa actgttatct ccagggcaag acatcttttg a 2991
<210> 9
   <211> 2782
   <212> DNA
   <213> Arabidopsis thaliana
<400> 9
   atgactaata ctctctgttt aagcctcatc accctaataa cgctttttat aagtttaacc     60
   ccaaccttaa tcaaatcaga tgagggcatt gatgttttct tacccatatc actcaacctt    120
   acggtcctaa ccgatccctt ctccatctct gccgcttctc acgacttcgg taacataacc    180
   gacgaaaatc ccggcgccgt cctctgccct tcctccacca cggaggtggc tcgtctcctc    240
   cgtttcgcta acggaggatt ctcttacaat aaaggctcaa ccagccccgc gtctactttc    300
   aaagtggctg ctcgaggcca aggccactcc ctccgtggcc aagcctctgc acccggaggt    360
   gtcgtcgtga acatgacgtg tctcgccatg gcggctaaac cagcggcggt tgttatctcg    420
   gcagacggga cttacgctga cgtggctgcc gggacgatgt gggtggatgt tctgaaggcg    480
   gcggtggata gaggcgtctc gccggttaca tggacggatt atttgtatct cagcgtcggc    540
   gggacgttgt cgaacgctgg aatcggtggt cagacgttta gacacggccc tcagattagt    600
   aacgttcatg agcttgacgt tattaccggt acgtaaatac caaaacttca ctaatctcgt    660
   tacaattttt taattttttg gtaatataaa ttttgtacgg ctcaactctt aattaagaat 720
   gaaacagtat ctatgatctt ctagatgctc tttttttgtc tgcaagcttt aattgtagta 780
   acatcagcga tatatatatc acatgcatgt gtattattga tgataatata taatgtttta 840
   gttacaaatt tgattctcaa ggtaaaactc acacgccata accagtataa aactccaaaa 900
   atcacgtttt ggtcagaaat acatatcctt cattaacagt agttatgcta taatttgtga 960
   ttataaataa ctccggagtt tgttcacaat actaaatttc aggaaaaggt gaaatgatga 1020
   cttgctctcc aaagttaaac cctgaattgt tctatggagt tttaggaggt ttgggtcaat 1080
   tcggtattat aacgagggcc aggattgcgt tggatcatgc acccacaagg gtatgtatca 1140
   tgcatctata gtgtaatcaa tttataattt taatgtagtg gtcctaaatc caaaatttga 1200
   tttgatttgg ttggaacgta cgtatatata ataagtcaaa aggctgattt tgaagacgaa 1260
   tttatatact tttgttgaat taaatctgat tttgcttacg ttttattaga ttctgcgtaa 1320
   taaatcctag gacttgctcg agtgtaatct tgtcttatgc ttgcaaatct tgttgatgtc 1380
   aatatctaat cttttttatt atatttccct acgtaagttt tagatatagt tattttaaac 1440
   tgctataaat tgtgtacgta tagactttag ataaaaagtt gtggtcgctt gcacctattt 1500
   gtttatcgct atagtgattc aaaggtctat atatgattct tggtttttct ttttgaaaaa 1560
   aatagaccat acaatccaag gaagatgatc ttaaatggac taatttatgg atataaattg 1620
   atatacaaat ctgcaggtga aatggtctcg catactctac agtgacttct cggcttttaa 1680
   aagagaccaa gagcgtttaa tatcaatgac caatgatctc ggagttgact ttttggaagg 1740
   tcaacttatg atgtcaaatg gcttcgtaga cacctctttc ttcccactct ccgatcaaac 1800
   aagagtcgca tctcttgtga atgaccaccg gatcatctat gttctcgaag tagccaagta 1860
   ttatgacaga accacccttc ccattattga ccaggtacta aaatccatta ttcatgatga 1920
   ttatcttcac acaatcagta tcatcaccaa ttaccatcat cacttgtcat atatgatcca 1980
   aagtaaatat atcacatgat ataaataaat cgttcaaatc ttttttttta aagaataaaa 2040
   gaatcatttt caagcattac tcatacacat ctacgaatca ccgtgaccat atataaccat 2100
   acgcttatta aataatcatt tttgtttgta ggtgattgac acgttaagta gaactctagg 2160
   tttcgctcca gggtttatgt tcgtacaaga tgttccgtat ttcgatttct tgaaccgtgt 2220
   ccgaaacgaa gaagataaac tcagatcttt aggactatgg gaagttcctc atccatggct 2280
   taacatcttt gtcccggggt ctcgaatcca agattttcat gatggtgtta ttaatggcct 2340
   tcttctaaac caaacctcaa cttctggtgt tactctcttc tatcccacaa accgaaacaa 2400
   gtaaatattt actttttgat tttgttttat ttgaaagtat atcccaataa tgtatgttaa 2460
   attgttaaca agaatttatt ttattaatag atggaacaac cgcatgtcaa cgatgacacc 2520
   ggacgaagat gttttttatg tgatcggatt actgcaatca gctggtggat ctcaaaattg 2580
   gcaagaactt gaaaatctca acgacaaggt tattcagttt tgtgaaaact cgggaattaa 2640
   gattaaggaa tatttgatgc actatacaag aaaagaagat tgggttaaac attttggacc 2700
   aaaatgggat gattttttaa gaaagaaaat tatgtttgat cccaaaagac tattgtctcc 2760
   aggacaagac atatttaatt aa 2782
<210> 10
   <211> 2817
   <212> DNA
   <213> Arabidopsis thaliana
<400> 10
   atgaatcgtg aaatgacgtc aagctttctt ctcctgacgt tcgccatatg taaactgatc    60
   atagccgtgg gtctaaacgt gggccccagt gagctcctcc gcatcggagc catagatgtc   120
   gacggccact tcaccgtcca cccttccgac ttagcctccg tctcctcaga cttcggtatg   180
   ctgaagtcac ctgaagagcc attggccgtg cttcatccat catcggccga agacgtggca   240
   cgactcgtca gaacagctta cggttcagcc acggcgtttc cggtctcagc ccgaggccac   300
   ggccattcca taaacggaca agccgcggcg gggaggaacg gtgtggtggt tgaaatgaac   360
   cacggcgtaa ccgggacgcc caagccactc gtccgaccgg atgaaatgta tgtggatgta   420
   tggggtggag agttatgggt cgatgtgttg aagaaaacgt tggagcatgg cttagcacca   480
   aaatcatgga cggattactt gtatctaacc gttggaggta cactctccaa tgcaggaatc   540
   agtggtcaag cttttcacca tggtcctcaa attagtaacg tccttgagct cgacgttgta   600
   actggttagt attaaaacat tcaagttcat atattttaaa tgcttttgtc tgaagtttta   660
   ctaataacaa gaaattgata ccaaaaagta gggaaaggag aggtgatgag atgctcagaa   720
   gaagagaaca caaggctatt ccatggagtt cttggtggat taggtcaatt tgggatcatc   780
   actcgagcac gaatctctct cgaaccagct ccccaaaggg taatattttt ttaatgacta   840
   gctatcaaaa atccctggcg ggtccatacg ttgtaatctt tttagttttt actgttgatg   900
   gtatttttta tatattttgg ataataaaac cctaaaatgg tatattgtga tgacaggtga   960
   gatggatacg ggtattgtat tcgagcttca aagtgtttac ggaggaccaa gagtacttaa  1020
   tctcaatgca tggtcaatta aagtttgatt acgtggaagg ttttgtgatt gtggacgaag  1080
   gactcgtcaa caattggaga tcttctttct tctctccacg taaccccgtc aagatctcct  1140
   ctgttagttc caacggctct gttttgtatt gccttgagat caccaagaac taccacgact  1200
   ccgactccga aatcgttgat caggtcactt tcattattca cttagaaaaa agcgatattt  1260
   tcatttttta tattgatgaa tatctggaag gatttaacgc tatgcgacta ttgggaaatc   1320
   attatgaaaa aatatttagt ttatatgatt gaaagtggtc tccatagtat ttttgttgtg  1380
   tcgactttat tataacttaa atttggaaga ggacatgaag aagaagccag agaggatcta  1440
   cagagatcta gcttttccac ctgaacttaa taatgcacat ttatataatt atttttcttc  1500
   ttctaaagtt tagtttatca ctagcgaatt aatcatggtt actaattaag tagtggacag  1560
   ggtcatggac cactcactca ccaaataatg attcctcttt actcttaagt ttaattttaa   1620
   taaaaccaac tctactggaa tcttaactta tccttggttt tggtaggctt ttatagcaac   1680
   acggtttttt taattttcct attccagatt ttgtatatta aatgtcgatt tttttctttt   1740
   ttgtttcagg aagttgagat tctgatgaag aaattgaatt tcataccgac atcggtcttt   1800
   acaacggatt tacaatatgt ggactttctc gaccgggtac acaaggccga attgaagctc   1860
   cggtccaaga atttatggga ggttccacac ccatggctca acctcttcgt gccaaaatca   1920
   agaatctctg acttcgataa aggcgttttc aagggcattt tgggaaataa aacaagtggc   1980
   cctattctta tctaccccat gaacaaagac aagtaagtct tgacattacc attgattact   2040
   acttctaaat ttcttctcta gaaaaaagaa taaaacgagt tttgcattgc atgcatgcaa   2100
   agttacactt gtggggatta attagtggtc caagaaaaaa agtttgtcaa aattgaaaaa   2160
   aactagacac gtggtacatg ggattgtccg aaaaacgttg tccacatgtg catcgaacca   2220
   gctaagattg acaacaacac ttcgtcggct cgtatttctc tttttgtttt gtgaccaaat   2280
   ccgatggtcc agattgggtt tatttgtttt taagttccta gaactcatgg tgggtgggtc   2340
   ccaatcagat tctcctagac caaaccgatc tcaacgaacc ctccgcacat cattgattat   2400
   tacattaata tagatattgt cgttgctgac gtgtcgtaat ttgatgttat tgtcagatgg   2460
   gacgagagga gctcagccgt gacgccggat gaggaagttt tctatctggt ggctctattg   2520
   agatcagctt taacggacgg tgaagagaca cagaagctag agtatctgaa agatcagaac   2580
   cgtcggatct tggagttctg tgaacaagcc aagatcaatg tgaagcagta tcttcctcac   2640
   cacgcaacac aggaagagtg ggtggctcat tttggggaca agtgggatcg gttcagaagc   2700
   ttaaaggctg agtttgatcc gcgacacata ctcgctactg gtcagagaat ctttcaaaac   2760
   ccatctttgt ctttgtttcc tccgtcgtcg tcttcttcgt cagcggcttc atggtga      2817
<210> 11
   <211> 1975
   <212> DNA
   <213> Arabidopsis thaliana
<400> 11
   atgagctatc tacatgcaag cctcctcagg aaaagaacca tgcttatagt aagaagtttc     60
   accatcttgc ttctcagctg catagccttt aagttggctt gctgcttctc tagcagcatt    120
   tcttctttga aggcgcttcc cctagtaggc catttggagt ttgaacatgt ccatcacgcc    180
   tccaaagatt ttggaaatcg ataccagttg atccctttgg cggtcttaca tcccaaatcg    240
   gtaagcgaca tcgcctcaac gatacgacac atctggatga tgggcactca ttcacagctt    300
   acagtggcag cgagaggtcg tggacattca ctccaaggcc aagctcaaac aagacatgga    360
   attgttatac acatggaatc actccatccc cagaagctgc aggtctacag tgtggattcc    420
   cctgctccat atgttgatgt gtctggtggt gagctgtgga taaacatttt gcatgagacc    480
   ctcaagtacg ggcttgcacc aaaatcatgg acggattacc tgcatttaac tgtaggtggt     540
   actctgtcca atgctggaat aagcggccag gcattccgac atggaccaca gatcagcaat     600
   gttcatcaac tggagattgt cacaggttag ttcagagttg cagtattcgt gttttgaaag     660
   catagactct atatggttgg tgactattaa caacatgaag agattcccga gaatagctac     720
   ccactaatgt catgcctatt tattgactgc aggaaaaggc gagatcctaa actgtacaaa     780
   gaggcagaac agcgacttat ttaatggtgt tcttggtggt ttaggtcagt ttggcatcat     840
   aacgcgggca agaatagcat tggaaccagc accaaccatg gtaaacaata aataaataaa     900
   aaacttaaaa actgaacacg cgtgtgtcct cctaactctg tataatggac aggtaaaatg     960
   gataagagtg ttatacctgg attttgcagc ttttgccaag gaccaagagc aactaatatc    1020
   tgcccagggc cacaaattcg attacataga agggtttgtg ataataaaca ggacaggcct    1080
   cctgaacagc tggaggttgt ctttcaccgc agaagagcct ttagaagcaa gccaattcaa    1140
   gtttgatgga aggactctgt attgtctgga gctagccaag tatttgaagc aagataacaa    1200
   agacgtaatc aaccaggtga gaaaacagag tagaagcaat cggtagaatc ttctttggta    1260
   gatgacattc attggaactg aaaatatata tatatttgtc caatccagga agtgaaagaa    1320
   acattatcag agctaagcta cgtgacgtcg acactgttta caacggaggt agcatatgaa    1380
   gcattcttgg acaggtacta tgtgtctgag gtaaaactcc gatcgaaagg gcagtgggag    1440
   gtgccacatc catggctgaa cctcctggta ccaagaagca aaatcaatga atttgcaaga    1500
   ggtgtatttg gaaacatact aacggataca agcaacggcc cagtcatcgt ctacccagtg    1560
   aacaaatcaa agtaagaaag aaagaaagaa agagctagtc atgattttgt ttcttttcac    1620
   ttgttgacaa aacaaaagca tgttggtgag caggtgggac aatcaaacat cagcagtaac    1680
   accggaggaa gaggtattct acctggtggc gatcctaaca tcggcatctc cagggtcggc    1740
   aggaaaggat ggagtagaag agatcttgag gcggaacaga agaatactgg aattcagtga    1800
   agaagcaggg atagggttga agcagtatct gccacattac acgacaagag aagagtggag    1860
   atcccatttc ggggacaagt ggggagaatt tgtgaggagg aaatccagat atgatccatt    1920
   ggcaattctt gcgcctggcc accgaatttt tcaaaaggca gtctcatact catga         1975
<210> 12
   <211> 3211
   <212> DNA
   <213> Arabidopsis thaliana
<400> 12
   atgatagctt acatagaacc atacttcttg gaaaacgacg ccgaggctgc ctctgccgcc     60
   accgccgccg gaaaatctac ggatggtgtt tctgagtcac ttaacatcca aggagaaatc    120
   ttatgtggtg gagctgcggc ggatatcgcc gggagagatt ttggcggcat gaactgtgtg    180
   aagcctcttg ctgtggtgag accagtggga ccggaagata tcgccggagc ggtgaaagcg    240
   gctctgaggt cagataaact aacggtggcg gcgcgtggaa acggccattc tatcaacggt 300
   caagccatgg cggaaggagg actcgttgtc gatatgagta ccacggcgga gaatcatttc 360
   gaggttggtt atttatccgg cggtgatgcc acggcgtttg ttgatgtctc cggaggggca 420
   ttatgggaag atgtattgaa acggtgcgtt tcggagtacg gtttggctcc gaggtcttgg 480
   actgattatc ttgggttaac ggtgggaggt acgttgtcaa atgccggcgt tagtggtcaa 540
   gcgttccgtt acggaccaca gacgtcaaat gtaacggagt tggacgtcgt tacgggaaat 600
   ggtgacgtcg ttacttgctc ggagattgag aattcagagc tattcttctc tgttttaggt 660
   ggtcttggtc agtttggtat catcaccaga gctagggttt tgctacagcc agctcctgat 720
   atggtgaata cttaaaacca acaatataaa taacaatctc agttatatat atatatattt 780
   tctctaatcc aatcaaaaat aagatatttg gtccataata taaatgattg ttgtgttagg 840
   tgagatggat aagagtagta tacaccgagt tcgatgagtt cactcaagac gccgagtggc 900
   tagtaagtca gaagaacgag tcatcgttcg attacgtgga aggattcgtg tttgtcaacg 960
   gtgctgaccc ggttaacgga tggccaacag ttcccctcca cccggaccac gagtttgacc 1020
   cgacccgact accacaatct tgcgggtcgg ttctttattg cctcgaactc ggtcttcact 1080
   acagagactc cgattccaac tcaaccattg acaaggtaat aataactttg agaaacttta 1140
   taacattttt cagaaattca agaaccgttc atcttttatg atctaacggc ggtggaagat 1200
   tctgatgttc tagaaacttt gtttgaccga aattgacctt agattgaagt gtgaagttga 1260
   cccgttttat ttcactaact gttatacgac acgtagttca tataggaccg ttttcagatt 1320
   tctcgacctc catgattaca caaacataca attcaaaaaa cagtaaaaag atgataataa 1380
   taataatata tggtttagtt aaggaaatat aatagggtgg gaaagggaat tatacagtct 1440
   cttgtctgac tgcataatat gaaactgacg agacattgtg taatgtatct tcgattttgg 1500
   attgtctgac atgaaaaaaa tatttatttg cttctctcta atgcccttgt cgtaaccacg 1560
   ttattacgaa aaggacattt gtcttcgttt tctttttctt ttcttttttt ttgttgcttt 1620
   tgttgtcttt ctcatgaaac acatatttta agatcacttt gcctttttct actcaattat 1680
   ttagattaaa ccaacacgtg tgacgtgtcc attggtcgtg cggtatggga cgtaaggttg 1740
   aaatcgtaat tgtagcatgt aaacgtttct gtagtaaaac attgatgata tgatttcaaa 1800
   cggtcccggc taaaatctgg ccatcgtttt atatggaatc atctatgtat gtaccgaaat 1860
   accccctgac tgattttttt ccattttttt gtgtagaggg tggagagatt gatcggacgg 1920
   ctaagattta atgaaggatt aagattcgag gtagatctgc cgtacgttga ctttttacta 1980
   cgagtcaaac ggtcagaaga aatcgcgaag gagaacggta cgtgggaaac gcctcaccct 2040
   tggctcaacc tcttcgtgtc gaagcgagac atcggagatt tcaatcggac ggtgttcaaa 2100
   gaacttgtca agaacggagt caatggtcca atgcttgtgt acccactctt gcgaagcagg 2160
   tgaatattgc tctctcttcc tctcttttaa ctaggaccca tctttttatt ttgggttaga   2220
   caaggcacct atctaaaaga ctaaaagaag atcggtctag tttagtttta tggcatgtgt   2280
   ttatcacgtg tgatgattag tcgtgcatgc ttaaactaaa aaaaggctcc acaagtcgca   2340
   agtcgtgtga tcaacaaatt gtcgccaatg tggcacacgt gtctttcttc agtcccctcg   2400
   tcattttttt tacccgtacg gggttttaag tacaataaaa gttggaattt agtgtggtcg   2460
   tttagatttt gtaggcgaga taaaaaaaag aatactaaac taatacgatg ccgtattagg   2520
   tattacggtt gggtgggtga cggatatgta ttgtaaccgt cgttaaggtt agcgtcatat   2580
   agggaaagag atgaaatttg tagggaccca atatgaacta acgttaaatt ttttatttta   2640
   catttctaaa atagcctttt gtaggttact gaagggtagt ttcgtcttta tatgttttac   2700
   tttatgtgga aaatgagatt tgctggtaca aatagtagac gtaagaaatg aaaccaatcg   2760
   tgagcaaagg gccaccaaaa tgtttatttt ttattctccg atttttttat ggaaaatgtc   2820
   ttttgttcca tttagattta gtgggtattt gttttataat atgaatgatt aaataataat   2880
   ttggttggtt tttatcaggt gggatgatcg gacgtccgtg gttataccgg aagaaggaga   2940
   gatattctac attgtggcat tgcttcggtt cgtgccgccg tgtgcgaaag tctcttcggt   3000
   agagaaaatg gtagctcaaa accaagagat cgttcattgg tgtgtcaaaa acggaattga   3060
   ttacaaattg tatcttcctc attacaagtc tcaagaggaa tggattcgcc attttggaaa   3120
   ccgatggtcg agatttgttg ataggaaagc tatgtttgat cccatggcta tactttcacc   3180
   gggtcaaaag attttcaata ggtctctttg a                                  3211
<210> 13
   <211> 575
   <212> PRT
   <213> Arabidopsis thaliana
<400> 13
   Met Gly Leu Thr Ser Ser Leu Arg Phe His Arg Gln Asn Asn Lys Thr
   1               5                   10                  15
   Phe Leu Gly Ile Phe Met Ile Leu Val Leu Ser Cys Ile Pro Gly Arg
               20                  25                  30
   Thr Asn Leu Cys Ser Asn His Ser Val Ser Thr Pro Lys Glu Leu Pro
           35                  40                  45
   Ser Ser Asn Pro Ser Asp Ile Arg Ser Ser Leu Val Ser Leu Asp Leu
       50                  55                  60
   Glu Gly Tyr Ile Ser Phe Asp Asp Val His Asn Val Ala Lys Asp Phe
   65                  70                  75                  80
   Gly Asn Arg Tyr Gln Leu Pro Pro Leu Ala Ile Leu His Pro Arg Ser
                   85                  90                  95
   Val Phe Asp Ile Ser Ser Met Met Lys His Ile Val His Leu Gly Ser
               100                 105                 110
   Thr Ser Asn Leu Thr Val Ala Ala Arg Gly His Gly His Ser Leu Gln
           115                 120                 125
   Gly Gln Ala Leu Ala His Gln Gly Val Val Ile Lys Met Glu Ser Leu
       130                 135                 140
   Arg Ser Pro Asp Ile Arg Ile Tyr Lys Gly Lys Gln Pro Tyr Val Asp
   145                 150                 155                 160
   Val Ser Gly Gly Glu Ile Trp Ile Asn Ile Leu Arg Glu Thr Leu Lys
                   165                 170                 175
   Tyr Gly Leu Ser Pro Lys Ser Trp Thr Asp Tyr Leu His Leu Thr Val
               180                 185                 190
   Gly Gly Thr Leu Ser Asn Ala Gly Ile Ser Gly Gln Ala Phe Lys His
           195                 200                 205
   Gly Pro Gln Ile Asn Asn Val Tyr Gln Leu Glu Ile Val Thr Gly Lys
       210                 215                 220
   Gly Glu Val Val Thr Cys Ser Glu Lys Arg Asn Ser Glu Leu Phe Phe
   225                 230                 235                 240
   Ser Val Leu Gly Gly Leu Gly Gln Phe Gly Ile Ile Thr Arg Ala Arg
                   245                 250                 255
   Ile Ser Leu Glu Pro Ala Pro His Met Val Lys Trp Ile Arg Val Leu
               260                 265                 270
   Tyr Ser Asp Phe Ser Ala Phe Ser Arg Asp Gln Glu Tyr Leu Ile Ser
           275                 280                 285
   Lys Glu Lys Thr Phe Asp Tyr Val Glu Gly Phe Val Ile Ile Asn Arg
       290                 295                 300
   Thr Asp Leu Leu Asn Asn Trp Arg Ser Ser Phe Ser Pro Asn Asp Ser
   305                 310                 315                 320
   Thr Gln Ala Ser Arg Phe Lys Ser Asp Gly Lys Thr Leu Tyr Cys Leu
                   325                 330                 335
   Glu Val Val Lys Tyr Phe Asn Pro Glu Glu Ala Ser Ser Met Asp Gln
               340                 345                 350
   Glu Thr Gly Lys Leu Leu Ser Glu Leu Asn Tyr Ile Pro Ser Thr Leu
   355 360 365
   Phe Ser Ser Glu Val Pro Tyr Ile Glu Phe Leu Asp Arg Val His Ile
   370 375 380
   Ala Glu Arg Lys Leu Arg Ala Lys Gly Leu Trp Glu Val Pro His Pro
   385 390 395 400
   Trp Leu Asn Leu Leu Ile Pro Lys Ser Ser Ile Tyr Gln Phe Ala Thr
   405 410 415
   Glu Val Phe Asn Asn Ile Leu Thr Ser Asn Asn Asn Gly Pro Ile Leu
   420 425 430
   Ile Tyr Pro Val Asn Gln Ser Lys Trp Lys Lys His Thr Ser Leu Ile
   435 440 445
   Thr Pro Asn Glu Asp Ile Phe Tyr Leu Val Ala Phe Leu Pro Ser Ala
   450 455 460
   Val Pro Asn Ser Ser Gly Lys Asn Asp Leu Glu Tyr Leu Leu Lys Gln
   465 470 475 480
   Asn Gln Arg Val Met Asn Phe Cys Ala Ala Ala Asn Leu Asn Val Lys
   485 490 495
   Gln Tyr Leu Pro His Tyr Glu Thr Gln Lys Glu Trp Lys Ser His Phe
   500 505 510
   Gly Lys Arg Trp Glu Thr Phe Ala Gln Arg Lys Gln Ala Tyr Asp Pro
   515 520 525
   Leu Ala Ile Leu Ala Pro Gly Gln Arg Ile Phe Gln Lys Thr Thr Gly
   530 535 540
   Lys Leu Ser Pro Ile Gln Leu Ala Lys Ser Lys Ala Thr Gly Ser Pro
   545 550 555 560
   Gln Arg Tyr His Tyr Ala Ser Ile Leu Pro Lys Pro Arg Thr Val
   565 570 575
<210> 14
   <211> 2236
   <212> DNA
   <213> Arabidopsis thaliana
<400> 14
   atgggattga cctcatcctt acggttccat agacaaaaca acaagacttt cctcggaatc 60
   ttcatgatct tggttctaag ctgtatacca ggtagaacca atctttgttc caatcattct 120
   gttagtaccc caaaagaatt accttcttca aatccttcag atattcgttc ctcattagtt 180
   tcactagatt tggagggtta tataagcttc gacgatgtcc acaatgtggc caaggacttt 240
   ggcaacagat accagttacc acctttggca attctacatc caaggtcagt ttttgatatt 300
   tcatcgatga tgaagcatat agtacatctg ggctccacct caaatcttac agtagcagct 360
   agaggccatg gtcactcgct tcaaggacaa gctctagctc atcaaggtgt tgtcatcaaa 420
   atggagtcac ttcgaagtcc tgatatcagg atttataagg ggaagcaacc atatgttgat 480
   gtctcaggtg gtgaaatatg gataaacatt ctacgcgaga ctctaaaata cggtctttca 540
   ccaaagtcct ggacagacta ccttcatttg accgttggag gtacactatc taatgctgga 600
   atcagcggtc aagcattcaa gcatggaccc caaatcaaca acgtctacca gctagagatt 660
   gttacaggta tttcattcat gctttatctc tgcggtagtc tcaaaaaaat atgcacctgt 720
   aaagaatatc catctcttca tgagcaaaaa cactgacgac tttaaataat ttttgactat 780
   aaaacaagag tgcataggca caaatgtgaa atatgcaaca cacaattgta acttgcacca 840
   agaaaaaagt tataaaaaca aacaactgat aagcaatata tttccaatat ttaatcaggg 900
   aaaggagaag tcgtaacctg ttctgagaag cggaattctg aacttttctt cagtgttctt 960
   ggcgggcttg gacagtttgg cataatcacc cgggcacgga tctctcttga accagcaccg 1020
   catatggtaa agttctatct tgaacaaagt tcaaacaata tacgctatga ttctaagaac 1080
   cactttcctg acacagtcaa ataactttta ataggttaaa tggatcaggg tactctactc 1140
   tgacttttct gcattttcaa gggaccaaga atatctgatt tcgaaggaga aaacttttga 1200
   ttacgttgaa ggatttgtga taatcaatag aacagacctt ctcaataatt ggcgatcgtc 1260
   attcagtccc aacgattcca cacaggcaag cagattcaag tcagatggga aaactcttta 1320
   ttgcctagaa gtggtcaaat atttcaaccc agaagaagct agctctatgg atcaggtaag 1380
   atgtgaaagc aatatataac tagacttagt ttccacagag agctccaaat caaccgttgg 1440
   ctactagcct actaacataa tgaatggttg ccgtgcagga aactggcaag ttactttcag 1500
   agttaaatta tattccatcc actttgtttt catctgaagt gccatatatc gagtttctgg 1560
   atcgcgtgca tatcgcagag agaaaactaa gagcaaaggg tttatgggag gttccacatc 1620
   cctggctgaa tctcctgatt cctaagagca gcatatacca atttgctaca gaagttttca 1680
   acaacattct cacaagcaac aacaacggtc ctatccttat ttatccagtc aatcaatcca 1740
   agtaagtgag caaaatgcca aaagcaaatg cgtccagtga ttctgaaaca taaattacta 1800
   accatatcca acattttgtg gtttcaggtg gaagaaacat acatctttga taactccaaa 1860
   tgaagatata ttctatctcg tagcctttct cccctctgca gtgccaaatt cctcagggaa 1920
   aaacgatcta gagtaccttt tgaaacaaaa ccaaagagtt atgaacttct gcgcagcagc 1980
   aaacctcaac gtgaagcagt atttgcccca ttatgaaact caaaaagagt ggaaatcaca 2040
   ctttggcaaa agatgggaaa catttgcaca gaggaaacaa gcctacgacc ctctagcgat 2100
   tctagcacct ggccaaagaa tattccaaaa gacaacagga aaattatctc ccatccaact 2160
   cgcaaagtca aaggcaacag gaagtcctca aaggtaccat tacgcatcaa tactgccgaa 2220
   acctagaact gtataa 2236
<210> 15
   <211> 20
   <212> DNA
   <213> artificial
<220>
   <223> primer
<400> 15
   gaatggtgga attggtggtc 20
<210> 16
   <211> 20
   <212> DNA
   <213> artificial
<220>
   <223> primer
<400> 16
   gcgagcatgt caacatttca 20
<210> 17
   <211> 22
   <212> DNA
   <213> artificial
<220>
   <223> primer
<400> 17
   tggttcacgt agtgggccat cg 22
<210> 18
   <211> 20
   <212> DNA
   <213> artificial
<220>
   <223> primer
<400> 18
   tcaaaagcct cccaattgtc 20
<210> 19
   <211> 20
   <212> DNA
   <213> artificial
<220>
   <223> primer
<400> 19
   ctcggctaaa gacggagttg 20
<210> 20
   <211> 22
   <212> DNA
   <213> artificial
<220>
   <223> primer
<400> 20
   tggttcacgt agtgggccat cg 22
<210> 21
   <211> 26
   <212> DNA
   <213> artificial
<220>
   <223> primer
<400> 21
   ctctgccgct tctcacgact tcggta 26
<210> 22
   <211> 26
   <212> DNA
   <213> artificial
<220>
   <223> primer
<400> 22
   cataaaccct ggagcgaaac ctagag 26
<210> 23
   <211> 22
   <212> DNA
   <213> artificial
<220>
   <223> primer
<400> 23
   tggttcacgt agtgggccat cg 22
<210> 24
   <211> 26
   <212> DNA
   <213> artificial
<220>
   <223> primer
<400> 24
   caaggtaaaa ctcacacgcc ataacc 26
<210> 25
   <211> 26
   <212> DNA
   <213> artificial
<220>
   <223> primer
<400> 25
   cataaaccct ggagcgaaac ctagag 26
<210> 26
   <211> 26
   <212> DNA
   <213> artificial
<220>
   <223> primer
<400> 26
   gagcgtcggt ccccacactt ctatac 26
<210> 27
   <211> 29
   <212> DNA
   <213> artificial
<220>
   <223> primer
<400> 27
   ttgttgcagc aacgaccaac cgataatga 29
<210> 28
   <211> 29
   <212> DNA
   <213> artificial
<220>
   <223> primer
<400> 28
   aatggtatat tgtgatgaca ggtgagatg 29
<210> 29
   <211> 22
   <212> DNA
   <213> artificial
<220>
   <223> primer
<400> 29
   tggttcacgt agtgggccat cg 22
<210> 30
   <211> 29
   <212> DNA
   <213> artificial
<220>
   <223> primer
<400> 30
   aatggtatat tgtgatgaca ggtgagatg 29
<210> 31
   <211> 29
   <212> DNA
   <213> artificial
<220>
   <223> primer
<400> 31
   ttgttgcagc aacgaccaac cgataatga 29
<210> 32
   <211> 23
   <212> DNA
   <213> artificial
<220>
   <223> primer
<400> 32
   atattgacca tcatactcat tgc 23
<210> 33
   <211> 21
   <212> DNA
   <213> artificial
<220>
   <223> primer
<400> 33
   accctgtcca agaatgcttc a 21
<210> 34
   <211> 21
   <212> DNA
   <213> artificial
<220>
   <223> primer
<400> 34
   tgtggattcc cctgctccat a 21
<210> 35
   <211> 22
   <212> DNA
   <213> artificial
<220>
   <223> primer
<400> 35
   tggttcacgt agtgggccat cg 22
<210> 36
   <211> 20
   <212> DNA
   <213> artificial
<220>
   <223> primer
<400> 36
   ttagccgtcc gatcaatctc 20
<210> 37
   <211> 20
   <212> DNA
   <213> artificial
<220>
   <223> primer
<400> 37
   cggaaaatct acggatggtg 20
<210> 38
   <211> 23
   <212> DNA
   <213> artificial
<220>
   <223> primer
<400> 38
   atattgacca tcatactcat tgc 23
<210> 39
   <211> 27
   <212> DNA
   <213> artificial
<220>
   <223> primer
<400> 39
   gctagtaagt cagaagaacg agtcatc 27
<210> 40
   <211> 20
   <212> DNA
   <213> artificial
<220>
   <223> primer
<400> 40
   ttagccgtcc gatcaatctc 20
<210> 41
   <211> 34
   <212> DNA
   <213> artificial
<220>
   <223> primer
<400> 41
   gccttttcag aaatggataa atagccttgc ttcc 34
<210> 42
   <211> 20
   <212> DNA
   <213> artificial
<220>
   <223> primer
<400> 42
   gaatggtgga attggtggtc 20
<210> 43
   <211> 20
   <212> DNA
   <213> artificial
<220>
   <223> primer
<400> 43
   agtcccgaag ctgatttttg 20
<210> 44
   <211> 20
   <212> DNA
   <213> artificial
<220>
   <223> primer
<400> 44
   ctcggctaaa gacggagttg 20
<210> 45
   <211> 26
   <212> DNA
   <213> artificial
<220>
   <223> primer
<400> 45
   aataggtggt tgtaaacgta gacgca 26
<210> 46
   <211> 26
   <212> DNA
   <213> artificial
<220>
   <223> primer
<400> 46
   ctctgccgct tctcacgact tcggta 26
<210> 47
   <211> 26
   <212> DNA
   <213> artificial
<220>
   <223> primer
<400> 47
   cataaaccct ggagcgaaac ctagag 26
<210> 48
   <211> 26
   <212> DNA
   <213> artificial
<220>
   <223> primer
<400> 48
   ctctgccgct tctcacgact tcggta 26
<210> 49
   <211> 26
   <212> DNA
   <213> artificial
<220>
   <223> primer
<400> 49
   cataaaccct ggagcgaaac ctagag 26
<210> 50
   <211> 20
   <212> DNA
   <213> artificial
<220>
   <223> primer
<400> 50
   gcacgaatct ctctcgaacc 20
<210> 51
   <211> 19
   <212> DNA
   <213> artificial
<220>
   <223> primer
<400> 51
   cgctgacgaa gaagacgac 19
<210> 52
   <211> 20
   <212> DNA
   <213> artificial
<220>
   <223> primer
<400> 52
   gcacgaatct ctctcgaacc 20
<210> 53
   <211> 20
   <212> DNA
   <213> artificial
<220>
   <223> primer
<400> 53
   aaattcttgg accggagctt 20
<210> 54
   <211> 21
   <212> DNA
   <213> artificial
<220>
   <223> primer
<400> 54
   tgtggattcc cctgctccat a 21
<210> 55
   <211> 21
   <212> DNA
   <213> artificial
<220>
   <223> primer
<400> 55
   accctgtcca agaatgcttc a 21
<210> 56
   <211> 20
   <212> DNA
   <213> artificial
<220>
   <223> primer
<400> 56
   ttagccgtcc gatcaatctc 20
<210> 57
   <211> 20
   <212> DNA
   <213> artificial
<220>
   <223> primer
<400> 57
   cggaaaatct acggatggtg 20
<210> 58
   <211> 20
   <212> DNA
   <213> artificial
<220>
   <223> primer
<400> 58
   ttagccgtcc gatcaatctc 20
<210> 59
   <211> 20
   <212> DNA
   <213> artificial
<220>
   <223> primer
<400> 59
   cggaaaatct acggatggtg 20
<210> 60
   <211> 20
   <212> DNA
   <213> artificial
<220>
   <223> primer
<400> 60
   tacaacgagc ttcgtgttgc 20
<210> 61
   <211> 20
   <212> DNA
   <213> artificial
<220>
   <223> primer
<400> 61
   gattgatcct ccgatccaga 20

## Description

In order to be able to supply a continuously growing population with food and other plant-derived products, people have always been interested in improving the productivity in agriculture.

The productivity of a plant can be influenced in various different ways, e.g. by improving plant growth characteristics or by delaying leaf senescence. There are many mechanisms and pathways known which are involved in plant growth and development.

Cytokinin is a plant hormone that plays positive and negative regulatory roles in many aspects of plant growth and development. It stimulates the formation and activity of shoot meristems, is able to establish sink tissues, retard leaf senescence, inhibits root growth and branching, and plays a role in seed germination and stress responses (Mok, D. W. S. & Mok, M. C. (2001) Ann. Rev. Plant Physiol. Mol. Bio. 52, 89-1 18). Analysis of cytokinin-deficient plants has shown that cytokinin plays opposite roles in shoot and root meristems and suggests that the hormone has an essential function in quantitative control of organ growth (Werner T, Motyka V, Laucou V, Smets R, Van Onckelen H, Schmulling T, Plant Cell 2003,15(11):2532-50; Werner T, Motyka V, Strnad M, Schmülling T, Proc Natl Acad Sci U S A 2001, 98(18):10487-92).

Cytokinin oxidases/dehydrogenases (CKX) are an important factor to regulate the homeostasis of the plant hormone cytokinin. The genome of Arabidopsis encodes seven *CKX* genes, which have distinct expression domains (Werner et al., 2001; Werner et al., 2003). The CKX proteins differ in their subcellular localization and biochemical features (Werner et al., 2003). Overexpression of individual *CKX* genes established cytokinin-deficient plants and revealed that cytokinin is a positive regulator of the shoot meristem activity and a negative regulator of root meristem activity.

Recently it was shown that in a rice plant inhibition of the function of a particular CKX gene, the rice orthologue to CKX3 of *Arabidopsis thaliana,* has led to an increase in particle-bearing number of said rice plant (see US 2006/0123507 A1). Although these results are promising, there remains a need for further improving the productivity of plants.

It is an object of the present invention to provide means and methods suitable to produce transgenic plants with improved productivity and/or growth characteristics.

This object is achieved by the present invention as defined in the claims and as set out in detail below.

The present invention provides isolated plant cells and transgenic plants as defined in the claims in which the expression and/or activity of at least two different cytokininoxidase/dehydrogenase genes is inhibited by disruption compared to a control plant cell or a control plant lacking such disruptions, wherein the first cytokininoxidase/dehydrogenase gene is an endogenous gene encoding for CKX3 or an orthologue thereof and the second cytokininoxidase/dehydrogenase gene is an endogenous gene encoding for a cytokininoxidase/dehydrogenase and being different from CKX3 or the orthologue thereof.

Surprisingly it has been found that in a plant simultaneous disruption of the CKX3 gene and a second cytokininoxidase/dehydrogenase gene encoding for one of CKX2, CKX4, CKX5 or CKX6 leads to transgenic plants with a seed yield that is higher than that of a plant lacking such disruptions or transgenic plants where only one cytokininoxidase/dehydrogenase gene is disrupted. Whereas single disruption of CKX3 led to a slight (but not significant) increase in seed yield, as reported in US 2006/0123507 A1, single disruption of CKX5 had no measurable effect on seed yield.

Surprisingly the simultaneous disruption of CKX3 and one of CKX2, CKX4, CKX5 or CKX6, but not simultaneous disruption of CKX2 and CKX4 or CKX2 and CKX4 and CKX5 or CKX4 and CKX6 or CKX5 and CKX6, led to a significant increase in seed yield compared to wild type and single disruptions of CKX3 and CKX5. Most significant increase in seed yield was observed for a simultaneous disruption of CKX3 and CKX5. Even more surprisingly it was found that simultaneous disruption of CKX3 and one of CKX2, CKX4, CKX5 or CKX6, in particular of CKX3 and CKX5, led to transgenic plants with significantly improved plant height compared to wild-type plants and transgenic plants comprising single disruptions of CKX3 or CKX5. Thus, simultaneous disruption of at least CKX3 and one further endogenous gene encoding for a cytokininoxidase/dehydrogenase, preferably of CKX1, CKX2, CKX4, CKX5, CKX6 or CKX7 leads to transgenic plants with improved productivity and/or growth characteristics.

In a first aspect the present invention relates to an isolated plant cell as defined in the claims comprising a disruption in at least:
i) an endogenous CKX3 gene encoding for a cytokininoxidase/dehydrogenase comprising a polypeptide sequence being identical to or having at least 95% identity with SEQ ID No. 1 or an orthologue thereof;
   and
ii) one further endogenous gene encoding for a cytokininoxidase/dehydrogenase and being different from the gene defined in i);
wherein said disruptions inhibit expression and/or activity of a product of the at least two disrupted cytokininoxidase/dehydrogenase genes compared to a corresponding control plant cell lacking such disruptions.

In a second aspect, the present invention is directed to a transgenic plant as defined in the claims comprising a disruption in at least:
i) an endogenous CKX3 gene encoding for a cytokininoxidase/dehydrogenase comprising a polypeptide sequence being identical to or having at least 95% identity with SEQ ID No. 1 or an orthologue thereof; and
ii) one further endogenous gene encoding for a cytokininoxidase/dehydrogenase and being different from the gene defined in i);
wherein said disruptions inhibit expression and/or activity of a product of the at least two disrupted cytokininoxidase/dehydrogenase genes compared to a corresponding control plant lacking such disruptions. It is understood that for the purpose of the present invention the term "transgenic plant" not only encompasses the plant comprising the disruptions of the invention as such, but also refers to any progeny thereof irrespective of the generation No., i.e. the term "transgenic plant" covers progeny of first generation as well as progeny of the X^{th} generation, provided that said progeny still comprises the disruptions of the invention encompassed by the parent transgenic plant.

In a third aspect, the invention relates to a method as defined in the claims of increasing a seed yield in a plant and/or increasing plant height and/or increasing stem thickness relative to a corresponding control plant, the method comprising introducing in a plant a disruption in at least:
i) an endogenous CKX3 gene encoding for a cytokininoxidase/dehydrogenase comprising a polypeptide sequence being identical to or having at least 95% identity with SEQ ID No. 1 or an orthologue thereof;
   and
ii) one further endogenous gene encoding for a cytokininoxidase/dehydrogenase and being different from the gene defined in i);
wherein said disruptions inhibit expression and/or activity of a product of the at least two disrupted cytokininoxidase/dehydrogenase genes compared to a corresponding control plant lacking such disruptions.

In a fourth aspect, the present invention is directed to a method as defined in the claims for producing a plant with an increased seed yield and/or plant height relative to a corresponding control plant, comprising disrupting in a plant at least:
i) an endogenous CKX3 gene encoding for a cytokininoxidase/dehydrogenase comprising a polypeptide sequence being identical to or having at least 95% identity with SEQ ID No. 1 or an orthologue thereof;
   and
ii) one further endogenous gene encoding for a cytokininoxidase/dehydrogenase and being different from the gene defined in i);
wherein said disruptions inhibit expression and/or activity of a product of the at least two disrupted cytokininoxidase/dehydrogenase genes compared to a corresponding control plant lacking such disruptions.

The present invention also relates to an isolated plant cell as defined in the claims comprising a disruption in at least:
i) an endogenous CKX3 gene encoding for a cytokininoxidase/dehydrogenase comprising a polypeptide sequence being identical to or having at least 95% identity with SEQ ID No. 1 or an orthologue thereof, wherein the orthologue is an endogenous gene encoding for a cytokininoxidase/dehydrogenase comprising a polypeptide sequence with at least 80%, or at least 90% sequence identity to SEQ ID No. 1 over the whole length of SEQ ID No. 1;
   and
ii) in at least one further endogenous gene being:
   a) a CKX2 gene encoding for a cytokininoxidase/dehydrogenase comprising a polypeptide sequence being identical to or having at least 95% identity with SEQ ID No. 2 or an orthologue thereof, wherein the orthologue is an endogenous gene encoding for a cytokininoxidase/dehydrogenase comprising a polypeptide sequence with at least 80%, or at least 90% sequence identity to one of SEQ ID No. 2 over the whole length of SEQ ID No. 2;
   b) a CKX4 gene encoding for a cytokininoxidase/dehydrogenase comprising a polypeptide sequence being identical to or having at least 95% identity with SEQ ID No. 3 or an orthologue thereof, wherein the orthologue is an endogenous gene encoding for a cytokininoxidase/dehydrogenase comprising a polypeptide sequence with at least 80%, or at least 90% sequence identity to one of SEQ ID No. 3 over the whole length of SEQ ID No. 3;
   c) a CKX5 gene encoding for a cytokininoxidase/dehydrogenase comprising a polypeptide sequence being identical to or having at least 95% identity with SEQ ID No. 4 or an orthologue thereof, wherein the orthologue is an endogenous gene encoding for a cytokininoxidase/dehydrogenase comprising a polypeptide sequence with at least 80%, or at least 90% sequence identity to one of SEQ ID No. 4 over the whole length of SEQ ID No. 4;
      or
   d) a CKX6 gene encoding for a cytokininoxidase/dehydrogenase comprising a polypeptide sequence being identical to or having at least 95% identity with SEQ ID No. 5 or an orthologue thereof, wherein the orthologue is an endogenous gene encoding for a cytokininoxidase/dehydrogenase comprising a polypeptide sequence with at least 80%, or at least 90% sequence identity to one of SEQ ID No. 5 over the whole length of SEQ ID No. 5;
   wherein said disruptions inhibit expression and/or activity of a product of the at least two disrupted cytokininoxidase/dehydrogenase genes compared to a corresponding control plant cell lacking such disruptions.

The present invention also refers to a transgenic plant comprising a disruption in at least:
i) an endogenous CKX3 gene encoding for a cytokininoxidase/dehydrogenase comprising a polypeptide sequence being identical to or having at least 95% identity with SEQ ID No. 1 or an orthologue thereof, wherein the
   orthologue is an endogenous gene encoding for a cytokininoxidase/dehydrogenase comprising a polypeptide sequence with at least 80%, or at least 90% sequence identity to SEQ ID No. 1 over the whole length of SEQ ID No. 1;
   and
ii) in at least one further endogenous gene being:
   a) a CKX2 gene encoding for a cytokininoxidase/dehydrogenase comprising a polypeptide sequence being identical to or having at least 95% identity with SEQ ID No. 2 or an orthologue thereof, wherein the orthologue is an endogenous gene encoding for a cytokininoxidase/dehydrogenase comprising a polypeptide sequence with at least 80%, or at least 90% sequence identity to one of SEQ ID No. 2 over the whole length of SEQ ID No. 2;
   b) a CKX4 gene encoding for a cytokininoxidase/dehydrogenase comprising a polypeptide sequence being identical to or having at least 95% identity with SEQ ID No. 3 or an orthologue thereof, wherein the orthologue is an endogenous gene encoding for a
      cytokininoxidase/dehydrogenase comprising a polypeptide sequence with at least 80%, or at least 90% sequence identity to one of SEQ ID No. 3 over the whole length of SEQ ID No. 3;
   c) a CKX5 gene encoding for a cytokininoxidase/dehydrogenase comprising a polypeptide sequence being identical to or having at least 95% identity with SEQ ID No. 4 or an orthologue thereof, wherein the orthologue is an endogenous gene encoding for a cytokininoxidase/dehydrogenase comprising a polypeptide sequence with at least 80%, or at least 90% sequence identity to one of SEQ ID No. 4 over the whole length of SEQ ID No. 4;
      or
   d) a CKX6 gene encoding for a cytokininoxidase/dehydrogenase comprising a polypeptide sequence being identical to or having at least 95% identity with SEQ ID No. 5 or an orthologue thereof, wherein the orthologue is an endogenous gene encoding for a cytokininoxidase/dehydrogenase comprising a polypeptide sequence with at least 80%, or at least 90% sequence identity to one of SEQ ID No. 5 over the whole length of SEQ ID No. 5;
   wherein said disruptions inhibit expression and/or activity of a product of the at least two disrupted cytokininoxidase/dehydrogenase genes compared to a corresponding control plant lacking such disruptions.

The isolated plant cell of the invention and/or the transgenic plant of the invention can comprise a disruption in at least:
i) an endogenous CKX3 gene comprising a nucleic acid sequence being identical to or having at least 95% identity with SEQ ID No. 7 or an orthologue thereof, wherein the orthologue is a gene comprising a nucleic acid sequence with at least 80%, or at least 90% sequence identity to SEQ ID No. 7 over the whole length of SEQ ID No. 7;
   and
ii) in at least one further endogenous gene being:
   a) a CKX2 gene comprising a nucleic acid sequence being identical to or having at least 95% identity with SEQ ID No. 8 or an orthologue thereof, wherein the orthologue is an endogenous gene comprising a
      nucleic acid sequence with at least 80%, or at least 90% sequence identity to SEQ ID No. 8 over the whole length of SEQ ID No. 8;
   b) a CKX4 gene comprising a nucleic acid sequence being identical to or having at least 95% identity with SEQ ID No. 9 or an orthologue thereof, wherein the orthologue is an endogenous gene comprising a nucleic acid sequence with at least 80%, or at least 90% sequence identity to SEQ ID No. 9 over the whole length of SEQ ID No. 9;
   c) a CKX5 gene comprising a nucleic acid sequence being identical to or having at least 95% identity with SEQ ID No. 10 or an orthologue thereof, wherein the orthologue is an endogenous gene comprising a nucleic acid sequence with at least 80%, or at least 90% sequence identity to SEQ ID No. 10 over the whole length of SEQ ID No. 10;
      or
   d) a CKX6 gene comprising a nucleic acid sequence being identical to or having at least 95% identity with SEQ ID No. 11 or an orthologue thereof, wherein the orthologue is an endogenous gene comprising a nucleic acid sequence with at least 80%, or at least 90% sequence identity to SEQ ID No. 11 over the whole length of SEQ ID No. 11;
wherein said disruptions inhibit expression and/or activity of a product of the at least two disrupted cytokininoxidase/dehydrogenase genes compared to a corresponding control plant or control plant cell lacking such disruptions.

Preferably the isolated plant cell of the invention and/or the transgenic plant of the invention comprises a disruption in
i) at least an endogenous CKX3 gene encoding for a cytokininoxidase/dehydrogenase comprising a polypeptide sequence being identical to or having at least 95% identity with SEQ ID No. 1 or an orthologue thereof, wherein the orthologue is an endogenous gene encoding for a cytokininoxidase/dehydrogenase comprising a polypeptide sequence with at least 80%, or at least 90% sequence identity to SEQ ID No. 1 over the whole length of SEQ ID No. 1;
   and
ii) in an endogenous CKX5 gene encoding for a cytokininoxidase/dehydrogenase comprising a polypeptide sequence being identical to or having at least 95% identity with SEQ ID No. 4 or an orthologue thereof, wherein the orthologue is an endogenous gene encoding for a cytokininoxidase/dehydrogenase comprising a polypeptide sequence with at least 80%, or at least 90% sequence identity to SEQ ID No. 4 over the whole length of SEQ ID No. 4.

Preferably the isolated plant cell of the invention and/or the transgenic plant of the invention comprises a disruption in
i) an endogenous CKX3 gene comprising a nucleic acid sequence being identical to or having at least 95% identity with SEQ ID No. 7 or an orthologue thereof, wherein the orthologue is a gene comprising a nucleic acid sequence with at least 80%, or at least 90% sequence identity to SEQ ID No. 7 over the whole length of SEQ ID No. 7;
   and
ii) an endogenous CKX5 gene comprising a nucleic acid sequence being identical to or having at least 95% identity with SEQ ID No. 10 or an orthologue thereof, wherein the orthologue is an endogenous gene comprising a nucleic acid sequence with at least 80%, or at least 90% sequence identity to SEQ ID No. 10 over the whole length of SEQ ID No. 10.

In the isolated plant cell of the invention and/or in the transgenic plant of the invention, one, more than one or all disruptions of the invention may be facilitated by structural disruption, antisense polynucleotide gene suppression, double stranded RNA induced gene silencing, ribozyme techniques, genomic disruptions, tilling, and/or homologous recombination.

In the isolated plant cell of the invention and/or in the transgenic plant of the invention, one, more than one or all disruptions of the invention may be homozygous disruptions.

The transgenic plant of the invention is preferably selected from the family *Brassicaceae,* more preferably from the genera *Brassica* or *Arabidopsis.*

The present invention is also directed to a cell, organ, tissue or transgenic propagation material derived from a transgenic plant of the invention. Transgenic propagation material encompasses parts of a transgenic plant of the invention such as seeds, tubers, beets/swollen tap roots or fruits derived from a transgenic plant of the invention.

The present invention is also directed to a method of increasing a seed yield of a plant and/or increasing plant height relative to a corresponding control plant, the method comprising introducing in a plant a disruption in at least:
i) an endogenous CKX3 gene encoding for a cytokininoxidase/dehydrogenase comprising a polypeptide sequence being identical to or having at least 95% identity with SEQ ID No. 1 or an orthologue thereof, wherein the orthologue is an endogenous gene encoding for a cytokininoxidase/dehydrogenase comprising a polypeptide sequence with at least 80%, or at least 90% sequence identity to SEQ ID No. 1 over the whole length of SEQ ID No. 1;
   and
ii) in at least one further endogenous gene being:
   a) a CKX2 gene encoding for a cytokininoxidase/dehydrogenase comprising a polypeptide sequence being identical to or having at least 95% identity with SEQ ID No. 2 or an orthologue thereof, wherein the orthologue is an endogenous gene encoding for a cytokininoxidase/dehydrogenase comprising a polypeptide sequence with at least 80%, or at least 90% sequence identity to one of SEQ ID No. 2 over the whole length of SEQ ID No. 2;
   b) a CKX4 gene encoding for a cytokininoxidase/dehydrogenase comprising a polypeptide sequence being identical to or having at least 95% identity with SEQ ID No. 3 or an orthologue thereof, wherein the orthologue is an endogenous gene encoding for a cytokininoxidase/dehydrogenase comprising a polypeptide sequence with at least 80%, or at least 90% sequence identity to one of SEQ ID No. 3 over the whole length of SEQ ID No. 3;
   c) a CKX5 gene encoding for a cytokininoxidase/dehydrogenase comprising a polypeptide sequence being identical to or having at least 95% identity with SEQ ID No. 4 or an orthologue thereof, wherein the orthologue is an endogenous gene encoding for a cytokininoxidase/dehydrogenase comprising a polypeptide sequence with at least 80%, or at least 90% sequence identity to one of SEQ ID No. 4 ver the whole length of SEQ ID No. 4;
      or
   d) a CKX6 gene encoding for a cytokininoxidase/dehydrogenase comprising a polypeptide sequence being identical to or having at least 95% identity with SEQ ID No. 5 or an orthologue thereof, wherein the orthologue is an endogenous gene encoding for a cytokininoxidase/dehydrogenase comprising a polypeptide sequence with at least 80%, or at least 90% sequence identity to one of SEQ ID No. 5 over the whole length of SEQ ID No. 5;
wherein said disruptions inhibit expression and/or activity of a product of the at least two disrupted cytokininoxidase/dehydrogenase genes compared to a corresponding control plant lacking such disruptions.

In a further aspect the present invention is directed to a method for producing a plant, preferably a transgenic plant, with an increased seed yield and/or plant height relative to a corresponding control plant, comprising disrupting in a plant at least:
i) an endogenous CKX3 gene encoding for a cytokininoxidase/dehydrogenase comprising a polypeptide sequence being identical to or having at least 95% identity with SEQ ID No. 1 or an orthologue thereof, wherein the
   orthologue is an endogenous gene encoding for a cytokininoxidase/dehydrogenase comprising a polypeptide sequence with at least 80%, or at least 90% sequence identity to SEQ ID No. 1 over the whole length of SEQ ID No. 1;
   and
ii) in at least one further endogenous gene being:
   a) a CKX2 gene encoding for a cytokininoxidase/dehydrogenase comprising a polypeptide sequence being identical to or having at least 95% identity with SEQ ID No. 2 or an orthologue thereof, wherein the orthologue is an endogenous gene encoding for a cytokininoxidase/dehydrogenase comprising a polypeptide sequence with at least 80%, or at least 90% sequence identity to one of SEQ ID No. 2 over the whole length of SEQ ID No. 2;
   b) a CKX4 gene encoding for a cytokininoxidase/dehydrogenase comprising a polypeptide sequence being identical to or having at least 95% identity with SEQ ID No. 3 or an orthologue thereof, wherein the orthologue is an endogenous gene encoding for a
      cytokininoxidase/dehydrogenase comprising a polypeptide sequence with at least 80%, or at least 90% sequence identity to one of SEQ ID No. 3 over the whole length of SEQ ID No. 3;
   c) a CKX5 gene encoding for a cytokininoxidase/dehydrogenase comprising a polypeptide sequence being identical to or having at least 95% identity with SEQ ID No. 4 or an orthologue thereof, wherein the orthologue is an endogenous gene encoding for a cytokininoxidase/dehydrogenase comprising a polypeptide sequence with at least 80%, or at least 90% sequence identity to one of SEQ ID No. 4 over the whole length of SEQ ID No. 4;
      or
   d) a CKX6 gene encoding for a cytokininoxidase/dehydrogenase comprising a polypeptide sequence being identical to or having at least 95% identity with SEQ ID No. 5 or an orthologue thereof, wherein the orthologue is an endogenous gene encoding for a cytokininoxidase/dehydrogenase comprising a polypeptide sequence with at least 80%, or at least 90% sequence identity to one of SEQ ID No. 5 over the whole length of SEQ ID No. 5;
wherein said disruptions inhibit expression and/or activity of a product of the at least two disrupted cytokininoxidase/dehydrogenase genes compared to a corresponding control plant lacking such disruptions.

In the methods of the invention, preferably
i) at least an endogenous CKX3 gene encoding for a cytokininoxidase/dehydrogenase comprising a polypeptide sequence being identical to or having at least 95% identity with SEQ ID No. 1 or an orthologue thereof, wherein the orthologue is an endogenous gene encoding for a cytokininoxidase/dehydrogenase comprising a polypeptide sequence with at least 80%, or at least 90% sequence identity to SEQ ID No. 1 over the whole length of SEQ ID No. 1;
   and
ii) in an endogenous CKX5 gene encoding for a cytokininoxidase/dehydrogenase comprising a polypeptide sequence being identical to or having at least 95% identity with SEQ ID No. 4 or an orthologue thereof, wherein the orthologue is an endogenous gene encoding for a cytokininoxidase/dehydrogenase comprising a polypeptide sequence with at least 80%, or at least 90% sequence identity to SEQ ID No. 4 over the whole length of SEQ ID No. 4,
   can preferably be disrupted.

In the method of the invention, preferably:
i) an endogenous CKX3 gene comprising a nucleic acid sequence being identical to or having at least 95% identity with SEQ ID No. 7 or an orthologue thereof, wherein the orthologue is a gene comprising a nucleic acid sequence with at least 80%, or at least 90% sequence identity to SEQ ID No. 7 over the whole length of SEQ ID No. 7;
   and
ii) in at least one further endogenous gene being:
   a) a CKX2 gene comprising a nucleic acid sequence being identical to or having at least 95% identity with SEQ ID No. 8 or an orthologue thereof, wherein the orthologue is an endogenous gene comprising a nucleic acid sequence with at least 80%, or at least 90% sequence identity to SEQ ID No. 8 over the whole length of SEQ ID No. 8;
   b) a CKX4 gene comprising a nucleic acid sequence being identical to or having at least 95% identity with SEQ ID No. 9 or an orthologue thereof, wherein the orthologue is an endogenous gene comprising a nucleic acid sequence with at least 80%, or at least 90% sequence identity to SEQ ID No. 9 over the whole length of SEQ ID No. 9;
   c) a CKX5 gene comprising a nucleic acid sequence being identical to or having at least 95% identity with SEQ ID No. 10 or an orthologue thereof, wherein the orthologue is an endogenous gene comprising a nucleic acid sequence with at least 80%, or at least 90% sequence identity to SEQ ID No. 10 over the whole length of SEQ ID No. 10;
      or
   d) a CKX6 gene comprising a nucleic acid sequence being identical to or having at least 95% identity with SEQ ID No. 11 or an orthologue thereof, wherein the orthologue is an endogenous gene comprising a nucleic acid sequence with at least 80%, or at least 90% sequence identity to SEQ ID No. 11 over the whole length of SEQ ID No. 11;
are disrupted.

In another preferred method of the invention:
i) an endogenous CKX3 gene comprising a nucleic acid sequence being identical to or having at least 95% identity with SEQ ID No. 7 or an orthologue thereof, wherein the orthologue is a gene comprising a nucleic acid sequence with at least 80%, or at least 90% sequence identity to SEQ ID No. 7 over the whole length of SEQ ID No. 7;
   and
ii) an endogenous CKX5 gene comprising a nucleic acid sequence being identical to or having at least 95% identity with SEQ ID No. 10 or an orthologue thereof, wherein the orthologue is an endogenous gene comprising a nucleic acid sequence with at least 80%, or at least 90% sequence identity to SEQ ID No. 10 over the whole length of SEQ ID No. 10,
are disrupted.

In the methods of the invention, preferably one, more than one or all disruptions are homozygous disruptions.

The present disclosure is also directed to an isolated plant cell or a transgenic plant obtainable or obtained by one of the methods of the invention.

Also disclosed is that at least one of the disruptions in the isolated plant cell of the invention or in the transgenic plant of the invention is produced by introducing at least one polynucleotide sequence comprising a nucleic acid sequence which has at least about 70%, at least about 75%, at least about 80%, at least about 85%, at least about 90%, at least about 95%, at least about 99%, about 99.5% or more sequence identity to SEQ ID No. 14 (CKX1), SEQ ID No. 7 (CKX3), SEQ ID No. 8 (CKX2), SEQ ID No. 9 (CKX4), SEQ ID No. 10 (CKX5), SEQ ID No. 11 (CKX6), SEQ ID No. 12 (CKX7) or a subsequence thereof, or a complement thereof, into a plant cell, such that the at least one polynucleotide sequence is linked to a promoter in a sense or antisense orientation. In another embodiment, the disruption is introduced into the plant cell or the transgenic plant of the invention by introducing at least one polynucleotide sequence configured for RNA silencing or interference.

Also disclosed is that one, more than one or all disruptions in at least one of the above-mentioned endogenous genes comprise insertion of one or more transposons.

Also disclosed is that one, more than one or all disruptions can comprise one or more point mutations in at least one of the above-mentioned endogenous genes.

One, more than one or all disruptions in at least one of the above-mentioned endogenous genes can be homozygous disruptions. Alternatively, one, more than one or all disruptions in at least one of the above-mentioned endogenous genes can be a heterozygous disruption. The disruptions in at least one of the above-mentioned endogenous genes can include homozygous disruptions, heterozygous disruptions or a combination of homozygous disruptions and heterozygous disruptions.

Unless defined otherwise, all technical and scientific terms used herein have the same meaning as commonly understood by one of ordinary skill in the art to which the invention pertains. In describing and claiming the present invention, the following terminology will be used in accordance with the definitions set out below.

As used in this specification and the appended claims, the singular forms "a", "an" and "the" include singular and plural referents unless the content clearly dictates otherwise. Thus, for example, reference to "a cell" includes one cell and a combination of two or more cells, and the like.

The term "plant" refers generically to any of: whole plants, plant parts or organs (e. g. leaves, stems, roots, etc.), shoot vegetative organs/structures (e. g. leaves, stems and tubers), roots, flowers and floral organs/structures (e. g. bracts, sepals, petals, stamens, carpels, anthers and ovules), seed (including embryo, endosperm, and seed coat), fruit (the mature ovary), plant tissue (e. g. vascular tissue, ground tissue, and the like), tissue culture callus, and plant cells (e. g. guard cells, egg cells, trichomes and the like), and progeny of same. The term "plant" generally means all those organisms which are capable of photosynthesis. Included as plant within the scope of the invention are all genera and species of the higher and lower plants of the plant kingdom. Mature plants means plants at any developmental stage beyond the seedling. Seedling means a young immature plant in an early developmental stage. Annual, perennial, monocotyledonous and/or dicotyledonous plants are preferred. Preference is given to plants of the following plant family: *Brassicaceae,* in particular to plants of the genera *Brassica* and *Arabidopsis.*

Plant cell, as used herein, further includes, without limitation, cells obtained from or found in a plant or a part thereof: seeds, cultures, suspension cultures, embryos, meristematic regions, callus tissue, leaves, roots, shoots, gametophytes, sporophytes, pollen, and microspores. Plant cells can also be understood to include modified cells, such as protoplasts, obtained from the aforementioned tissues.

The term "disruption" or "disrupted" as used herein means that a gene can be structurally disrupted so as to comprise at least one mutation or structural alteration such that the disrupted gene is incapable of directing the efficient expression of a full-length fully functional gene product. The term "disruption" or "disrupted" also encompasses that the disrupted gene or one of its products can be functionally inhibited or inactivated such that a gene is either not expressed or is incapable of efficiently expressing a full-length and/or fully functional gene product. Functional inhibition or inactivation can result from a structural disruption and/or interruption of expression at either level of transcription or translation. Functional inhibition or inactivation can also be achieved e.g. by methods such as antisense polynucleotide gene suppression, double stranded RNA induced gene silencing, ribozyme techniques, and the like. The inhibition of expression and/or activity can be the result of, e.g. antisense constructs, sense constructs, RNA silencing constructs, RNA interference, genomic disruptions (e.g. transposons, tilling, homologous recombination, etc.), and/or the like. Disruption by functional inhibition also encompasses an inhibition of a gene or one of its products by interaction with a chemical compound, preferably a chemical compound interacting specifically with said gene or gene product. The inhibition of expression and/or activity can be measured by determining the presence and/or amount of transcript (e.g. by Northern blotting or RT-PCR techniques) and/or by determining the presence and/or amount of full length or truncated polypeptide encoded by said gene (e.g. by ELISA or Western blotting) and/or by determining presence and/or amount of cytokininoxidase/dehydrogenase activity of the product of the disrupted cytokininoxidase/dehydrogenase gene. The term "disruption" or "disrupted" as used herein is to be understood that a disruption also encompasses a disruption which is effective only in a part of a plant, in a particular cell type or tissue like e.g. the reproductive meristem or the shoot apex. A disruption may be achieved by interacting with or affecting within a coding region, within a non-coding region and/or within a regulatory region like e.g. a promoter region of a particular gene.

The term "transgenic" refers to a plant that has incorporated nucleic acid sequences, including but not limited to genes, polynucleotides, DNA, RNA, etc., and/or alterations thereto (e.g. mutations, point mutations or the like), which have been introduced into a plant compared to a non-introduced plant by processes which are not essentially biological processes for the production of plants. Thus, the term "transgenic plant" encompasses not only plants comprising non-endogenous nucleic acids, but explicitly refers also to plants that bear mutations in an endogenous gene, e.g. point mutations, which have been introduced into said transgenic plant compared to a non-introduced plant by processes which are not essentially biological processes for the production of plants.

The term "endogenous" relates to any gene or nucleic acid sequence that is already present in a given cell or organism like e.g. a plant. The term "exogenous" relates to any gene or nucleic acid sequences that is not endogenous.

A "transposable element" (TE) or "transposable genetic element" is a DNA sequence that can move from one location to another in a cell. Movement of a transposable element can occur from episome to episome, from episome to chromosome, from chromosome to chromosome, or from chromosome to episome. Transposable elements are characterized by the presence of inverted repeat sequences at their termini. Mobilization is mediated enzymatically by a "transposase". Structurally, a transposable element is categorized as a "transposon" (TN) or an "insertion sequence element" (IS element) based on the presence or absence, respectively, of genetic sequences in addition to those necessary for mobilization of the element. A mini-transposon or mini-IS element typically lacks sequences encoding a transposase.

The term "nucleic acid" or "polynucleotide" is generally used in its art- recognized meaning to refer to a ribose nucleic acid (RNA) or deoxyribose nucleic acid (DNA) polymer, or analog thereof, e. g., a nucleotide polymer comprising modifications of the nucleotides, a peptide nucleic acid, or the like. In certain applications, the nucleic acid can be a polymer that includes multiple monomer types, e. g., both RNA and DNA subunits. A nucleic acid can be, e. g., a chromosome or chromosomal segment, a vector (e. g., an expression vector), an expression cassette, a naked DNA or RNA polymer, the product of a polymerase chain reaction (PCR), an oligonucleotide, a probe, etc. A nucleic acid can be, e. g., single-stranded and/or double-stranded. Unless otherwise indicated, a particular nucleic acid sequence of the invention optionally comprises or encodes complementary sequences, in addition to any sequence explicitly indicated.

The term "polynucleotide sequence", "nucleic acid sequence" or "nucleotide sequence" refers to a contiguous sequence of nucleotides in a single nucleic acid or to a representation, e. g., a character string, thereof. That is, a "polynucleotide sequence" is a polymer of nucleotides (an oligonucleotide, a DNA, a nucleic acid, etc.) or a character string representing a nucleotide polymer, depending on context. From any specified polynucleotide sequence, either the given nucleic acid or the complementary polynucleotide sequence (e.g. , the complementary nucleic acid) can be determined.

The term "subsequence" or "fragment" is any portion of an entire sequence.

An "expression cassette" is a nucleic acid construct, e.g. vector, such as a plasmid, a viral vector, etc., capable of producing transcripts and, potentially, polypeptides encoded by a polynucleotide sequence. An expression vector is capable of producing transcripts in an exogenous cell, e.g. a bacterial cell, or a plant cell, *in vivo* or *in vitro,* e.g. a cultured plant protoplast. Expression of a product can be either constitutive or inducible depending, e.g. on the promoter selected. Antisense, sense or RNA interference or silencing configurations that are not or cannot be translated are expressly included by this definition. In the context of an expression vector, a promoter is said to be "operably linked" or "functionally linked" to a polynucleotide sequence if it is capable of regulating expression of the associated polynucleotide sequence. The term also applies to alternative exogenous gene constructs, such as expressed or integrated transgenes. Similarly, the term operably or functionally linked applies equally to alternative or additional transcriptional regulatory sequences such as enhancers, associated with a polynucleotide sequence.

A polynucleotide sequence, nucleic acid sequence or gene is said to "encode" a sense or antisense RNA molecule, or RNA silencing or interference molecule or a polypeptide, if the polynucleotide sequence can be transcribed (in spliced or unspliced form) and/or translated into the RNA or polypeptide, or a subsequence thereof. The skilled person is well aware of the degeneracy of the genetic code, allowing for a number of different nucleic acid sequences encoding for the same amino acid sequence or polypeptide and has no difficulties in determining whether a given nucleic acid sequence encodes for a given amino acid sequence or polypeptide.

"Expression of a gene" or "expression of a nucleic acid" means transcription of DNA into RNA (optionally including modification of the RNA, e.g. splicing), translation of RNA into a polypeptide (possibly including subsequent modification of the polypeptide, e.g. posttranslational modification), or both transcription and translation, as indicated by the context.

The term "gene" or "gene sequence" is used broadly to refer to any nucleic acid associated with a biological function. Genes typically include coding sequences and/or the regulatory sequences required for expression of such coding sequences. The term "gene" applies to a specific genomic sequence, as well as to a cDNA or an mRNA encoded by that genomic sequence. Genes also include non-expressed nucleic acid segments that, for example, form recognition sequences for other proteins. Non-expressed regulatory sequences include promoters and enhancers, to which regulatory proteins such as transcription factors bind, resulting in transcription of adjacent or nearby sequences.
A "polypeptide" is a polymer comprising two or more amino acid residues (e.g. a peptide or a protein). The polymer can additionally comprise non-amino acid elements such as labels, quenchers, blocking groups, or the like and can optionally comprise modifications such as glycosylation or the like. The amino acid residues of the polypeptide can be natural or non-natural and can be unsubstituted, unmodified, substituted or modified.

As used herein the term "cytokininoxidase/dehydrogenase gene" refers to a gene encoding for a polypeptide with cytokininoxidase/dehydrogenase activity. A cytokininoxidase/dehydrogenase is an enzyme that catalyzes the chemical reaction:

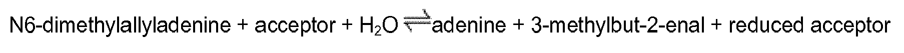

The three substrates of this enzyme are N6-dimethylallyladenine, acceptor, and H₂O, whereas its three products are adenine, 3-methylbut-2-enal, and reduced acceptor. Preferably the term "cytokininoxidase/dehydrogenase activity" encompasses the activity of a given polypeptide to catalyse an oxidoreductase reaction with at least one of the cytokinins as substrate. The skilled person is well aware of means and methods to determine whether a given polypeptide has cytokininoxidase/dehydrogenase activity or not and to determine the level of cytokininoxidase/dehydrogenase activity of a particular polypeptide or probe in absolute values and/or relative to another polypeptide or probe. There is ample guidance in the literature how a given polypeptide can be tested for such an activity, see e.g. EC 1.5.99.12. More preferably the term "cytokinin oxidase/dehydrogenase activity" encompasses the activity of a given polypeptide to catalyse an oxidoreductase reaction with at least one of the cytokinins as substrate, preferably with an activity of not less than 30% of the activity of AtCKX3 (CKX3 with SEQ ID No. 1), preferably of not less than 50% of the activity of AtCKX3.

The term "orthologue" as used herein refers to a gene from a species, preferably different from *Arabidopsis thaliana,* that shows highest similarity, preferably highest sequence identity, to the specified gene of *Arabidopsis thaliana* because both genes originated from a common ancestor. Preferably the term "orthologue" denotes an endogenous gene encoding for a cytokininoxidase/dehydrogenase and comprising a sequence (polypeptide or nucleic acid) with at least 80%, at least 85%, at least 90%, at least 95%, or at least 99% sequence identity to a given sequence the respective orthologue refers to, preferably over a particular sequence length. More preferably the term "orthologue" denotes an endogenous gene, which is derived from a species different from *Arabidopsis thaliana,* encoding for a cytokininoxidase/dehydrogenase and comprising a sequence with at least 80%, at least 85%, at least 90%, at least 95%, or at least 99% sequence identity to a given sequence of *Arabidopsis thaliana* the respective orthologue refers to, preferably over a particular sequence length.

The term "recombinant" indicates that the material (e.g. a cell, a nucleic acid, or a protein) has been artificially or synthetically (non-naturally) altered by human intervention. The alteration can be performed on the material within, or removed from, its natural environment or state. For example, a "recombinant nucleic acid" is one that is made by recombining nucleic acids, e.g. during cloning, DNA shuffling or other procedures; a "recombinant polypeptide" or "recombinant protein" is a polypeptide or protein which is produced by expression of a recombinant nucleic acid. Examples of recombinant cells include cells containing recombinant nucleic acids and/or recombinant polypeptides.

The term "vector" refers to the means by which a nucleic acid can be propagated and/or transferred between organisms, cells, or cellular components. Vectors include plasmids, viruses, bacteriophage, pro-viruses, phagemids, transposons, and artificial chromosomes, and the like, that replicate autonomously or can integrate into a chromosome of a host cell. A vector can also be a naked RNA polynucleotide, a naked DNA polynucleotide, a polynucleotide composed of both DNA and RNA within the same strand, a poly-lysine-conjugated DNA or RNA, a peptide-conjugated DNA or RNA, a liposome- conjugated DNA, or the like, that are not autonomously replicating.

In the context of the present invention, the term "isolated" refers to a biological material, such as a nucleic acid or a polypeptide, which is substantially free from components that normally accompany or interact with it in its naturally occurring environment. The isolated material optionally comprises material not found with the material in its natural environment, e.g. a cell. For example, if the material is in its natural environment, such as a cell, the material has been placed at a location in the cell (e. g., genome or genetic element) not native to a material found in that environment. For example, a naturally occurring nucleic acid (e.g. a coding sequence, a promoter, an enhancer, etc.) becomes isolated if it is introduced by non-naturally occurring means to a locus of the genome (e.g. a vector, such as a plasmid or virus vector, or amplicon) not native to that nucleic acid. An isolated plant cell, for example, can be in an environment (e.g. a cell culture system, or purified from cell culture) other than the native environment of wild-type plant cells (e.g. a whole plant).

A "promoter", as used herein, includes reference to a region of DNA upstream from the start of transcription and involved in recognition and binding of RNA polymerase and other proteins to initiate transcription. A "plant promoter" is a promoter capable of initiating transcription in plant cells. Exemplary plant promoters include, but are not limited to, those that are obtained from plants, plant viruses, and bacteria which comprise genes expressed in plant cells, such as Agrobacterium or Rhizobium. Examples of promoters under developmental control include promoters that preferentially initiate transcription in certain tissues, such as leaves, roots, or seeds or spatially in regions such as endosperm, embryo, or meristematic regions. Such promoters are referred to as "tissue- preferred" or "tissue-specific". A temporally regulated promoter drives expression at particular times, such as between 0-25 days after pollination. A "cell-type-preferred" promoter primarily drives expression in certain cell types in one or more organs, for example, vascular cells in roots or leaves. An "inducible" promoter is a promoter that is under environmental control and may be inducible or de-repressible. Examples of environmental conditions that may effect transcription by inducible promoters include anaerobic conditions or the presence of light. Tissue-specific, cell-type-specific, and inducible promoters constitute the class of "non-constitutive" promoters. A "constitutive" promoter is a promoter that is active under most environmental conditions and in all or nearly all tissues, at all or nearly all stages of development.

"Transformation", as used herein, is the process by which a cell is "transformed" by exogenous DNA when such exogenous DNA has been introduced inside the cell membrane. Exogenous DNA may or may not be integrated (covalently linked) into chromosomal DNA making up the genome of the cell. In prokaryotes and yeasts, for example, the exogenous DNA may be maintained on an episomal element, such as a plasmid. With respect to higher eukaryotic cells, a stably transformed or transfected cell is one in which the exogenous DNA has become integrated into the chromosome so that it is inherited by daughter cells through chromosome replication. This stability is demonstrated by the ability of the eukaryotic cell to establish cell lines or clones comprised of a population of daughter cells containing the exogenous DNA.

For the purpose of the present invention, sequence "identity" is objectively determined by any of a number of methods. The skilled person is well aware of these methods and can choose a suitable method without undue burden. A variety of methods for determining relationships between two or more sequences (e.g. identity, similarity and/or homology) are available and well known in the art. The methods include manual alignment, computer assisted sequence alignment and combinations thereof, for example. A number of algorithms (which are generally computer implemented) for performing sequence alignment are widely available or can be produced by one of skill. These methods include, e.g. the local homology algorithm of Smith and Waterman (1981) Adv. Appl. Math. 2: 482; the homology alignment algorithm of Needleman and Wunsch (1970) J. Mol. Biol. 48 : 443; the search for similarity method of Pearson and Lipman (1988) Proc. Natl. Acad. Sci. (USA) 85: 2444; and/or by computerized implementations of these algorithms (e.g. GAP, BESTFIT, FASTA, and TFASTA in the Wisconsin Genetics Software Package Release 7.0, Genetics Computer Group, 575 Science Dr. , Madison, WI).

For example, software for performing sequence identity (and sequence similarity) analysis using the BLAST algorithm is described in Altschul et al. (1990) J. Mol. Biol. 215: 403-410. This software is publicly available, e.g. through the National Center for Biotechnology Information on the world wide web at ncbi. nlm. nih. gov. This algorithm involves first identifying high scoring sequence pairs (HSPs) by identifying short words of length W in the query sequence, which either match or satisfy some positive-valued threshold score T when aligned with a word of the same length in a database sequence. T is referred to as the neighbourhood word score threshold. These initial neighbourhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and, speed of the alignment. The BLASTN program (for nucleotide sequences) uses as defaults a word length (W) of 11, an expectation (E) of 10, a cutoff of 100, M=5, N=-4, and a comparison of both strands. For amino acid sequences, the BLASTP (BLAST Protein) program uses as defaults a word length (W) of 3, an expectation (E) of 10, and the BLOSUM62 scoring matrix (see, Henikoff & Henikoff (1989) Proc. Natl. Acad. Sci. USA 89: 10915).

Additionally, the BLAST algorithm performs a statistical analysis of the similarity between two sequences (see, e.g. Karlin & Altschul (1993) Proc. Natl. Acad. Sci. USA 90: 5873-5787). One measure of similarity provided by the BLAST algorithm is the smallest sum probability (p (N) ), which provides an indication of the probability by which a match between two nucleotide or amino acid sequences would occur by chance. For example, a nucleic acid is considered similar to a reference sequence (and, therefore, in this context, homologous) if the smallest sum probability in a comparison of the test nucleic acid to the reference nucleic acid is less than about 0.1, or less than about 0. 01, and or even less than about 0.001.

Another example of a useful sequence alignment algorithm is PILEUP. PILEUP creates a multiple sequence alignment from a group of related sequences using progressive, pairwise alignments. It can also plot a tree showing the clustering relationships used to create the alignment. PILEUP uses a simplification of the progressive alignment method of Feng & Doolittle (1987) J. Mol. Evol. 35: 351-360. The method used is similar to the method described by Higgins & Sharp (1989) CABIOS5 : 151-153. The program can align, e.g. up to 300 sequences of a maximum length of 5,000 letters. The multiple alignment procedure begins with the pairwise alignment of the two most similar sequences, producing a cluster of two aligned sequences. This cluster can then be aligned to the next most related sequence or cluster of aligned sequences. Two clusters of sequences can be aligned by a simple extension of the pairwise alignment of two individual sequences. The final alignment is achieved by a series of progressive, pairwise alignments. The program can also be used to plot a dendogram or tree representation of clustering relationships. The program is run by designating specific sequences and their amino acid or nucleotide coordinates for regions of sequence comparison.

An additional example of an algorithm that is suitable for multiple DNA, or amino acid, sequence alignments is the CLUSTALW program (Thompson, J. D. et al. (1994) Nucl. Acids. Res. 22: 4673-4680). CLUSTALW performs multiple pairwise comparisons between groups of sequences and assembles them into a multiple alignment based on homology. Gap open and Gap extension penalties can be, e. g., 10 and 0.05 respectively. For amino acid alignments, the BLOSUM algorithm can be used as a protein weight matrix. See, e. g., Henikoff and Henikoff (1992) Proc. Natl. Acad. Sci. USA 89: 10915-10919.

The isolated plant cell or the transgenic plant of the invention can be produced by conventional means like e.g. transformation. The transformation of plant cells and protoplasts can be carried out in essentially any of the various ways known to those skilled in the art of plant molecular biology, including, but not limited to, the methods described herein. See, in general, Methods in Enzymology, Vol. 153 (Recombinant DNA Part D) Wu and Grossman (eds.) 1987, Academic Press. As used herein, the term "transformation" means alteration of the genotype of a host plant or plant cell by the introduction of a nucleic acid sequence, e.g. a "heterologous" ,"exogenous" or "foreign" nucleic acid sequence. The heterologous nucleic acid sequence need not necessarily originate from a different source but it will, at some point, have been external to the cell into which is introduced. In addition to Berger, Ausubel and Sambrook, useful general references for plant cell cloning, culture and regeneration include Jones (ed) (1995) Plant Gene Transfer and Expression Protocols--Methods in Molecular Biology, Volume 49 Humana Press Towata NJ; Payne et al. (1992) Plant Cell and Tissue Culture in Liquid Systems John Wiley & Sons, Inc. New York, NY (Payne); and Gamborg and Phillips (eds) (1995) Plant Cell, Tissue and Organ Culture; Fundamental Methods Springer Lab Manual, Springer-Verlag (Berlin Heidelberg New York) (Gamborg). A variety of cell culture media are described in Atlas and Parks (eds) The Handbook of Microbiological Media (1993) CRC Press, Boca Raton, FL (Atlas). Additional information for plant cell culture is found in available commercial literature such as the Life Science Research Cell Culture Catalogue (1998) from Sigma-Aldrich, Inc (St Louis, MO) (Sigma-LSRCCC) and, e.g. the Plant Culture Catalogue and supplement (1997) also from Sigma-Aldrich, Inc (St Louis, MO) (Sigma- PCCS). Additional details regarding plant cell culture are found in Croy, (ed.) (1993) Plant Molecular Biology Bios Scientific Publishers, Oxford, U. K.

One, more than one or all of the disruptions in at least one of the above-mentioned endogenous genes can be facilitated by introducing and expressing in a plant cell or a plant a transgenic polynucleotide sequence, e.g. in antisense or sense configurations, or RNA silencing or interference configurations, etc, wherein the transgenic polynucleotide sequence comprises a nucleic acid sequence being or being complementary to:
a) a sequence or subsequence of an endogenous CKX3 gene encoding for a cytokininoxidase/dehydrogenase comprising a polypeptide sequence being identical to or having at least 95% identity with SEQ ID No. 1 or an orthologue thereof;
b) a sequence or subsequence of an endogenous CKX2 gene encoding for a cytokininoxidase/dehydrogenase comprising a polypeptide sequence being identical to or having at least 95% identity with SEQ ID No. 2 or an orthologue thereof;
c) a sequence or subsequence of an endogenous CKX4 gene encoding for a cytokininoxidase/dehydrogenase comprising a polypeptide sequence being identical to or having at least 95% identity with SEQ ID No. 3 or an orthologue thereof;
d) a sequence or subsequence of an endogenous CKX5 gene encoding for a cytokininoxidase/dehydrogenase comprising a polypeptide sequence being identical to or having at least 95% identity with SEQ ID No. 4 or an orthologue thereof;
e) a sequence or subsequence of an endogenous CKX6 gene encoding for a cytokininoxidase/dehydrogenase comprising a polypeptide sequence being identical to or having at least 95% identity with SEQ ID No. 5 or an orthologue thereof;
   or
f) a sequence or subsequence of an endogenous gene encoding for a cytokininoxidase/dehydrogenase comprising a polypeptide sequence with at least 80%, or at least 90% sequence identity to one of SEQ ID Nos. 1, 2, 3, 4, or 5 over the whole length;
and comprise a promoter, thereby inhibiting expression and/or activity of at least the disrupted cytokininoxidase/dehydrogenase gene compared to a corresponding control plant cell or plant lacking such disruptions (e.g. its non-transgenic parent or a non- transgenic plant of the same species). The transgenic polynucleotide sequence can be introduced by techniques including, but not limited to, e.g. electroporation, micro-projectile bombardment, Agrobacterium-mediated transfer, or other available methods. In certain aspects of the invention, the polynucleotide is linked to the promoter in a sense orientation or in an antisense orientation or is configured for RNA silencing or interference.

Relevant literature describing the application of homology-dependent gene silencing includes: Jorgensen, Trends Biotechnol. 8 (12): 340-344 (1990); Flavell, Proc. Natl. Acad. Sci. (USA) 91: 3490-3496 (1994); Finnegan et al., Bio/Technology 12: 883-888 (1994); Neuhuber et al., Mol. Gen. Genet. 244: 230-241 (1994); Flavell et al. (1994) Proc. Natl. Acad. Sci. USA 91: 3490-3496; Jorgensen et al. (1996) Plant Mol. Biol. 31: 957-973; Johansen and Carrington (2001) Plant Physiol. 126: 930-938 ; Broin et al. (2002) Plant Cell 14: 1417-1432; Stoutjesdijk et al. (2002) Plant Physiol. 129: 1723- 1731; Yu et al. (2003) Phytochemistry 63: 753-763; and U.S. Patent Nos. 5,034, 323, 5,283, 184, and 5,942, 657.

Alternatively, another approach to gene silencing can be with the use of antisense technology (Rothstein et al. in Plant Mol. Cell. Biol. 6: 221-246 (1989); Liu et al. (2002) Plant Physiol. 129: 1732-1743 and U. S. Patent Nos. 5,759, 829 and 5,942, 657. Use of antisense nucleic acids is well known in the art. An antisense nucleic acid has a region of complementarity to a target nucleic acid, e.g. a particular genomic gene sequence, an mRNA, or cDNA. The antisense nucleic acid can be RNA, DNA, a PNA or any other appropriate molecule. A duplex can form between the antisense sequence and its complementary sense sequence, resulting in inactivation of the gene. The antisense nucleic acid can inhibit gene expression by forming a duplex with an RNA transcribed from the gene, by forming a triplex with duplex DNA, etc. An antisense nucleic acid can be produced by a number of well-established techniques (e. g., chemical synthesis of an antisense RNA or oligonucleotide (optionally including modified nucleotides and/or linkages that increase resistance to degradation or improve cellular uptake) or in vitro transcription). Antisense nucleic acids and their use are described, e.g. in USP 6,242, 258 to Haselton and Alexander (June 5,2001) entitled "Methods for the selective regulation of DNA and RNA transcription and translation by photoactivation"; USP 6,500, 615; USP 6,498, 035; USP 6,395, 544; USP 5,563, 050; E. Schuch et al (1991) Symp Soc. Exp Biol 45: 117-127; de Lange et al., (1995) Curr Top Microbiol Immunol 197: 57-75; Hamilton et al. (1995) Curr Top Microbiol Immunol 197: 77-89; Finnegan et al., (1996) Proc Natl Acad Sci USA 93: 8449-8454; Uhlmann and A. Pepan (1990), Chem. Rev. 90: 543; P. D. Cook (1991), Anti-Cancer Drug Design 6: 585; J. Goodchild, Bioconjugate Chem. 1 (1990) 165; and, S. L. Beaucage and R. P. lyer (1993), Tetrahedron 49: 6123; and F. Eckstein, Ed. (1991), Oligonucleotides and Analogues--A Practical Approach, IRL Press.

Catalytic RNA molecules or ribozymes can also be used to inhibit expression of particular selected genes. It is possible to design ribozymes that specifically pair with virtually any desired target RNA and cleave the phosphodiester backbone at a specific location, thereby functionally inactivating the target RNA. In carrying out this cleavage, the ribozyme is not itself altered, and is thus capable of recycling and cleaving other molecules. The inclusion of ribozyme sequences within antisense RNAs confers RNA-cleaving activity upon them, thereby increasing the activity of the constructs. A number of classes of ribozymes have been identified. For example, one class of ribozymes is derived from a number of small circular RNAs that are capable of self- cleavage and replication in plants. The RNAs can replicate either alone (viroid RNAs) or with a helper virus (satellite RNAs). Examples of RNAs include RNAs from avocado sunblotch viroid and the satellite RNAs from tobacco ringspot virus, lucerne transient streak virus, velvet tobacco mottle virus, solanum nodiflorum mottle virus and subterranean clover mottle virus. The design and use of target RNA-specific ribozymes has been described. See, e. g., Haseloff et al. (1988) Nature, 334: 585-591.

Another method to inactivate a particular selected gene by inhibiting expression is by sense suppression. Introduction of expression cassettes in which a nucleic acid is configured in the sense orientation with respect to the promoter has been shown to be an effective means by which to block the transcription of a desired target gene. See, e. g., Napoli et al. (1990), The Plant Cell 2: 279-289, and U.S. Pat. Nos. 5,034,323, 5,231,020 and 5,283,184.

Disruptions of the invention can also be produced by using RNA silencing or interference (RNAi), which can also be termed post-transcriptional gene silencing (PTGS) or co-suppression. In the context of this invention, "RNA silencing" (also called RNAi or RNA-mediated interference) refers to any mechanism through which the presence of a single-stranded or, typically, a double-stranded RNA in a cell results in inhibition of expression of a target gene comprising a sequence identical or nearly identical to that of the RNA, including, but not limited to, RNA interference, repression of translation of a target mRNA transcribed from the target gene without alteration of the mRNA's stability, and transcriptional silencing (e.g. histone acetylation and heterochromatin formation leading to inhibition of transcription of the target mRNA). In "RNA interference" the presence of the single-stranded or double-stranded RNA in the cell leads to endonucleolytic cleavage and then degradation of the target mRNA.

In one embodiment, a transgene (e.g. a sequence and/or subsequence of a gene or coding sequence of interest) is introduced into a plant cell to disrupt one or more genes by RNA silencing or interference (RNAi). For example, a sequence or subsequence (the transgene) includes a small subsequence, e.g. about 21-25 bases in length, a larger subsequence, e.g. about 25-100 or about 100-2000 (or about 200-1500, about 250-1000, etc.) bases in length, and/or the entire coding sequence or gene selected from or being complementary to:
a) a sequence or subsequence of an endogenous CKX3 gene encoding for a cytokininoxidase/dehydrogenase comprising a polypeptide sequence being identical to or having at least 95% identity with SEQ ID No. 1 or an orthologue thereof;
b) a sequence or subsequence of an endogenous CKX2 gene encoding for a cytokininoxidase/dehydrogenase comprising a polypeptide sequence being identical to or having at least 95% identity with SEQ ID No. 2 or an orthologue thereof;
c) a sequence or subsequence of an endogenous CKX4 gene encoding for a cytokininoxidase/dehydrogenase comprising a polypeptide sequence being identical to or having at least 95% identity with SEQ ID No. 3 or an orthologue thereof;
d) a sequence or subsequence of an endogenous CKX5 gene encoding for a cytokininoxidase/dehydrogenase comprising a polypeptide sequence being identical to or having at least 95% identity with SEQ ID No. 4 or an orthologue thereof;
e) a sequence or subsequence of an endogenous CKX6 gene encoding for a cytokininoxidase/dehydrogenase comprising a polypeptide sequence being identical to or having at least 95% identity with SEQ ID No. 5 or an orthologue thereof;
   or
h) a sequence or subsequence of an endogenous gene encoding for a cytokininoxidase/dehydrogenase comprising a polypeptide sequence with at least 80%, or at least 90% sequence identity to one of SEQ ID Nos. 1, 2, 3, 4, or 5over the whole length.

Preferably, a transgene includes a region in the sequence or subsequence that is about 21-25 bases in length with at least 80%, at least 90%, or at least 99% identity to a subsequence of one of the sequences with the SEQ ID No. 7, 8, 9, 10, 11, 12 or 14.

Use of RNAi for inhibiting gene expression in a number of cell types (including, e.g. plant cells) and organisms, e.g. by expression of a hairpin (stem-loop) RNA or of the two strands of an interfering RNA, for example, is well described in the literature, as are methods for determining appropriate interfering RNA (s) to target a desired gene, and for generating such interfering RNAs. For example, RNA interference is described e.g. in US patent application publications 20020173478, 20020162126, and 20020182223 and in Cogoni and Macino (2000), "Post-transcriptional gene silencing across kingdoms" Genes Dev. , 10: 638-643; Guru T. (2000), "A silence that speaks volumes" Nature 404: 804-808; Hammond et al., (2001), "Post-transcriptional Gene Silencing by Double-stranded RNA" Nature Rev. Gen. 2: 110-119; Napoli et al. , (1990) "Introduction of a chalcone synthase gene into Petunia results in reversible co-suppression of homologous genes in trayas." Plant Cell 2: 279-289; etc..

The polynucleotide sequence(s) or subsequence(s) to be expressed to induce RNAi can be expressed, e. g., under control of a constitutive promoter, an inducible promoter, or a tissue specific promoter. Expression from a tissue-specific promoter can be advantageous in certain embodiments.

One, more than one or all disruptions in at least one of the above-mentioned endogenous genes can be introduced by, e.g. transposon-based gene inactivation. For example, the inactivating step comprises producing one or more mutations in a gene being:
i) an endogenous CKX3 gene encoding for a cytokininoxidase/dehydrogenase comprising a polypeptide sequence being identical to or having at least 95% identity with SEQ ID No. 1 or an orthologue thereof, wherein the orthologue is an endogenous gene encoding for a cytokininoxidase/dehydrogenase comprising a polypeptide sequence with at least 80%, or at least 90% sequence identity to SEQ ID No. 1 over the whole length of SEQ ID No. 1;
   and/or
ii) in at least one further endogenous gene being:
   a) a CKX2 gene encoding for a cytokininoxidase/dehydrogenase comprising a polypeptide sequence being identical to or having at least 95% identity with SEQ ID No. 2 or an orthologue thereof, wherein the orthologue is an endogenous gene encoding for a cytokininoxidase/dehydrogenase comprising a polypeptide sequence with at least 80%, or at least 90% sequence identity to one of SEQ ID No. 2
      over the whole length of SEQ ID No. 2;
   b) a CKX4 gene encoding for a cytokininoxidase/dehydrogenase comprising a polypeptide sequence being identical to or having at least 95% identity with SEQ ID No. 3 or an orthologue thereof, wherein the orthologue is an endogenous gene encoding for a cytokininoxidase/dehydrogenase comprising a polypeptide sequence with at least 80%, or at least 90% sequence identity to one of SEQ ID No. 3 over the whole length of SEQ ID No. 3;
   c) a CKX5 gene encoding for a cytokininoxidase/dehydrogenase comprising a polypeptide sequence being identical to or having at least 95% identity with SEQ ID No. 4 or an orthologue thereof, wherein the orthologue is an endogenous gene encoding for a cytokininoxidase/dehydrogenase comprising a polypeptide sequence with at least 80%, or at least 90% sequence identity to one of SEQ ID No. 4 over the whole length of SEQ ID No. 4;
      or
   d) a CKX6 gene encoding for a cytokininoxidase/dehydrogenase comprising a polypeptide sequence being identical to or having at least 95% identity with SEQ ID No. 5 or an orthologue thereof, wherein the orthologue is an endogenous gene encoding for a cytokininoxidase/dehydrogenase comprising a polypeptide sequence with at least 80%, or at least 90% sequence identity to one of SEQ ID No. 5
over the whole length of SEQ ID No. 5;
wherein the one or more mutations in the gene sequence comprise one or more transposon insertions and wherein the disruptions inhibit expression and/or activity of at least the disrupted cytokininoxidase/dehydrogenase gene compared to a corresponding control plant cell or plant lacking such disruptions. For example, the one or more mutations comprise a homozygous disruption in one or more genes mentioned above or the one or more mutations comprise a heterozygous disruption in one or more genes mentioned above or a combination of both homozygous disruptions and heterozygous disruptions.

Transposons were first identified in maize by Barbara McClintock in the late 1940s. The Mutator family of transposable elements, e.g. Robertson's Mutator (Mu) transposable elements, are typically used in plant gene mutagenesis, because they are present in high copy number (10-100) and insert preferentially within and around genes.

Transposable elements can be categorized into two broad classes based on their mode of transposition. These are designated Class I and Class II; both have applications as mutagens and as delivery vectors. Class I transposable elements transpose by an RNA intermediate and use reverse transcriptases, i.e. they are retroelements. There are at least three types of Class I transposable elements, e.g. retrotransposons, retroposons, SINE-like elements. Retrotransposons typically contain LTRs, and genes encoding viral coat proteins (gag) and reverse transcriptase, RnaseH, integrase and polymerase (pol) genes. Numerous retrotransposons have been described in plant species. Such retrotransposons mobilize and translocate via a RNA intermediate in a reaction catalyzed by reverse transcriptase and RNase H encoded by the transposon. Examples fall into the Tyl-copia and Ty3-gypsy groups as well as into the SINE-like and LINE-like classifications. A more detailed discussion can be found in Kumar and Bennetzen (1999) Plant Retrotransposons in Annual Review of Genetics 33: 479.

In addition, DNA transposable elements such as Ac, Taml and En/Spm are also found in a wide variety of plant species, and can be utilized in the invention.

Transposons (and IS elements) are common tools for introducing mutations in plant cells. These mobile genetic elements are delivered to cells, e.g. through a sexual cross, transposition is selected for and the resulting insertion mutants are screened, e.g. for a phenotype of interest. The disrupted genes can then be introduced into other plants by crossing the isolated or transgenic plants with a non-disrupted plant, e.g. by a sexual cross. Any of a number of standard breeding techniques can be used, depending upon the species to be crossed. The location of a TN within a genome of an isolated or transgenic plant can be determined by known methods, e.g. sequencing of flanking regions as described herein. For example, a PCR reaction from the plant can be used to amplify the sequence, which can then be diagnostically sequenced to confirm its origin. Optionally, the insertion mutants are screened for a desired phenotype, such as the inhibition of expression or activity of agene of interest compared to a control plant.

TILLING can also be used to identify a disruption of the present invention. TILLING is Targeting Induced Local Lesions In Genomes. See, e. g., McCallum et al., (2000), "Targeting Induced Local Lesions In Genomes (TILLING) for Plant Functional Genomics" Plant Physiologv 123: 439-442; McCallum et al., (2000), "Targeted screening for induced mutations" Nature Biotechnologv 18: 455-457; and, Colbert et al., (2001), "High- Throughput Screening for Induced Point Mutations" Plant Physiology 126: 480-484.

TILLING combines high density point mutations with rapid sensitive detection of the mutations. Typically, ethyl methanesulfonate (EMS) is used to mutagenize plant seed. EMS alkylates guanine, which typically leads to mispairing. For example, seeds are soaked in an about 10-20 mM solution of EMS for about 10 to 20 hours; the seeds are washed and then sown. The plants of this generation are known as M1. M1 plants are then self-fertilized. Mutations that are present in cells that form the reproductive tissues are inherited by the next generation (M2). Typically, M2 plants are screened for mutation in the desired gene and/or for specific phenotypes. For example, DNA from M2 plants is pooled and mutations in a gene of interest are detected by detection of heteroduplex formation. Typically, DNA is prepared from each M2 plant and pooled. The desired gene is amplified by PCR. The pooled sample is then denatured and annealed to allow formation of heteroduplexes. If a mutation is present in one of the plants; the PCR products will be of two types: wild-type and mutant. Pools that include the heteroduplexes are identified by separating the PCR reaction, e.g. by Denaturing High Performance Liquid Chromatography (DPHPLC). DPHPLC detects mismatches in heteroduplexes created by melting and annealing of heteroallelic DNA. Chromatography is performed while heating the DNA. Heteroduplexes have lower thermal stability and form melting bubbles resulting in faster movement in the chromatography column. When heteroduplexes are present in addition to the expected homoduplexes, a double peak is seen. As a result, the pools that carry the mutation in a gene of interest are identified. Individual DNA from plants that make up the selected pooled population can then be identified and sequenced. Optionally, the plant possessing a desired mutation in a gene of interest can be crossed with other plants to remove background mutations.

Other mutagenic methods can also be employed to introduce a disruption of the invention. Methods for introducing genetic mutations into plant genes and selecting plants with desired traits are well known. For instance, seeds or other plant material can be treated with a mutagenic chemical substance, according to standard techniques. Such chemical substances include, but are not limited to, the following: diethyl sulfate, ethylene imine, and N-nitroso-N-ethylurea. Alternatively, ionizing radiation from sources such as X-rays or gamma rays can be used.

Other detection methods for detecting mutations in a gene of interest can be employed, e.g. capillary electrophoresis (e.g. constant denaturant capillary electrophoresis and single-stranded conformational polymorphism). In another example, heteroduplexes can be detected by using mismatch repair enzymology (e.g. CEL I endonuclease from celery). CEL I recognizes a mismatch and cleaves exactly at the 3'side of the mismatch. The precise base position of the mismatch can be determined by cutting with the mismatch repair enzyme followed by, e.g. denaturing gel electrophoresis. See, e.g. Oleykowski et al., (1998), "Mutation detection using a novel plant endonuclease" Nucleic Acid Res. 26: 4597-4602; and, Colbert et al., (2001), "High-Throughput Screening for Induced Point Mutations" Plant Physiology 126: 480-484.

The plant containing the desired disruption(s) of the invention can be crossed with other plants to introduce the disruptions into another plant. This can be done using standard breeding techniques.

Homologous recombination can also be used to introduce a disruption of the invention. Homologous recombination has been demonstrated in plants. See, e.g. Puchta et al. (1994), Experientia 50: 277-284 ; Swoboda et al. (1994), EMBOJ. 13: 484- 489 ; Offringa et al. (1993), Proc. Natl. Acad. Sci. USA 90: 7346-7350; Kempin et al. (1997) Nature 389: 802-803; and, Terada et al., (2002), "Efficient gene targeting by homologous recombination in rice" Nature Biotechnology, 20 (10): 1030-1034.

Homologous recombination can be used to induce targeted gene modifications by specifically targeting a gene of interest *in vivo.* Mutations in selected portions of a selected gene sequence (including 5' upstream, 3' downstream, and intragenic regions) such as e.g.:
i) an endogenous CKX3 gene encoding for a cytokininoxidase/dehydrogenase comprising a polypeptide sequence being identical to or having at least 95% identity with SEQ ID No. 1 or an orthologue thereof, wherein the orthologue is an endogenous gene encoding for a cytokininoxidase/dehydrogenase comprising a polypeptide sequence with at least 80%, or at least 90% sequence identity to SEQ ID No. 1 over the whole length of SEQ ID No. 1;
   and/or
ii) in at least one further endogenous gene being:
   a) a CKX2 gene encoding for a cytokininoxidase/dehydrogenase comprising a polypeptide sequence being identical to or having at least 95% identity with SEQ ID No. 2 or an orthologue thereof, wherein the orthologue is an endogenous gene encoding for a cytokininoxidase/dehydrogenase comprising a polypeptide sequence with at least 80%, or at least 90% sequence identity to one of SEQ ID No. 2 over the whole length of SEQ ID No. 2;
   b) a CKX4 gene encoding for a cytokininoxidase/dehydrogenase comprising a polypeptide sequence being identical to or having at least 95% identity with SEQ ID No. 3 or an orthologue thereof, wherein the orthologue is an endogenous gene encoding for a cytokininoxidase/dehydrogenase comprising a polypeptide sequence with at least 80%, or at least 90% sequence identity to one of SEQ ID No. 3 over the whole length of SEQ ID No. 3;
   c) a CKX5 gene encoding for a cytokininoxidase/dehydrogenase comprising a polypeptide sequence being identical to or having at least 95% identity with SEQ ID No. 4 or an orthologue thereof, wherein the orthologue is an endogenous gene encoding for a cytokininoxidase/dehydrogenase comprising a polypeptide sequence with at least 80%, or at least 90% sequence identity to one of SEQ ID No. 4 over the whole length of SEQ ID No. 4;
      or
   d) a CKX6 gene encoding for a cytokininoxidase/dehydrogenase comprising a polypeptide sequence being identical to or having at least 95% identity with SEQ ID No. 5 or an orthologue thereof, wherein the orthologue is an endogenous gene encoding for a cytokininoxidase/dehydrogenase comprising a polypeptide sequence with at least 80%, or at least 90% sequence identity to one of SEQ ID No. 5 over the whole length of SEQ ID No. 5;
are made in vitro and introduced into the desired plant using standard techniques. The mutated gene will interact with the target wild-type gene in such a way that homologous recombination and targeted replacement of the wild-type gene will occur in transgenic plants.

Isolated plant cells and/or transgenic plants of the invention, which can be consumed by humans and animals, may also be used, for example directly or after preparation known per se, as foodstuffs or feedstuffs.

The invention further relates to the use of the above-described isolated plant cells and/or transgenic plants of the invention and of the cells, cell cultures, parts, such as, for example, roots, leaves, etc., in the case of transgenic plant organisms, and transgenic propagation material such as seeds, tubers, beets/swollen tap roots or fruits derived from them for the production of food- or feedstuffs, pharmaceuticals or fine chemicals.

In the following the present invention is further described by way of examples.

### FIGURES:

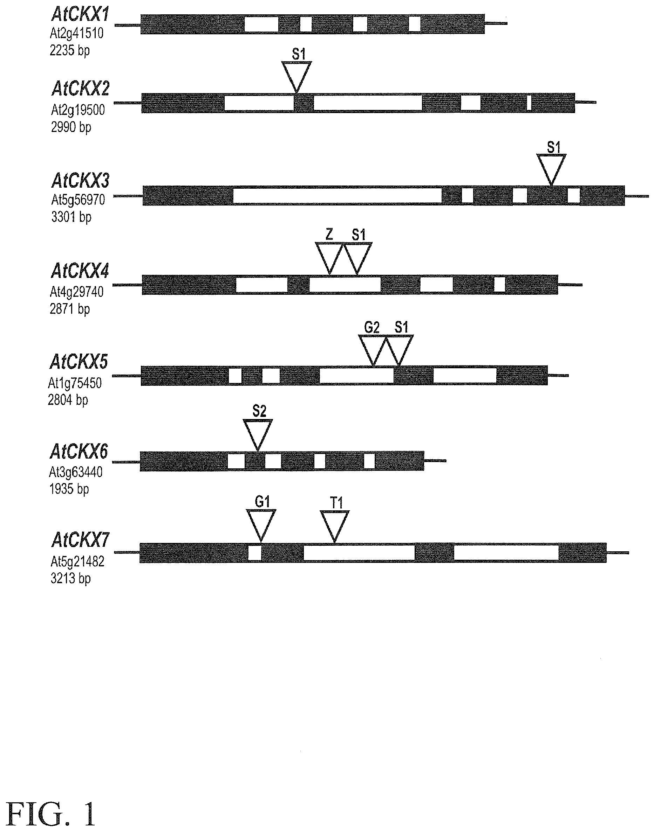

- FIG. 1:: shows positions of T-DNA and transposon insertions in the *ckx* mutants. The insertional mutants were identified by PCR screening, and the site of insertion determined by DNA sequencing of the border fragment. Black boxes represent exons, white boxes represent introns, and triangles indicate T-DNA insertions. G, GABI-KAT T-DNA-collection; S, Salk T-DNA-collection; T, Torrey Mesa T-DNA- collection; Z, ZIGIA transposon collection.

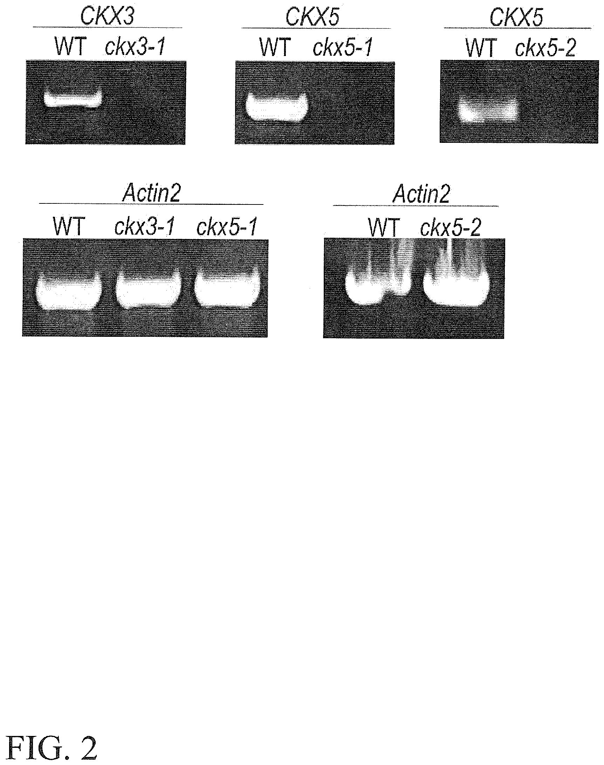

- FIG. 2: shows characterization of *ckx* T-DNA and transposon insertion alleles. Absence of *CKX* gene expression in insertional mutants. RNA from 10-d-old seedlings was used as template for the RT-PCR analysis. *Actin2* was amplified as control.

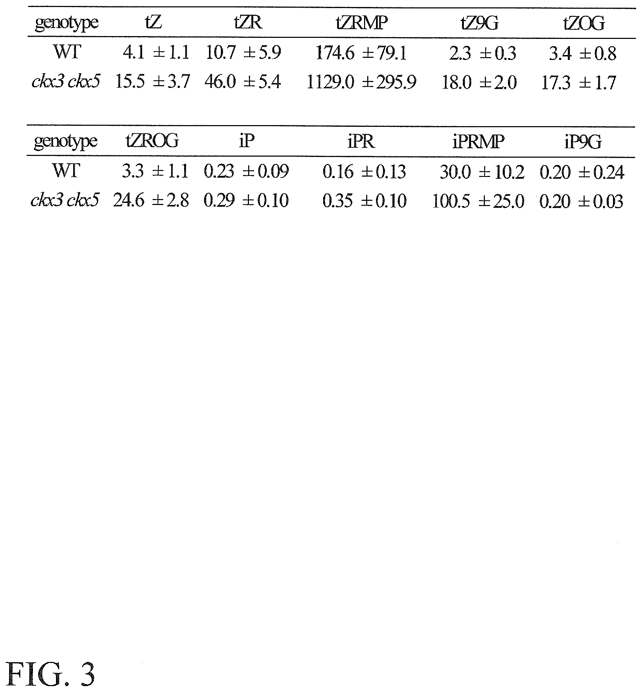

- FIG.3: shows cytokinin content in *ckx3 ckx5* mutant and wild-type inflorescences. 0.5 g of Arabidopsis inflorescences per sample was harvested and pooled 30 DAG. Five independent biological samples were harvested for each genotype. Data shown are mean values of cytokinin content [pmol/g fresh weight] ± s.d.; n = 5. tZ, *trans-*zeatin; tZR; *trans-zeatin* riboside; tZRMP, *trans*-zeatin riboside 5'-monophosphate; tZ9G, *trans*-zeatin 9-glucoside; tZROG, *trans*-zeatin riboside O-glucoside; iP, *N*⁶-(Δ²isopentenyl)adenine; iPR, *N*⁶-(Δ²isopentenyl)adenosine; iPRMP, *N*⁶-(Δ²isopentenyl)adenosine 5'-monophospate; iP9G, *N*⁶-(Δ²isopentenyl)adenine 9-glucoside.

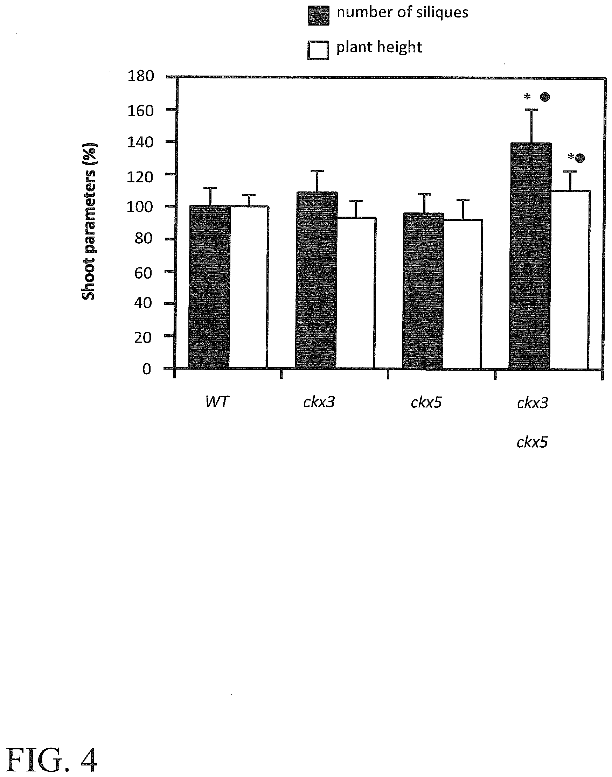

- FIG.4: shows a comparison of shoot morphology from wild-type and *ckx* mutants. Number of siliques generated by wild type and *ckx* mutants on the main stem during one life cycle. Wild-type plants had formed 54,7 siliques (100%). Plant height of Arabidopsis wild type and *ckx* mutants at the end of the flowering time. The height of wild-type plants was 39,5 cm (100%). Data represent mean values ± s.d. (n = 13-17). *, P < 0,01 compared to wild type; • = P < 0,01 compared to *ckx3.*

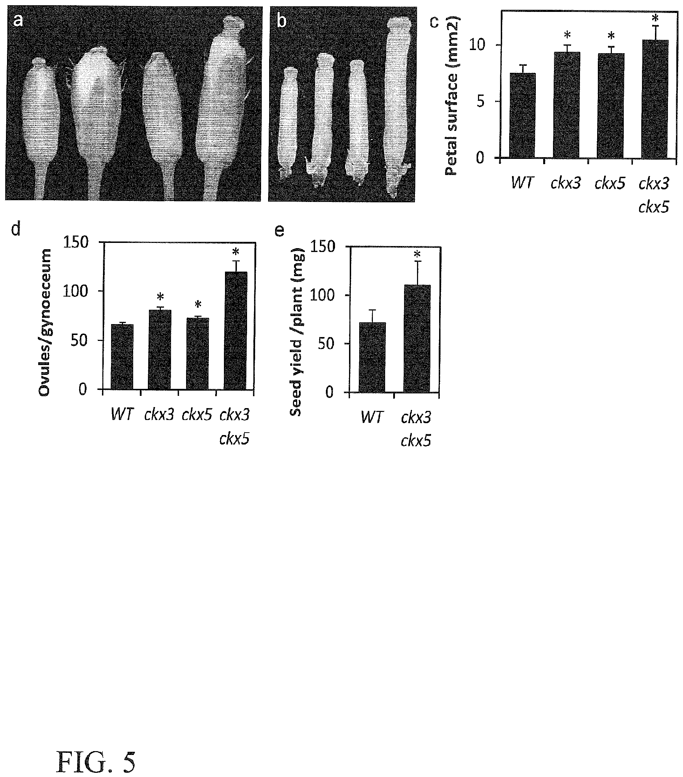

- FIG. 5: shows flower phenotype and seed yield of *ckx* mutants. a, b, Stage 13 flowers (a) and the corresponding gynoecia (b) From left to right are shown wild type, *ckx3, ckx5* and *ckx3 ckx5.* c, Petal surface of *ckx* mutants, stage 14 flowers, 39 DAG (n = 30). d, Number of ovules per gynoeceum (n = 12). e, Seed yield of wild type and *ckx3 ckx5* under growth chamber conditions (n = 30). Data represent mean values ± s.d. *, *P* < 0,01 compared to wild type.

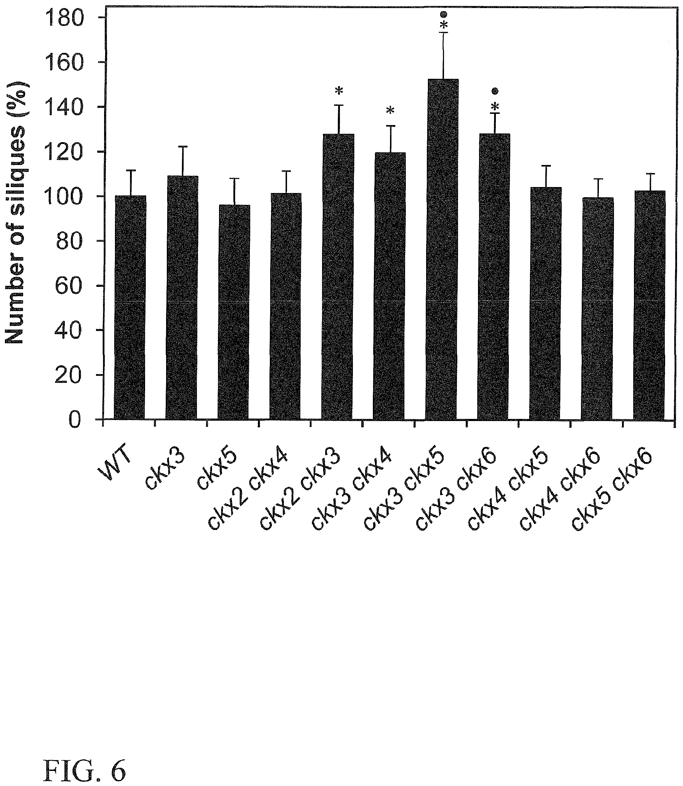

- FIG. 6: shows number of siliques generated by wild type and *ckx* mutants on the main stem during one life cycle (n = 15). Wild-type plants had formed 54,7 siliques (100%). Data represent mean values ± s.d. *, *P* < 0,01 in comparison to WT.•, *P* < 0,01 in comparison to *ckx3.*

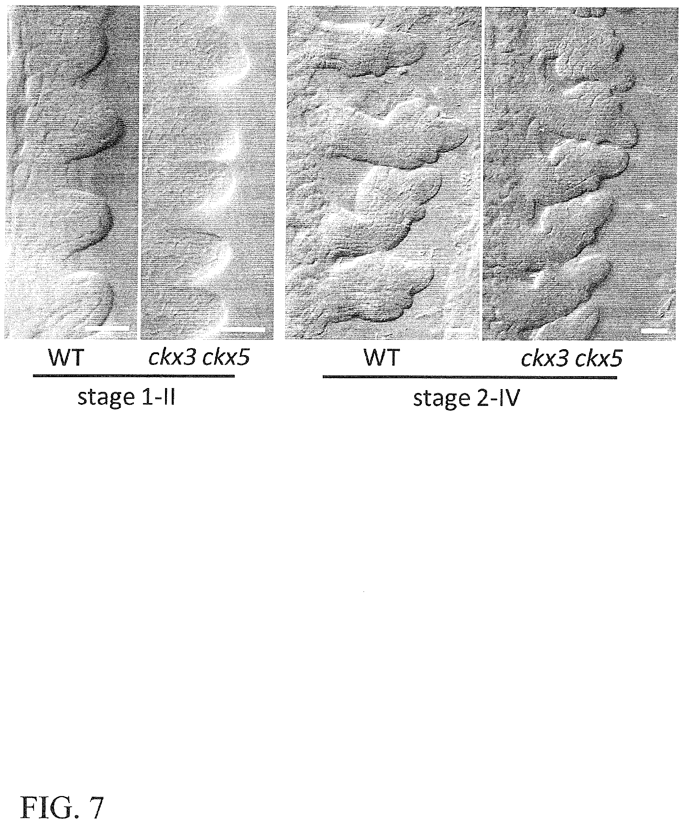

- FIG. 7: shows young ovules of wild type and *ckx3 ckx5* mutant. Staging of ovules according to Schneitz et al.. Scale bar: 10µm. The number of ovules is increased in *ckx3 ckx5* mutants compared to wild type plants.

### EXAMPLES:

### METHODS

### Plant material and growth conditions

The Columbia (Col-0) ecotype of *Arabidopsis thaliana* was used as the wild type. The T-DNA insertion mutants *ckx2-S1* (SALK_068485), *ckx3-S1* (SALK_050938), *ckx4-S1* (SALK_055204), *ckx5-S1* (SALK_064309), and *ckx6-S1* (SALK_070071) were from the Salk Institute Genomic Analysis Laboratory (Alonso et al., (2003) Science 301, 653-657), the transposon insertion mutant *ckx4-Z* was from the ZIGIA transposon collection (Baumann E, Lewald J, Saedler H, Schulz B, Wisman E (1998) Successful PCR-based reverse genetic screens using an En-1-mutagenised Arabidopsis thaliana population generated via single-seed descent. Theoretical and Applied Genetics 97: 729-734), *ckx5-G2* (Line ID 332B10) and *ckx7-G1* (Line ID 363C02) were from the GABI-KAT collection (Rosso, M.G., Li, Y., Strizhov, N., Reiss, B., Dekker, K., and Weisshaar, B. (2003) Plant Mol. Biol. 53, 247-259) and *ckx7-T1* (SAIL_515_A07) was from the Torrey Mesa Research Institute (now Syngenta). To verify the T-DNA insertion genomic primer 1 and left border primer, and to find homozygous lines genomic primer 1 and 2 were used (table 1). Double mutants were obtained by crossing and insertions were confirmed by genomic PCR with gene-specific and T-DNA border primers (Table 1). The mutant line *ckx4-Z* was not used as a crossing partner. Plants were grown in the greenhouse on soil at 22°C under long-day conditions (16 h light/8 h dark). For seed yield measurement plants were grown in growth chambers (Percival AR-66L) on soil at 24°C in ∼100 µE and 65% humidity under long-day conditions.

### Analysis of CKX expression

Total RNA was extracted from seedlings according to Verwoerd et al. (Verwoerd et al., 1989). The RNA was treated with RNase-free DNase I (Fermentas, St. Leon-Rot, Germany) at 37°C for 30 min. One microliter of 25 mM EDTA was added at 65°C for 10 min. RNA (0,5 µg) was used for a RT-PCR reaction. All used primer pairs span the respective T-DNA insertion site (Table 2). In all RT-PCR reactions, the primers for *Actin2* were used as controls. RT-PCR was performed with the One-Step RT-PCR kit (Qiagen, Hilden, Germany) according to the manufacturer's instructions. The PCR comprised 35 cycles of 30 s at 94°C, 30 s at 57°C, and 2 min at 72°C.

### Scanning electron microscopy

Scanning electron microscopy was performed as described by Krupková et al. (Krupková, E., Immerzeel, P., Pauly, M., and Schmülling, T. (2007) Plant J. 50, 735-750) using a LEO 430 microscope (Zeiss, Oberkochen, Germany).

### Cytokinin measurement

Plants were grown on soil until the main inflorescence was about 10 cm high (approx. 30 DAG). For each sample ∼0,5 g of inflorescences with stage 1 to stage 15 flowers (Smyth, D.R., Bowman, J.L., and Meyerowitz, E.M. (1990) Plant Cell 2, 755-767) were pooled and five independent samples were collected and analyzed for each genotype. The cytokinin content was determined by ultra-performance liquid chromatography-electrospray tandem mass spectrometry (Novák, O., Hauserová, E., Amakorová, P., Dole 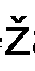 al, K., and Strnad, M. (2008) *Phytochemistry* 69, 2214-2224).

### Petal surface

The area of petals was measured from digital images of dissected organs with the Scion Image program (Scion Corporation, Frederick, Maryland, USA).

### Determination of final plant height and yield parameters

The final plant height and the number of siliques on the main stem were determined after termination of flowering. For analysis of seed yield, plants were put into paper bags after termination of flowering. After plants were kept dry for additional three weeks, total seed weight was determined.

### Light microscopy

For ovule counting and observation gynoecia were cleared and mounted as described (Malamy and Benfey, 1997). All samples were viewed with an Axioskop 2 plus microscope (Zeiss, Jena, Germany).

### Ovules counting and staging

Ovules of wild type and *ckx3 ckx5* mutants were prepared, analysed and staged as described in Schneitz er al (1995). Wild-type ovule development in Arabidopsis thaliana: a light microscope study of cleared whole-mount tissue. Plant J. 7, /31-749.

It appeared that the capacity of the placenta tissue to initiate ovule primordia is enhanced in *ckx3 ckx5* mutants compared to wild type plants, resulting in a higher overall number and density of ovules and seeds within the carpels.

**Table 1. Primer used to identify T-DNA insertions shown in Fig. 1.**

| | **genomic primer 1** | **genomic primer 2** | **Left border primer of the T-DNA insertion** |
|---|---|---|---|
| ***ckx2-S1*** | GAATGGTGGAATTGGTGGTC (SEQ ID No. 15) | GCGAGCATGTCAACATTTCA (SEQ ID No. 16) | TGGTTCACGTAGTGGGCCATCG (SEQ ID No. 17) |
| ***ckx3-S1*** | TCAAAAGCCTCCCAATTGTC (SEQ ID No.18) | CTCGGCTAAAGACGGAGTTG (SEQ ID No. 19) | TGGTTCACGTAGTGGGCCATCG (SEQ ID No. 20) |
| ***ckx4-S1*** | CTCTGCCGCTTCTCACGACTTCGGTA (SEQ ID No. 21) | CATAAACCCTGGAGCGAAACCTAGAG (SEQ ID No. 22) | TGGTTCACGTAGTGGGCCATCG (SEQ ID No. 23) |
| ***ckx4-Z*** | CAAGGTAAAACTCACACGCCATAACC (SEQ ID No. 24) | CATAAACCCTGGAGCGAAACCTAGAG (SEQ ID No. 25) | GAGCGTCGGTCCCCACACTTCTATAC (SEQ ID No. 26) |
| ***ckx5-S1*** | TTGTTGCAGCAACGACCAACCGATAATGA (SEQ ID No. 27) | AATGGTATATTGTGATGACAGGTGAGATG (SEQ ID No. 28) | TGGTTCACGTAGTGGGCCATCG (SEQ ID No. 29) |
| ***ckx5-G2*** | AATGGTATATTGTGATGACAGGTGAGATG (SEQ ID No. 30) | TTGTTGCAGCAACGACCAACCGATAATGA (SEQ ID No. 31) | ATATTGACCATCATACTCATTGC (SEQ ID No. 32) |
| ***ckx6-S2*** | ACCCTGTCCAAGAATGCTTCA (SEQ ID No. 33) | TGTGGATTCCCCTGCTCCATA (SEQ ID No. 34) | TGGTTCACGTAGTGGGCCATCG (SEQ ID No. 35) |
| ***ckx7-G1*** | TTAGCCGTCCGATCAATCTC (SEQ ID No. 36) | CGGAAAATCTACGGATGGTG (SEQ ID No. 37) | ATATTGACCATCATACTCATTGC (SEQ ID No. 38) |
| ***ckx7-T1*** | GCTAGTAAGTCAGAAGAACGAGTCATC (SEQ ID No. 39) | TTAGCCGTCCGATCAATCTC (SEQ ID No. 40) | GCCTTTTCAGAAATGGATAAATAGCCTTGCTTCC (SEQ ID No. 41) |

**Table 2. Primer used for RT-PCR analyses shown in Fig. 2.**

| | **primer 1** | **primer 2** |
|---|---|---|
| ***ckx2-S1*** | GAATGGTGGAATTGGTGGTC (SEQ ID No. 42) | AGTCCCGAAGCTGATTTTTG (SEQ ID No. 43) |
| ***ckx3-S1*** | CTCGGCTAAAGACGGAGTTG (SEQ ID No. 44) | AATAGGTGGTTGTAAACGTAGACGCA (SEQ ID No. 45) |
| ***ckx4-S1*** | CTCTGCCGCTTCTCACGACTTCGGTA (SEQ ID No. 46) | CATAAACCCTGGAGCGAAACCTAGAG (SEQ ID No. 47) |
| ***ckx4-Z*** | CTCTGCCGCTTCTCACGACTTCGGTA (SEQ ID No. 48) | CATAAACCCTGGAGCGAAACCTAGAG (SEQ ID No. 49) |
| ***ckx5-S1*** | GCACGAATCTCTCTCGAACC (SEQ ID No. 50) | CGCTGACGAAGAAGACGAC (SEQ ID No. 51) |
| ***ckx5-G2*** | GCACGAATCTCTCTCGAACC (SEQ ID No. 52) | AAATTCTTGGACCGGAGCTT (SEQ ID No. 53) |
| ***ckx6-S2*** | TGTGGATTCCCCTGCTCCATA (SEQ ID No. 54) | ACCCTGTCCAAGAATGCTTCA (SEQ ID No. 55) |
| ***ckx7-G1*** | TTAGCCGTCCGATCAATCTC (SEQ ID No. 56) | CGGAAAATCTACGGATGGTG (SEQ ID No. 57) |
| ***ckx7- T1*** | TTAGCCGTCCGATCAATCTC (SEQ ID No. 58) | CGGAAAATCTACGGATGGTG (SEQ ID No. 59) |
| ***actin2*** | TACAACGAGCTTCGTGTTGC (SEQ ID No. 60) | GATTGATCCTCCGATCCAGA (SEQ ID No. 61) |

## Claims

1. An isolated plant cell comprising a disruption in at least:
i) an endogenous CKX3 gene encoding for a cytokininoxidase/dehydrogenase comprising a polypeptide sequence being identical to or having at least 80% identity over the whole length of SEQ ID No. 1;
and
ii) in at least one further endogenous gene being:
a) a CKX2 gene encoding for a cytokininoxidase/dehydrogenase comprising a polypeptide sequence being identical to or having at least 80% identity over the whole length of SEQ ID No. 2;
b) a CKX4 gene encoding for a cytokininoxidase/dehydrogenase comprising a polypeptide sequence being identical to or having at least 80% identity over the whole length of SEQ ID No. 3;
c) a CKX5 gene encoding for a cytokininoxidase/dehydrogenase comprising a polypeptide sequence being identical to or having at least 80% identity over the whole length of SEQ ID No. 4;
or
d) a CKX6 gene encoding for a cytokininoxidase/dehydrogenase comprising a polypeptide sequence being identical to or having at least 80% identity over the whole length of SEQ ID No. 5;
wherein said disruptions inhibit expression and/or activity of a product of the at least two disrupted cytokininoxidase/dehydrogenase genes compared to a corresponding control plant cell lacking such disruptions.

2. A transgenic plant comprising a disruption in at least:
i) an endogenous CKX3 gene encoding for a cytokininoxidase/dehydrogenase comprising a polypeptide sequence being identical to or having at least 80% identity over the whole length of SEQ ID No. 1;
and
ii) in at least one further endogenous gene being:
a) a CKX2 gene encoding for a cytokininoxidase/dehydrogenase comprising a polypeptide sequence being identical to or having at least 80% identity over the whole length of SEQ ID No. 2;
b) a CKX4 gene encoding for a cytokininoxidase/dehydrogenase comprising a polypeptide sequence being identical to or having at least 80% identity over the whole length of SEQ ID No. 3;
c) a CKX5 gene encoding for a cytokininoxidase/dehydrogenase comprising a polypeptide sequence being identical to or having at least 80% identity over the whole length of SEQ ID No. 4;
or
d) a CKX6 gene encoding for a cytokininoxidase/dehydrogenase comprising a polypeptide sequence being identical to or having at least 80% identity over the whole length of SEQ ID No. 5;wherein said disruptions inhibit expression and/or activity of a product of the at least two disrupted cytokininoxidase/dehydrogenase genes compared to a corresponding control plant lacking such disruptions.

3. An isolated plant cell of claim 1 or a transgenic plant of claim 2, comprising a disruption in at least:
i) an endogenous CKX3 gene encoding for a cytokininoxidase/dehydrogenase comprising a polypeptide sequence being identical to or having at least 95% identity over the whole length of SEQ ID No. 1;
and
ii) in at least one further endogenous gene being:
a) a CKX2 gene encoding for a cytokininoxidase/dehydrogenase comprising a polypeptide sequence being identical to or having at least 95% identity over the whole length of SEQ ID No. 2;
b) a CKX4 gene encoding for a cytokininoxidase/dehydrogenase comprising a polypeptide sequence being identical to or having at least 95% identity over the whole length of SEQ ID No. 3;
c) a CKX5 gene encoding for a cytokininoxidase/dehydrogenase comprising a polypeptide sequence being identical to or having at least 95% identity over the whole length of SEQ ID No. 4;
or
d) a CKX6 gene encoding for a cytokininoxidase/dehydrogenase comprising a polypeptide sequence being identical to or having at least 95% identity over the whole length of SEQ ID No. 5.

4. The isolated plant cell of anyone of claims 1 and 3 or the transgenic plant of anyone of claims 2 and 3, wherein at least:
i) an endogenous CKX3 gene comprising a nucleic acid sequence being identical to or having at least 80% identity over the whole length of SEQ ID No. 7;
and
ii) at least one further endogenous gene being:
a) a CKX2 gene comprising a nucleic acid sequence being identical to or having at least 80% identity over the whole length of SEQ ID No. 8;
b) a CKX4 gene comprising a nucleic acid sequence being identical to or having at least 80% identity over the whole length of SEQ ID No. 9;
c) a CKX5 gene comprising a nucleic acid sequence being identical to or having at least 80% identity over the whole length of SEQ ID No. 10;
or
d) a CKX6 gene comprising a nucleic acid sequence being identical to or having at least 80% identity over the whole length of SEQ ID No. 1;
are disrupted.

5. The isolated plant cell of claim 1 or the transgenic plant of claim 2, wherein
i) at least an endogenous CKX3 gene encoding for a cytokininoxidase/dehydrogenase comprising a polypeptide sequence being identical to or having at least 80% identity over the whole length of SEQ ID No. 1;
and
ii) an endogenous CKX5 gene encoding for a cytokininoxidase/dehydrogenase comprising a polypeptide sequence being identical to or having at least 80% identity over the whole length of SEQ ID No. 4,
are disrupted.

6. The isolated plant cell of claim 4 or the transgenic plant of claim 4, wherein
i) an endogenous CKX3 gene comprising a nucleic acid sequence being identical to or having at least 80% identity over the whole length of SEQ ID No. 7;
and
ii) an endogenous CKX5 gene comprising a nucleic acid sequence being identical to or having at least 80% identity over the whole length of SEQ ID No. 10;
are disrupted.

7. The isolated plant cell of anyone of claims 1, 3, 4, 5, 6 or the transgenic plant of anyone of claims 2, 3, 4, 5, 6, wherein one, more than one or all disruptions are facilitated by structural disruption, antisense polynucleotide gene suppression, double stranded RNA induced gene silencing, ribozyme techniques, genomic disruptions, tilling, and/or homologous recombination.

8. The isolated plant cell of anyone of claims 1, 3, 4, 5, 6 and 7 or the transgenic plant of anyone of claims 2, 3, 4, 5, 6 and 7, wherein one, more than one or all disruptions are homozygous disruptions.

9. The transgenic plant of anyone of claims 2, 3, 4, 5, 6, 7 and 8, wherein the plant is selected from the family *Brassicaceae,* preferably from the genera *Brassica* or *Arabidopsis.*

10. A cell, organ, tissue or transgenic propagation material derived from the transgenic plant of anyone of claims 2, 3, 4, 5, 6, 7, 8 and 9, wherein the cell, organ, tissue or transgenic propagation material comprises said disruptions in said endogenous CKX3 gene and said at least one endogenous CKX2, CKX4, CKX5 or CKX6 gene.

11. A method of increasing a seed yield in a plant and/or increasing plant height and/or increasing stem thickness relative to a corresponding control plant, the method comprising introducing in a plant a disruption in at least:
i) an endogenous CKX3 gene encoding for a cytokininoxidase/dehydrogenase comprising a polypeptide sequence being identical to or having at least 80% identity over the whole length of SEQ ID No. 1;
and
ii) in at least one further endogenous gene being:
a) a CKX2 gene encoding for a cytokininoxidase/dehydrogenase comprising a polypeptide sequence being identical to or having at least 80% identity over the whole length of SEQ ID No. 2;
b) a CKX4 gene encoding for a cytokininoxidase/dehydrogenase comprising a polypeptide sequence being identical to or having at least 80% identity over the whole length of SEQ ID No. 3;
c) a CKX5 gene encoding for a cytokininoxidase/dehydrogenase comprising a polypeptide sequence being identical to or having at least 80% identity over the whole length of SEQ ID No. 4;
or
d) a CKX6 gene encoding for a cytokininoxidase/dehydrogenase comprising a polypeptide sequence being identical to or having at least 80% identity over the whole length of SEQ ID No. 5;
wherein said disruptions inhibit expression and/or activity of a product of the at least two disrupted cytokininoxidase/dehydrogenase genes compared to a corresponding control plant lacking such disruptions.

12. A method for producing a plant with an increased seed yield and/or plant height relative to a corresponding control plant, comprising disrupting in a plant at least:
i) an endogenous CKX3 gene encoding for a cytokininoxidase/dehydrogenase comprising a polypeptide sequence being identical to or having at least 80% identity over the whole length of SEQ ID No. 1;
and
ii) in at least one further endogenous gene being:
a) a CKX2 gene encoding for a cytokininoxidase/dehydrogenase comprising a polypeptide sequence being identical to or having at least 80% identity over the whole length of SEQ ID No. 2;
b) a CKX4 gene encoding for a cytokininoxidase/dehydrogenase comprising a polypeptide sequence being identical to or having at least 80% identity over the whole length of SEQ ID No. 3;
c) a CKX5 gene encoding for a cytokininoxidase/dehydrogenase comprising a polypeptide sequence being identical to or having at least 80% identity over the whole length of SEQ ID No. 4;
or
d) a CKX6 gene encoding for a cytokininoxidase/dehydrogenase comprising a polypeptide sequence being identical to or having at least 80% identity over the whole length of SEQ ID No. 5;wherein said disruptions inhibit expression and/or activity of a product of the at least two disrupted cytokininoxidase/dehydrogenase genes compared to a corresponding control plant lacking such disruptions.

13. The method of claim 11 or the method of claim 12, wherein at least
i) an endogenous CKX3 gene encoding for a cytokininoxidase/dehydrogenase comprising a polypeptide sequence being identical to or having at least 80% identity over the whole length of SEQ ID No. 1;
and
ii) an endogenous CKX5 gene encoding for a cytokininoxidase/dehydrogenase comprising a polypeptide sequence being identical to or having at least 80% identity over the whole length of SEQ ID No. 4,
are disrupted.

14. The method of anyone of claims 11 to 13, wherein one, more than one or all disruptions are homozygous disruptions.

## Patentansprüche

1. Isolierte pflanzliche Zelle, umfassend eine Disruption in wenigstens:
i) einem endogenen CKX3-Gen, das eine Cytokininoxidase/-dehydrogenase codiert und eine Polypeptidsequenz umfasst, die mit SEQ ID Nr. 1 identisch ist oder über deren gesamte Länge wenigstens 80 % Identität aufweist;
und
ii) in wenigstens einem weiteren endogenen Gen, bei dem es sich handelt um:
a) ein CKX2-Gen, das eine Cytokininoxidase/-dehydrogenase codiert und eine Polypeptidsequenz umfasst, die mit SEQ ID Nr. 2 identisch ist oder über deren gesamte Länge wenigstens 80 % Identität aufweist;
b) ein CKX4-Gen, das eine Cytokininoxidase/-dehydrogenase codiert und eine Polypeptidsequenz umfasst, die mit SEQ ID Nr. 3 identisch ist oder über deren gesamte Länge wenigstens 80 % Identität aufweist;
c) ein CKX5-Gen, das eine Cytokininoxidase/-dehydrogenase codiert und eine Polypeptidsequenz umfasst, die mit SEQ ID Nr. 4 identisch ist oder über deren gesamte Länge wenigstens 80 % Identität aufweist;
oder
d) ein CKX6-Gen, das eine Cytokininoxidase/-dehydrogenase codiert und eine Polypeptidsequenz umfasst, die mit SEQ ID Nr. 5 identisch ist oder über deren gesamte Länge wenigstens 80 % Identität aufweist;
wobei die Disruption eine Expression und/oder Wirksamkeit eines Produkts der wenigstens zwei gespaltenen Cytokininoxidase/-dehydrogenase-Gene hemmt, verglichen mit einer entsprechenden pflanzlichen Kontrollzelle, die keine derartigen Disruption aufweist.

2. Transgene Pflanze, umfassend eine Disruption in wenigstens:
i) einem endogenen CKX3-Gen, das eine Cytokininoxidase/-dehydrogenase codiert und eine Polypeptidsequenz umfasst, die mit SEQ ID Nr. 1 identisch ist oder über deren gesamte Länge wenigstens 80 % Identität aufweist;
und
ii) in wenigstens einem weiteren endogenen Gen, bei dem es sich handelt um:
a) ein CKX2-Gen, das eine Cytokininoxidase/-dehydrogenase codiert und eine Polypeptidsequenz umfasst, die mit SEQ ID Nr. 2 identisch ist oder über deren gesamte Länge wenigstens 80 % Identität aufweist;
b) ein CKX4-Gen, das eine Cytokininoxidase/-dehydrogenase codiert und eine Polypeptidsequenz umfasst, die mit SEQ ID Nr. 3 identisch ist oder über deren gesamte Länge wenigstens 80 % Identität aufweist;
c) ein CKX5-Gen, das eine Cytokininoxidase/-dehydrogenase codiert und eine Polypeptidsequenz umfasst, die mit SEQ ID Nr. 4 identisch ist oder über deren gesamte Länge wenigstens 80 % Identität aufweist;
oder
d) ein CKX6-Gen, das eine Cytokininoxidase/-dehydrogenase codiert und eine Polypeptidsequenz umfasst, die mit SEQ ID Nr. 5 identisch ist oder über deren gesamte Länge wenigstens 80 % Identität aufweist;
wobei die Disruption eine Expression und/oder Wirksamkeit eines Produkts der wenigstens zwei gespaltenen Cytokininoxidase/-dehydrogenase-Gene hemmt, verglichen mit einer entsprechenden Kontrollpflanze, die keine derartigen Disruptionen aufweist.

3. Isolierte pflanzliche Zelle nach Anspruch 1 oder transgene Pflanze nach Anspruch 2, umfassend eine Disruption in wenigstens:
i) einem endogenen CKX3-Gen, das eine Cytokininoxidase/-dehydrogenase codiert und eine Polypeptidsequenz umfasst, die mit SEQ ID Nr. 1 identisch ist oder über deren gesamte Länge wenigstens 95 % Identität aufweist;
und
ii) in wenigstens einem weiteren endogenen Gen, bei dem es sich handelt um:
a) ein CKX2-Gen, das eine Cytokininoxidase/-dehydrogenase codiert und eine Polypeptidsequenz umfasst, die mit SEQ ID Nr. 2 identisch ist oder über deren gesamte Länge wenigstens 95 % Identität aufweist;
b) ein CKX4-Gen, das eine Cytokininoxidase/-dehydrogenase codiert und eine Polypeptidsequenz umfasst, die mit SEQ ID Nr. 3 identisch ist oder über deren gesamte Länge wenigstens 95 % Identität aufweist;
c) ein CKX5-Gen, das eine Cytokininoxidase/-dehydrogenase codiert und eine Polypeptidsequenz umfasst, die mit SEQ ID Nr. 4 identisch ist oder über deren gesamte Länge wenigstens 95 % Identität aufweist;
oder
d) ein CKX6-Gen, das eine Cytokininoxidase/-dehydrogenase codiert und eine Polypeptidsequenz umfasst, die mit SEQ ID Nr. 5 identisch ist oder über deren gesamte Länge wenigstens 95 % Identität aufweist.

4. Isolierte pflanzliche Zelle nach einem der Ansprüche 1 und 3 oder transgene Pflanze nach einem der Ansprüche 2 und 3, wobei wenigstens:
i) ein endogenes CKX3-Gen, das eine Nukleinsäuresequenz umfasst, die mit SEQ ID Nr. 7 identisch ist oder über deren gesamte Länge wenigstens 80 % Identität aufweist;
und
ii) wenigstens ein weiteres endogenes Gen, bei dem es sich handelt um:
a) ein CKX2-Gen, das eine Nukleinsäuresequenz umfasst, die mit SEQ ID Nr. 8 identisch ist oder über deren gesamte Länge wenigstens 80 % Identität aufweist;
b) ein CKX4-Gen, das eine Nukleinsäuresequenz umfasst, die mit SEQ ID Nr. 9 identisch ist oder über deren gesamte Länge wenigstens 80 % Identität aufweist;
c) ein CKX5-Gen, das eine Nukleinsäuresequenz umfasst, die mit SEQ ID Nr. 10 identisch ist oder über deren gesamte Länge wenigstens 80 % Identität aufweist;
oder
d) ein CKX6-Gen, das eine Nukleinsäuresequenz umfasst, die mit SEQ ID Nr. 1 identisch ist oder über deren gesamte Länge wenigstens 80 % Identität aufweist;
gespalten sind.

5. Isolierte pflanzliche Zelle nach Anspruch 1 oder transgene Pflanze nach Anspruch 2, wobei
i) wenigstens ein endogenes CKX3-Gen, das eine
Cytokininoxidase/-dehydrogenase codiert und eine Polypeptidsequenz umfasst, die mit SEQ ID Nr. 1 identisch ist oder über deren gesamte Länge wenigstens 80 % Identität aufweist;
und
ii) ein endogenes CKX5-Gen, das eine Cytokininoxidase/-dehydrogenase codiert und eine Polypeptidsequenz umfasst, die mit SEQ ID Nr. 4 identisch ist oder über deren gesamte Länge wenigstens 80 % Identität aufweist,
gespalten sind.

6. Isolierte pflanzliche Zelle nach Anspruch 4 oder transgene Pflanze nach Anspruch 4, wobei
i) ein endogenes CKX3-Gen, das eine Nukleinsäuresequenz umfasst, die mit SEQ ID Nr. 7 identisch ist oder über deren gesamte Länge wenigstens 80 % Identität aufweist;
und
ii) ein endogenes CKX5-Gen, das eine Nukleinsäuresequenz umfasst, die mit SEQ ID Nr. 10 identisch ist oder über deren gesamte Länge wenigstens 80 % Identität aufweist;
gespalten sind.

7. Isolierte pflanzliche Zelle nach einem der Ansprüche 1, 3, 4, 5, 6 oder transgene Pflanze nach einem der Ansprüche 2, 3, 4, 5, 6, wobei eine, mehr als eine oder alle Disruptionen durch strukturelle Disruption, Antisense-Polynukleotid-Gensuppression, Doppelstrang-RNA-vermittelte Genstilllegung, Ribozym-Techniken, Disruptionen, TILLING und/oder homologe Rekombination von Genomen ermöglicht werden.

8. Isolierte pflanzliche Zelle nach einem der Ansprüche 1, 3, 4, 5, 6 und 7 oder transgene Pflanze nach einem der Ansprüche 2, 3, 4, 5, 6 und 7, wobei eine, mehr als eine oder alle Disruptionen homozygote Disruptionen sind.

9. Transgene Pflanze nach einem der Ansprüche 2, 3, 4, 5, 6, 7 und 8, wobei die Pflanze aus der Familie der *Brassicaceae,* vorzugsweise aus der Gattung *Brassica* oder *Arabidopsis,* ausgewählt ist.

10. Zelle, Organ, Gewebe oder transgenes Vermehrungsmaterial, das von der transgenen Pflanze nach einem der Ansprüche 2, 3, 4, 5, 6, 7, 8 und 9 abgeleitet ist, wobei die Zelle, das Organ, Gewebe oder transgene Vermehrungsmaterial die Disruptionen in dem endogenen CKX3-Gen und dem wenigstens einen endogenen CKX2-, CKX4-, CKX5- oder CKX6-Gen umfasst.

11. Verfahren zur Erhöhung der Samenausbeute in einer Pflanze und/oder Vergrößerung der Pflanzenhöhe und/oder Vergrößerung der Stammdicke bezogen auf eine entsprechende Kontrollpflanze, wobei das Verfahren umfasst, in die Pflanze eine Disruption einzuführen, in wenigstens:
i) einem endogenen CKX3-Gen, das eine Cytokininoxidase/-dehydrogenase codiert und eine Polypeptidsequenz umfasst, die mit SEQ ID Nr. 1 identisch ist oder über deren gesamte Länge wenigstens 80 % Identität aufweist;
und
ii) in wenigstens einem weiteren endogenen Gen, bei dem es sich handelt um:
a) ein CKX2-Gen, das eine Cytokininoxidase/-dehydrogenase codiert und eine Polypeptidsequenz umfasst, die mit SEQ ID Nr. 2 identisch ist oder über deren gesamte Länge wenigstens 80 % Identität aufweist;
b) ein CKX4-Gen, das eine Cytokininoxidase/-dehydrogenase codiert und eine Polypeptidsequenz umfasst, die mit SEQ ID Nr. 3 identisch ist oder über deren gesamte Länge wenigstens 80 % Identität aufweist;
c) ein CKX5-Gen, das eine Cytokininoxidase/-dehydrogenase codiert und eine Polypeptidsequenz umfasst, die mit SEQ ID Nr. 4 identisch ist oder über deren gesamte Länge wenigstens 80 % Identität aufweist;
oder
d) ein CKX6-Gen, das eine Cytokininoxidase/-dehydrogenase codiert und eine Polypeptidsequenz umfasst, die mit SEQ ID Nr. 5 identisch ist oder über deren gesamte Länge wenigstens 80 % Identität aufweist;
wobei die Disruptionen eine Expression und/oder Wirksamkeit eines Produkts der wenigstens zwei gespaltenen Cytokininoxidase/-dehydrogenase-Gene hemmt, verglichen mit einer entsprechenden Kontrollpflanze, die keine derartigen Disruptionen aufweist.

12. Verfahren zur Herstellung einer Pflanze mit einer erhöhten Samenausbeute und/oder vergrößerten Pflanzenhöhe bezogen auf eine entsprechende Kontrollpflanze, umfassend ein Spalten in einer Pflanze von wenigstens:
i) einem endogenen CKX3-Gen, das eine Cytokininoxidase/-dehydrogenase codiert und eine Polypeptidsequenz umfasst, die mit SEQ ID Nr. 1 identisch ist oder über deren gesamte Länge wenigstens 80 % Identität aufweist;
und
ii) in wenigstens einem weiteren endogenen Gen, bei dem es sich handelt um:
a) ein CKX2-Gen, das eine Cytokininoxidase/-dehydrogenase codiert und eine Polypeptidsequenz umfasst, die mit SEQ ID Nr. 2 identisch ist oder über deren gesamte Länge wenigstens 80 % Identität aufweist;
b) ein CKX4-Gen, das eine Cytokininoxidase/-dehydrogenase codiert und eine Polypeptidsequenz umfasst, die mit SEQ ID Nr. 3 identisch ist oder über deren gesamte Länge wenigstens 80 % Identität aufweist;
c) ein CKX5-Gen, das eine Cytokininoxidase/-dehydrogenase codiert und eine Polypeptidsequenz umfasst, die mit SEQ ID Nr. 4 identisch ist oder über deren gesamte Länge wenigstens 80 % Identität aufweist;
oder
d) ein CKX6-Gen, das eine Cytokininoxidase/-dehydrogenase codiert und eine Polypeptidsequenz umfasst, die mit SEQ ID Nr. 5 identisch ist oder über deren gesamte Länge wenigstens 80 % Identität aufweist;
wobei die Disruptionen eine Expression und/oder Wirksamkeit eines Produkts der wenigstens zwei gespaltenen Cytokininoxidase/-dehydrogenase-Gene hemmt, verglichen mit einer entsprechenden Kontrollpflanze, die keine derartigen Disruptionen aufweist.

13. Verfahren nach Anspruch 11 oder Verfahren nach Anspruch 12, wobei wenigstens
i) ein endogenes CKX3-Gen, das eine Cytokininoxidase/-dehydrogenase codiert und eine Polypeptidsequenz umfasst, die mit SEQ ID Nr. 1 identisch ist oder über deren gesamte Länge wenigstens 80 % Identität aufweist;
und
ii) ein endogenes CKX5-Gen, das eine Cytokininoxidase/-dehydrogenase codiert und eine Polypeptidsequenz umfasst, die mit SEQ ID Nr. 4 identisch ist oder über deren gesamte Länge wenigstens 80 % Identität aufweist,
gespalten werden.

14. Verfahren nach einem der Ansprüche 11 bis 13, wobei eine, mehr als eine oder alle Disruptionen homozygote Disruptionen sind.

## Revendications

1. Cellule végétale isolée présentant une disruption dans au moins :
i) un gène endogène CKX3 codant pour une cytokininoxydase/déshydrogénase comprenant une séquence polypeptide identique à SEQ ID N° 1, ou identique à au moins 80 % sur toute la longueur de celle-ci ;
et
ii) dans au moins un autre gène endogène, lequel est :
a) un gène CKX2 codant pour une cytokininoxydase/déshydrogénase comprenant une séquence polypeptide identique à SEQ ID N° 2, ou identique à au moins 80 % sur toute la longueur de celle-ci ;
b) un gène CKX4 codant pour une cytokininoxydase/déshydrogénase comprenant une séquence polypeptide identique à SEQ ID N° 3, ou identique à au moins 80 % sur toute la longueur de celle-ci ;
c) un gène CKX5 codant pour une cytokininoxydase/déshydrogénase comprenant une séquence polypeptide identique à SEQ ID N° 4, ou identique à au moins 80 % sur toute la longueur de celle-ci ;
ou
d) un gène CKX6 codant pour une cytokininoxydase/déshydrogénase comprenant une séquence polypeptide identique à SEQ ID N° 5, ou identique à au moins 80 % sur toute la longueur de celle-ci ;
où les disruptions empêchent l'expression et/ou l'activité d'un produit des au moins deux gènes de cytokininoxydase/déshydrogénase disruptés par rapport à une cellule végétale témoin correspondante, exempte de telles disruptions.

2. Végétal transgénique comprenant une disruption dans au moins :
i) un gène endogène CKX3 codant pour une cytokininoxydase/déshydrogénase comprenant une séquence polypeptide identique à SEQ ID N° 1, ou identique à au moins 80 % sur toute la longueur de celle-ci ;
et
ii) dans au moins un autre gène endogène, lequel est :
a) un gène CKX2 codant pour une cytokininoxydase/déshydrogénase comprenant une séquence polypeptide identique à SEQ ID N° 2, ou identique à au moins 80 % sur toute la longueur de celle-ci ;
b) un gène CKX4 codant pour une cytokininoxydase/déshydrogénase comprenant une séquence polypeptide identique à SEQ ID N° 3, ou identique à au moins 80 % sur toute la longueur de celle-ci ;
c) un gène CKX5 codant pour une cytokininoxydase/déshydrogénase comprenant une séquence polypeptide identique à SEQ ID N° 4, ou identique à au moins 80 % sur toute la longueur de celle-ci ;
ou
d) un gène CKX6 codant pour une cytokininoxydase/déshydrogénase comprenant une séquence polypeptide identique à SEQ ID N° 5, ou identique à au moins 80 % sur toute la longueur de celle-ci ; où les disruptions empêchent l'expression et/ou l'activité d'un produit des au moins deux gènes de cytokininoxydase/déshydrogénase disruptés par rapport à un végétal témoin correspondant, exempt de telles disruptions.

3. Cellule végétale isolée selon la revendication 1 ou végétal transgénique selon la revendication 2, comprenant une disruption dans au moins :
i) un gène endogène CKX3 codant pour une cytokininoxydase/déshydrogénase comprenant une séquence polypeptide identique à SEQ ID N° 1, ou identique à au moins 95 % sur toute la longueur de celle-ci ;
et
ii) dans au moins un autre gène endogène, lequel est :
a) un gène CKX2 codant pour une cytokininoxydase/déshydrogénase comprenant une séquence polypeptide identique à SEQ ID N° 2, ou identique à au moins 95 % sur toute la longueur de celle-ci;
b) un gène CKX4 codant pour une cytokininoxydase/déshydrogénase comprenant une séquence polypeptide identique à SEQ ID N° 3, ou identique à au moins 95 % sur toute la longueur de celle-ci ;
c) un gène CKX5 codant pour une cytokininoxydase/déshydrogénase comprenant une séquence polypeptide identique à SEQ ID N° 4, ou identique à au moins 95 % sur toute la longueur de celle-ci ;
ou
d) un gène CKX6 codant pour une cytokininoxydase/déshydrogénase comprenant une séquence polypeptide identique à SEQ ID N° 5, ou identique à au moins 95 % sur toute la longueur de celle-ci.

4. Cellule végétale isolée selon l'une des revendications 1 et 3 ou végétal transgénique selon la revendication 2 ou la revendication 3, où au moins :
i) un gène endogène CKX3 comprenant une séquence d'acide nucléique identique à SEQ ID N° 7, ou identique à au moins 80 % sur toute la longueur de celle-ci ;
et
ii) au moins un autre gène endogène, lequel est :
a) un gène CKX2 comprenant a séquence d'acide nucléique identique à SEQ ID N° 8, ou identique à au moins 80 % sur toute la longueur de celle-ci ;
b) un gène CKX4 comprenant a séquence d'acide nucléique identique à SEQ ID N° 9, ou identique à au moins 80 % sur toute la longueur de celle-ci ;
c) un gène CKX5 comprenant a séquence d'acide nucléique identique à SEQ ID N° 10, ou identique à au moins 80 % sur toute la longueur de celle-ci ;
ou
d) un gène CKX6 comprenant a séquence d'acide nucléique identique à SEQ ID N° 1, ou identique à au moins 80 % sur toute la longueur de celle-ci ;
sont disruptés.

5. Cellule végétale isolée selon la revendication 1 ou végétal transgénique selon la revendication 2, où
i) au moins un gène endogène CKX3 codant pour une cytokininoxydase/déshydrogénase comprenant une séquence polypeptide identique à SEQ ID N° 1, ou identique à au moins 80 % sur toute la longueur de celle-ci ;
et
ii) un gène endogène CKX5 codant pour une cytokininoxydase/déshydrogénase comprenant une séquence polypeptide identique à SEQ ID N° 4, ou identique à au moins 80 % sur toute la longueur de celle-ci,
sont disruptés.

6. Cellule végétale isolée selon la revendication 4 ou végétal transgénique selon la revendication 4, où
i) un gène endogène CKX3 comprenant a séquence d'acide nucléique identique à SEQ ID N° 7, ou identique à au moins 80 % sur toute la longueur de celle-ci ;
et
ii) un gène endogène CKX5 comprenant a séquence d'acide nucléique identique à SEQ ID N° 10, ou identique à au moins 80 % sur toute la longueur de celle-ci ;
sont disruptés.

7. Cellule végétale isolée selon l'une des revendications 1, 3, 4, 5, 6 ou végétal transgénique selon l'une des revendications 2, 3, 4, 5, 6, où une, plusieurs ou toutes les disruptions sont facilitées par disruption structurelle, suppression de gène par polynucléotide antisens, inactivation de gène induite par ADN double brin, techniques recourant à des ribozymes, disruptions génomiques, ciblage de lésions locales dans les génomes et/ou recombinaison homologue.

8. Cellule végétale isolée selon l'une des revendications 1, 3, 4, 5, 6 et 7 ou végétal transgénique selon l'une des revendications 2, 3, 4, 5, 6 et 7, où une, plusieurs ou toutes les disruptions sont des disruptions homozygotes.

9. Végétal transgénique selon l'une des revendications 2, 3, 4, 5, 6, 7 et 8, où ledit végétal est sélectionné dans la famille des *Brassicaceae,* préférentiellement dans les genres *Brassica* ou *Arabidopsis.*

10. Cellule, organe, tissu ou matériel de multiplication transgénique dérivés d'une végétal transgénique selon l'une des revendications 2, 3, 4, 5, 6, 7, 8 et 9, ladite cellule, ledit organe, tissu ou matériel de multiplication transgénique comprenant les disruptions dans le gène endogène CKX3 et au moins un gène endogène CKX2, CKX4, CKX5 ou CKX6.

11. Procédé d'accroissement du rendement en semences d'un végétal et/ou de la hauteur d'un végétal et/ou de l'épaisseur d'une tige par rapport à un végétal témoin correspondant, ledit procédé comprenant l'introduction dans un végétal d'une disruption dans au moins :
i) un gène endogène CKX3 codant pour une cytokininoxydase/déshydrogénase comprenant une séquence polypeptide identique à SEQ ID N° 1, ou identique à au moins 80 % sur toute la longueur de celle-ci ;
et
ii) dans au moins un autre gène endogène, lequel est :
a) un gène CKX2 codant pour une cytokininoxydase/déshydrogénase comprenant une séquence polypeptide identique à SEQ ID N° 2, ou identique à au moins 80 % sur toute la longueur de celle-ci ;
b) un gène CKX4 codant pour une cytokininoxydase/déshydrogénase comprenant une séquence polypeptide identique à SEQ ID N° 3, ou identique à au moins 80 % sur toute la longueur de celle-ci ;
c) un gène CKX5 codant pour une cytokininoxydase/déshydrogénase comprenant une séquence polypeptide identique à SEQ ID N° 4, ou identique à au moins 80 % sur toute la longueur de celle-ci ;
ou
d) un gène CKX6 codant pour une cytokininoxydase/déshydrogénase comprenant une séquence polypeptide identique à SEQ ID N° 5, ou identique à au moins 80 % sur toute la longueur de celle-ci ;
où les disruptions empêchent l'expression et/ou l'activité d'un produit des au moins deux gènes de cytokininoxydase/déshydrogénase disruptés par rapport à un végétal témoin correspondant, exempt de telles disruptions.

12. Procédé de production d'un végétal présentant un rendement en semences et/ou une hauteur accrus par rapport à un végétal témoin correspondant, comprenant la disruption dans un végétal d'au moins :
i) un gène endogène CKX3 codant pour cytokininoxydase/déshydrogénase comprenant une séquence polypeptide identique à SEQ ID N° 1, ou identique à au moins 80 % sur toute la longueur de celle-ci ;
et
ii) dans au moins un autre gène endogène, lequel est :
a) un gène CKX2 codant pour une cytokininoxydase/déshydrogénase comprenant une séquence polypeptide identique à SEQ ID N° 2, ou identique à au moins 80 % sur toute la longueur de celle-ci ;
b) un gène CKX4 codant pour une cytokininoxydase/déshydrogénase comprenant une séquence polypeptide identique à SEQ ID N° 3, ou identique à au moins 80 % sur toute la longueur de celle-ci ;
c) un gène CKX5 codant pour une cytokininoxydase/déshydrogénase comprenant une séquence polypeptide identique à SEQ ID N° 4, ou identique à au moins 80 % sur toute la longueur de celle-ci ;
ou
d) un gène CKX6 codant pour une cytokininoxydase/déshydrogénase comprenant une séquence polypeptide identique à SEQ ID N° 5, ou identique à au moins 80 % sur toute la longueur de celle-ci ; où les disruptions empêchent l'expression et/ou l'activité d'un produit des au moins deux gènes de cytokininoxydase/déshydrogénase disruptés par rapport à un végétal témoin correspondant, exempt de telles disruptions.

13. Procédé selon la revendication 11 ou procédé selon la revendication 12, où au moins
i) un gène endogène CKX3 codant pour une cytokininoxydase/déshydrogénase comprenant une séquence polypeptide identique à SEQ ID N° 1, ou identique à au moins 80 % sur toute la longueur de celle-ci ;
et
ii) un gène endogène CKX5 codant pour une cytokininoxydase/déshydrogénase comprenant une séquence polypeptide identique à SEQ ID N° 4, ou identique à au moins 80 % sur toute la longueur de celle-ci, sont disruptés.

14. Procédé selon l'une des revendications 11 à 13, où une, plusieurs ou toutes les disruptions sont des disruptions homozygotes.
